# EUROPEAN PATENT APPLICATION

(11) **EP 4 785 793 A1**
(43) Date of publication of application: **05.08.2026**
(21) Application number: 24872393.4
(22) Date of filing: 26.09.2024
(51) Int. Cl.: A01N 43/40, A01N 43/60, A01P 3/00, C07D 213/70, C07D 213/89, C07D 241/18, C07D 401/12, C07D 405/12, C07D 409/12

(54) **PLANT DISEASE CONTROL METHOD**

(30) Priority: 27.09.2023 JP 2023164816
(71) Applicant: Sumitomo Chemical Company, Limited, Tokyo 103-6020 (JP)
(72) Inventor: ARAI, Keisuke, Takarazuka-shi, Hyogo 665-8555 (JP); NOKURA, Yoshihiko, Takarazuka-shi, Hyogo 665-8555 (JP); MAEHATA, Nao, Takarazuka-shi, Hyogo 665-8555 (JP)
(74) Representative: Vossius & Partner Patentanwälte Rechtsanwälte mbB
(86) International application number: PCT/JP2024/034420
(87) International publication number: WO 2025/070608

(57) **Abstract**

The present invention provides an excellent control method against plant diseases. A compound represented by formula (I) 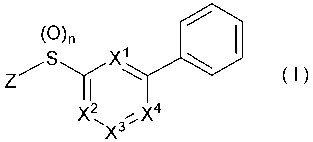 [wherein:
n represents 0, 1, or 2;
the combination of X³ and Z represents:
a combination wherein
X³ represents CR³, and
Z represents a C2-C6 chain hydrocarbon group or the like;

or, a combination wherein
X³ represents a nitrogen atom, and
Z represents a C2-C6 chain hydrocarbon group or the like;
X¹ represents CR¹ or a nitrogen atom;
X² represents CR² or a nitrogen atom;
X⁴ represents CR⁴ or a nitrogen atom (provided that among X¹, X², X³, and X⁴, the number of nitrogen atom is 0, 1, or 2); and
R¹, R², R³, and R⁴ are identical to or different from each other, and each represent a methyl group optionally substituted with one or more fluorine atom(s) or the like] or an N-oxide thereof, or a salt thereof can be used for controlling plant diseases.

## Description

### TECHNICAL FIELD

This application claims the priority to and the benefit of Japanese Patent Application No. 2023-164816 filed on September 27, 2023, the entire contents of which are incorporated herein by reference.

The present invention relates to methods for controlling plant diseases.

### BACKGROUND ART

Patent Document 1 discloses 3-arylphenyl sulfide compounds.

### CITATION LIST

### PATENT DOCUMENT

Patent Document 1: WO 99/55668 pamphlet

### SUMMARY OF THE INVENTION

### PROBLEMS TO BE SOLVED BY THE INVENTION

An object of the present invention is to provide excellent control methods against plant diseases.

### MEANS TO SOLVE PROBLEMS

The present inventors have studied to find out an excellent control method against plant diseases. As a result, they have found that a compound represented by the following formula (I) has excellent control efficacy against plant diseases.

Namely, the present invention provides the followings.
[1] A method for controlling a plant disease which comprises applying a compound represented by formula (I) 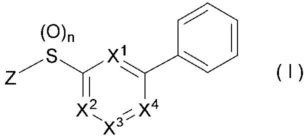 [wherein:
   n represents 0, 1, or 2;
   the combination of X³ and Z represents:
      a combination wherein
         X³ represents CR³, and
         Z represents a C2-C6 chain hydrocarbon group, a methyl group substituted with one or more fluorine atom(s), a C1-C2 alkyl group {wherein said C1-C2 alkyl group is substituted with a methoxy group or an ethoxy group}, a C3-C8 alicyclic hydrocarbon group optionally substituted with one or more substituent(s) selected from Group A, a 3-8 membered nonaromatic heterocyclic group {wherein said 3-8 membered nonaromatic heterocyclic group is bound to the S(O)ₙ at a ring-constituting carbon atom, and optionally substituted with one or more substituent(s) selected from Group A}, or a 5-10 membered aromatic heterocyclic group {wherein said 5-10 membered aromatic heterocyclic group is bound to the S(O)ₙ at a ring-constituting carbon atom, and optionally substituted with one or more substituent(s) selected from Group B};
      or, a combination wherein
         X³ represents a nitrogen atom, and
         Z represents a C2-C6 chain hydrocarbon group, a methyl group substituted with one or more fluorine atom(s), a C1-C2 alkyl group {wherein said C1-C2 alkyl group is substituted with a methoxy group or an ethoxy group}, a C3-C8 alicyclic hydrocarbon group optionally substituted with one or more substituent(s) selected from Group A, a 3-8 membered nonaromatic heterocyclic group {wherein said 3-8 membered nonaromatic heterocyclic group is bound to the S(O)ₙ at a ring-constituting carbon atom, and optionally substituted with one or more substituent(s) selected from Group A}, a phenyl group optionally substituted with one or more substituent(s) selected from Group B, or a 5-10 membered aromatic heterocyclic group {wherein said 5-10 membered aromatic heterocyclic group is bound to the S(O)ₙ at a ring-constituting carbon atom, and optionally substituted with one or more substituent(s) selected from Group B};
         X¹ represents CR¹ or a nitrogen atom;
         X² represents CR² or a nitrogen atom;
         X⁴ represents CR⁴ or a nitrogen atom (provided that among X¹, X², X³, and X⁴, the number of nitrogen atom is 0, 1, or 2); and
         R¹, R², R³, and R⁴ are identical to or different from each other, and each represent a methyl group optionally substituted with one or more fluorine atom(s), a halogen atom, or a hydrogen atom;
         Group A: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group C}, an oxo group, a thioxo group, a hydroxy group, a halogen atom, and a cyano group;
         Group B: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group C}, a halogen atom, and a cyano group;
         Group C: a group consisting of a C1-C3 alkoxy group, a halogen atom, and a cyano group]
            (hereinafter referred to as "Present compound N") or an N-oxide thereof, or a salt thereof (hereinafter the compound represented by formula (I) or an N-oxide thereof, or a salt thereof is referred to as "Present compound"), to a plant or soil for cultivating a plant.
[2] The method for controlling a plant disease according to [1], wherein the Present compound in [1] is a compound or an N-oxide thereof, or a salt thereof wherein
   the combination of X³ and Z represents:
   a combination wherein
      X³ represents CH, and
      Z represents a C2-C6 chain hydrocarbon group or a 5-10 membered aromatic heterocyclic group {wherein said 5-10 membered aromatic heterocyclic group is bound to the S(O)ₙ at a ring-constituting carbon atom, and optionally substituted with one or more substituent(s) selected from Group B};
   or, a combination wherein
      X³ represents a nitrogen atom, and
      Z represents a C2-C6 chain hydrocarbon group, a C3-C8 alicyclic hydrocarbon group optionally substituted with one or more substituent(s) selected from Group A, a 3-8 membered nonaromatic heterocyclic group {wherein said 3-8 membered nonaromatic heterocyclic group is bound to the S(O)ₙ at a ring-constituting carbon atom, and optionally substituted with one or more substituent(s) selected from Group A}, a phenyl group optionally substituted with one or more substituent(s) selected from Group B, or a 5-10 membered aromatic heterocyclic group {wherein said 5-10 membered aromatic heterocyclic group is bound to the S(O)ₙ at a ring-constituting carbon atom, and optionally substituted with one or more substituent(s) selected from Group B};
      X¹ represents CH or a nitrogen atom; and
      X² and X⁴ each represent CH.
[3] A compound represented by formula (II) 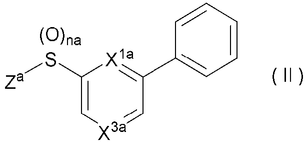 [wherein:
   na represents 1 or 2;
   X^{1a} represents CR^{1a} or a nitrogen atom;
   the combination of X^{3a} and Z^{a} represents:
      a combination wherein
         X^{3a} represents CR^{3a}, and
         Z^{a} represents a C2-C6 chain hydrocarbon group, a C1-C2 alkyl group {wherein said C1-C2 alkyl group is substituted with a methoxy group or an ethoxy group}, a C3-C8 alicyclic hydrocarbon group optionally substituted with one or more substituent(s) selected from Group A^{a}, a 3-8 membered nonaromatic heterocyclic group {wherein said 3-8 membered nonaromatic heterocyclic group is bound to the S(O)ₙₐ at a ring-constituting carbon atom, and optionally substituted with one or more substituent(s) selected from Group A^{a}}, or a 5-10 membered aromatic heterocyclic group {wherein said 5-10 membered aromatic heterocyclic group is bound to the S(O)ₙₐ at a ring-constituting carbon atom, and optionally substituted with one or more substituent(s) selected from Group B^{a}};
      or, a combination wherein
         X^{3a} represents a nitrogen atom, and
         Z^{a} represents a C2-C6 chain hydrocarbon group, a C1-C2 alkyl group {wherein said C1-C2 alkyl group is substituted with a methoxy group or an ethoxy group}, a C3-C8 alicyclic hydrocarbon group optionally substituted with one or more substituent(s) selected from Group A^{a}, a 3-8 membered nonaromatic heterocyclic group {wherein said 3-8 membered nonaromatic heterocyclic group is bound to the S(O)ₙₐ at a ring-constituting carbon atom, and optionally substituted with one or more substituent(s) selected from Group A^{a}}, a phenyl group optionally substituted with one or more substituent(s) selected from Group B^{a}, or a 5-10 membered aromatic heterocyclic group {wherein said 5-10 membered aromatic heterocyclic group is bound to the S(O)ₙₐ at a ring-constituting carbon atom, and optionally substituted with one or more substituent(s) selected from Group B^{a}}; and
         R^{1a} and R^{3a} are identical to or different from each other, and each represent a methyl group optionally substituted with one or more fluorine atom(s), a halogen atom, or a hydrogen atom;
         Group A^{a}: a group consisting of a C1-C4 chain hydrocarbon group, a C1-C6 alkoxy group {wherein said C1-C4 chain hydrocarbon group and said C1-C6 alkoxy group are optionally substituted with one or more substituent(s) selected from Group C^{a}}, an oxo group, a thioxo group, a halogen atom, and a cyano group;
         Group B^{a}: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group C^{a}}, a halogen atom, and a cyano group;
         Group C^{a}: a group consisting of a C1-C3 alkoxy group, a halogen atom, and a cyano group]
            (provided that 2-chloro-4,6-dimethyl-3-[(2-methylphenyl)sulfonyl]-5-phenylpyridine, 3-[(1-methylethyl)sulfonyl]-1,1'-biphenyl, and 4-methyl-3-[(1-methylethyl)sulfonyl]-1,1'-biphenyl are excluded) (hereinafter referred to as "Compound N of the present invention") or an N-oxide thereof, or a salt thereof (hereinafter the compound represented by formula (II) or an N-oxide thereof, or a salt thereof is referred to as "Compound of the present invention").
[4] The compound or an N-oxide thereof, or a salt thereof according to [3], wherein
   X^{1a} represents CH or a nitrogen atom; and
   the combination of X^{3a} and Z^{a} represents:
      a combination wherein
         X^{3a} represents CH, and
         Z^{a} represents a C2-C6 chain hydrocarbon group or a 5-10 membered aromatic heterocyclic group {wherein said 5-10 membered aromatic heterocyclic group is bound to the S(O)ₙₐ at a ring-constituting carbon atom, and optionally substituted with one or more substituent(s) selected from Group B^{a}};
      or, a combination wherein
         X^{3a} represents a nitrogen atom, and
         Z^{a} represents a C2-C6 chain hydrocarbon group, a C3-C8 alicyclic hydrocarbon group, a 3-8 membered nonaromatic heterocyclic group {wherein said 3-8 membered nonaromatic heterocyclic group is bound to the S(O)ₙₐ at a ring-constituting carbon atom, and optionally substituted with one or more substituent(s) selected from Group A^{a}}, a phenyl group optionally substituted with one or more substituent(s) selected from Group B^{a}, or a 5-10 membered aromatic heterocyclic group {wherein said 5-10 membered aromatic heterocyclic group is bound to the S(O)ₙₐ at a ring-constituting carbon atom, and optionally substituted with one or more substituent(s) selected from Group B^{a}}.
[5] A composition comprising the compound or an N-oxide thereof, or a salt thereof according to [3] or [4], and an inert carrier.
[6] A composition comprising one or more ingredient(s) selected from the group consisting of Group (a), Group (b), Group (c), and Group (d), and the compound or an N-oxide thereof, or a salt thereof according to [3] or [4]:
   Group (a): a group consisting of insecticidal active ingredients, miticidal active ingredients, and nematicidal active ingredients;
   Group (b): fungicidal active ingredients;
   Group (c): plant growth regulatory ingredients;
   Group (d): repellent ingredients.
[7] A method for controlling a plant disease which comprises applying an effective amount of the compound or an N-oxide thereof, or a salt thereof according to [3] or [4] or an effective amount of the composition according to [6], to a plant or soil for cultivating a plant.
[8] Use of the compound or an N-oxide thereof, or a salt thereof according to [3] or [4] or the composition according to [6] for controlling a plant disease.
[9] A seed or a vegetative reproductive organ holding an effective amount of the compound or an N-oxide thereof, or a salt thereof according to [3] or [4] or an effective amount of the composition according to [6].

### EFFECT OF INVENTION

According to the present invention, plant diseases can be controlled.

### MODE FOR CARRYING OUT THE INVENTION

The substituents in the present invention are explained as follows.

The term of "halogen atom" represents a fluorine atom, a chlorine atom, a bromine atom, or an iodine atom.

When a substituent is substituted with two or more halogen atoms or substituents, these halogen atoms or substituents may be identical to or different from each other.

Regarding the expression of "optionally substituted with one or more substituent(s) selected from Group X" (wherein X represents any one of A, B, C, B2, A^{a}, B^{a}, B^{a}2, and C^{a}) as described herein, when two or more substituents selected from Group X are present, these substituents may be identical to or different from each other.

The expression of "CX-CY" as described herein means that the number of carbon atom is X to Y. For example, the expression of "C1-C6" means that the number of carbon atom is 1 to 6.

The term of "chain hydrocarbon group" represents an alkyl group, an alkenyl group, or an alkynyl group.

Examples of the term of "alkyl group" include a methyl group, an ethyl group, a propyl group, an isopropyl group, a 1,1-dimethylpropyl group, a 1,2-dimethylpropyl group, a butyl group, a sec-butyl group, a tert-butyl group, a pentyl group, and a hexyl group.

Examples of the term of "alkenyl group" include a vinyl group, a 1-propenyl group, a 2-propenyl group, a 1-methyl-1-propenyl group, a 1-methyl-2-propenyl group, a 1,2-dimethyl-1-propenyl group, a 3-butenyl group, a 4-pentenyl group, and a 5-hexenyl group.

Examples of the term of "alkynyl group" include an ethynyl group, a 1-propynyl group, a 2-propynyl group, a 1-methyl-2-propynyl group, a 1,1-dimethyl-2-propynyl group, a 2-butynyl group, a 4-pentynyl group, and a 5-hexynyl group.

Examples of the term of "alkoxy group" include a methoxy group, an ethoxy group, a propoxy group, an isopropoxy group, a butoxy group, a tert-butoxy group, a pentyloxy group, and a hexyloxy group.

Examples of the term of "alicyclic hydrocarbon" include a cyclobutene ring, a cyclopentene ring, a cyclopentadiene ring, a cyclohexene ring, a cyclohexadiene ring, and a cycloheptene ring.

Examples of the term of "alicyclic hydrocarbon group" include a cycloalkyl group or a cycloalkenyl group.

Examples of the term of "cycloalkyl group" include a cyclopropyl group, a cyclobutyl group, a cyclopentyl group, and a cyclohexyl group.

Examples of the term of "cycloalkenyl group" include a cyclopentenyl group and a cyclohexenyl group.

Examples of the term of "nonaromatic heterocycle" include a dihydrofuran ring, a dihydrothiophene ring, a dihydropyrrole ring, a 1,3-dioxole ring, a pyran ring, and a dihydropyran ring.

Examples of the term of "aromatic heterocycle" include a furan ring, a thiophene ring, a pyrrole ring, a pyrazole ring, an imidazole ring, an oxazole ring, an isoxazole ring, a thiazole ring, an isothiazole ring, a triazole ring, an oxadiazole ring, a thiadiazole ring, a pyridine ring, a pyridazine ring, a pyrimidine ring, and a pyrazine ring.

Examples of the term of "aromatic heterocyclic group" include 5 membered aromatic heterocyclic groups such as a pyrrolyl group, a furanyl group, a thienyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, and a thiadiazolyl group; and 6 membered aromatic heterocyclic groups such as a pyridyl group, a pyridazinyl group, a pyrimidinyl group, a pyrazinyl group, a triazinyl group, and a tetrazinyl group.

Examples of the term of "nonaromatic heterocyclic group" include an aziridinyl group, an oxiranyl group, a thiiranyl group, an azetidinyl group, an oxetanyl group, a thietanyl group, a pyrrolidinyl group, a tetrahydrofuranyl group, a tetrahydrothienyl group, a pyrazolinyl group, a pyrazolidinyl group, an imidazolinyl group, an imidazolidinyl group, an oxazolinyl group, a thiazolinyl group, an oxazolidinyl group, a thiazolidinyl group, an isoxazolinyl group, an isoxazolidinyl group, an isothiazolinyl group, an isothiazolidinyl group, a dioxolanyl group, a dioxanyl group, a piperidyl group, a piperazinyl group, a morpholinyl group, a thiomorpholinyl group, a pyranyl group, a dihydropyranyl group, a tetrahydropyranyl group, a tetrahydrothiopyranyl group, an azepanyl group, an oxepanyl group, and a thiepanyl group.

2-Chloro-4,6-dimethyl-3-[(2-methylphenyl)sulfonyl]-5-phenylpyridine is a compound represented by the following formula.

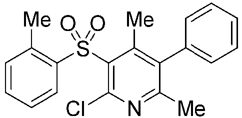

3-[(1-Methylethyl)sulfonyl]-1,1'-biphenyl is a compound represented by the following formula.

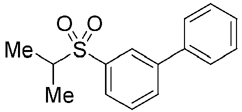

4-Methyl-3-[(1-methylethyl)sulfonyl]-1,1'-biphenyl is a compound represented by the following formula.

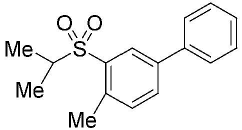

The Present compound or the Compound of the present invention may optionally have one or more stereoisomer(s). Examples of the stereoisomer(s) include enantiomers, diastereomers, atropisomers, and geometric isomers. The present invention encompasses each stereoisomer and mixtures of stereoisomers at any ratio.

An N-oxide of the compound represented by formula (I) or formula (II) means a structure in which at least one nitrogen atom contained in the compound represented by formula (I) or formula (II) is substituted with an oxo group.

The compound represented by formula (I) or formula (II), or an N-oxide thereof may optionally be mixed with an acid such as hydrochloric acid, sulfuric acid, nitric acid, phosphoric acid, acetic acid, and benzoic acid to form an acid addition salt such as hydrochloride, sulfate, nitrate, phosphate, acetate, and benzoate.

Aspects of the Present compound N include the following compounds.
[Aspect 1] The Present compound N, wherein Z represents a C2-C6 chain hydrocarbon group.
[Aspect 2] The Present compound N, wherein Z represents a C2-C6 alkyl group.
[Aspect 3] The Present compound N, wherein Z represents an ethyl group or a propyl group.
[Aspect 4] The Present compound N, wherein Z represents a methyl group substituted with one or more fluorine atom(s).
[Aspect 5] The Present compound N, wherein Z represents a trifluoromethyl group.
[Aspect 6] The Present compound N, wherein Z represents a C1-C2 alkyl group {wherein said C1-C2 alkyl group is substituted with a methoxy group or an ethoxy group}.
[Aspect 7] The Present compound N, wherein Z represents a 2-methoxyethyl group or a 2-ethoxyethyl group.
[Aspect 8] The Present compound N, wherein Z represents a C3-C8 alicyclic hydrocarbon group optionally substituted with one or more substituent(s) selected from Group A.
[Aspect 9] The Present compound N, wherein Z represents a C3-C8 alicyclic hydrocarbon group.
[Aspect 10] The Present compound N, wherein Z represents a cyclopropyl group, a cyclopentyl group, or a cyclohexyl group.
[Aspect 11] The Present compound N, wherein Z represents a 3-8 membered nonaromatic heterocyclic group {wherein said 3-8 membered nonaromatic heterocyclic group is bound to the S(O)ₙ at a ring-constituting carbon atom, and optionally substituted with one or more substituent(s) selected from Group A}.
[Aspect 12] The Present compound N, wherein Z represents a 3-8 membered nonaromatic heterocyclic group in which a ring-constituting carbon atom is bound to the S(O)ₙ.
[Aspect 13] The Present compound N, wherein Z represents a 3-tetrahydrofuranyl group or a 3-tetrahydrothienyl group.
[Aspect 14] The Present compound N, wherein Z represents a 5-10 membered aromatic heterocyclic group {wherein said 5-10 membered aromatic heterocyclic group is bound to the S(O)ₙ at a ring-constituting carbon atom, and optionally substituted with one or more substituent(s) selected from Group B}.
[Aspect 15] The Present compound N, wherein Z represents a 5 membered aromatic heterocyclic group {wherein said 5 membered aromatic heterocyclic group is bound to the S(O)ₙ at a ring-constituting carbon atom, and optionally substituted with one or more substituent(s) selected from Group B2};
   Group B2: a group consisting of a C1-C6 chain hydrocarbon group and a halogen atom.
[Aspect 16] The Present compound N, wherein Z represents a 2-furyl group, a 3-furyl group, a 2-thienyl group, or a 3-thienyl group.
[Aspect 17] The Present compound N, wherein Z represents a 6 membered aromatic heterocyclic group {wherein said 6 membered aromatic heterocyclic group is bound to the S(O)ₙ at a ring-constituting carbon atom, and optionally substituted with one or more substituent(s) selected from Group B2}.
[Aspect 18] The Present compound N, wherein Z represents a 2-pyridyl group, a 3-pyridyl group, a 4-pyridyl group, a 2-pyrimidinyl group, or a 2-pyrazinyl group.
[Aspect 19] The Present compound N, wherein Z represents a 5-6 membered aromatic heterocyclic group {wherein said 5-6 membered aromatic heterocyclic group is bound to the S(O)ₙ at a ring-constituting carbon atom, and optionally substituted with one or more substituent(s) selected from Group B2}.
[Aspect 20] The Present compound N, wherein Z represents a 2-furyl group, a 3-furyl group, a 2-thienyl group, a 3-thienyl group, a 2-pyridyl group, a 3-pyridyl group, a 4-pyridyl group, a 2-pyrimidinyl group, or a 2-pyrazinyl group.
[Aspect 21] The Present compound N, wherein Z represents a C2-C6 alkyl group or a 5-6 membered aromatic heterocyclic group {wherein said 5-6 membered aromatic heterocyclic group is bound to the S(O)ₙ at a ring-constituting carbon atom, and optionally substituted with one or more substituent(s) selected from Group B2}.
[Aspect 22] The Present compound N, wherein Z represents an ethyl group, a propyl group, a 2-furyl group, a 3-furyl group, a 2-thienyl group, a 3-thienyl group, a 2-pyridyl group, a 3-pyridyl group, a 4-pyridyl group, a 2-pyrimidinyl group, or a 2-pyrazinyl group.
[Aspect 23] The Present compound N, wherein Z represents a C2-C6 alkyl group or a 5 membered aromatic heterocyclic group {wherein said 5 membered aromatic heterocyclic group is bound to the S(O)ₙ at a ring-constituting carbon atom, and optionally substituted with one or more substituent(s) selected from Group B2}.
[Aspect 24] The Present compound N, wherein Z represents an ethyl group, a propyl group, a 2-furyl group, a 3-furyl group, a 2-thienyl group, or a 3-thienyl group.
[Aspect 25] The Present compound N, wherein Z represents a C2-C6 alkyl group or a 6 membered aromatic heterocyclic group {wherein said 6 membered aromatic heterocyclic group is bound to the S(O)ₙ at a ring-constituting carbon atom, and optionally substituted with one or more substituent(s) selected from Group B2}.
[Aspect 26] The Present compound N, wherein Z represents an ethyl group, a propyl group, a 2-pyridyl group, a 3-pyridyl group, a 4-pyridyl group, a 2-pyrimidinyl group, or a 2-pyrazinyl group.
[Aspect 27] The Present compound N, wherein Z represents a C2-C6 chain hydrocarbon group, a C3-C8 alicyclic hydrocarbon group, or a 5-6 membered aromatic heterocyclic group {wherein said 5-6 membered aromatic heterocyclic group is bound to the S(O)ₙ at a ring-constituting carbon atom, and optionally substituted with one or more substituent(s) selected from Group B2}.
[Aspect 28] The Present compound N, wherein Z represents a C2-C6 alkyl group, a C3-C8 alicyclic hydrocarbon group, or a 5-6 membered aromatic heterocyclic group {wherein said 5-6 membered aromatic heterocyclic group is bound to the S(O)ₙ at a ring-constituting carbon atom, and optionally substituted with one or more substituent(s) selected from Group B2}.
[Aspect 29] The Present compound N, wherein Z represents an ethyl group, a propyl group, a cyclopropyl group, a cyclopentyl group, a cyclohexyl group, a 2-furyl group, a 3-furyl group, a 2-thienyl group, a 3-thienyl group, a 2-pyridyl group, a 3-pyridyl group, a 4-pyridyl group, a 2-pyrimidinyl group, or a 2-pyrazinyl group.
[Aspect 30] The Present compound N, wherein
   Z represents a phenyl group optionally substituted with one or more substituent(s) selected from Group B; and
   X³ represents a nitrogen atom.
[Aspect 31] The Present compound N, wherein
   Z represents a phenyl group optionally substituted with one or more substituent(s) selected from Group B2; and
   X³ represents a nitrogen atom.
[Aspect 32] The Present compound N, wherein
   Z represents a phenyl group; and
   X³ represents a nitrogen atom.
[Aspect 33] The Present compound N, wherein
   the combination of X³ and Z represents:
   a combination wherein
      X³ represents CR³, and
      Z represents a C2-C6 chain hydrocarbon group or a 5-10 membered aromatic heterocyclic group {wherein said 5-10 membered aromatic heterocyclic group is bound to the S(O)ₙ at a ring-constituting carbon atom, and optionally substituted with one or more substituent(s) selected from Group B};
   or, a combination wherein
      X³ represents a nitrogen atom, and
      Z represents a C2-C6 chain hydrocarbon group, a 5-10 membered aromatic heterocyclic group {wherein said 5-10 membered aromatic heterocyclic group is bound to the S(O)ₙ at a ring-constituting carbon atom, and optionally substituted with one or more substituent(s) selected from Group B}, or a phenyl group optionally substituted with one or more substituent(s) selected from Group B.
[Aspect 34] The Present compound N, wherein
   the combination of X³ and Z represents:
   a combination wherein
   X³ represents CR³, and
   Z represents a C2-C6 alkyl group or a 5-6 membered aromatic heterocyclic group {wherein said 5-6 membered aromatic heterocyclic group is bound to the S(O)ₙ at a ring-constituting carbon atom, and optionally substituted with one or more substituent(s) selected from Group B2}; or, a combination wherein
   X³ represents a nitrogen atom, and
   Z represents a C2-C6 alkyl group, a 5-6 membered aromatic heterocyclic group {wherein said 5-6 membered aromatic heterocyclic group is bound to the S(O)ₙ at a ring-constituting carbon atom, and optionally substituted with one or more substituent(s) selected from Group B2}, or a phenyl group optionally substituted with one or more substituent(s) selected from Group B2.
[Aspect 35] The Present compound N, wherein
   the combination of X³ and Z represents:
   a combination wherein
      X³ represents CR³, and
      Z represents an ethyl group, a propyl group, a 2-furyl group, a 3-furyl group, a 2-thienyl group, a 3-thienyl group, a 2-pyridyl group, a 3-pyridyl group, a 4-pyridyl group, a 2-pyrimidinyl group, or a 2-pyrazinyl group;
   or, a combination wherein
      X³ represents a nitrogen atom, and
      Z represents an ethyl group, a propyl group, a 2-furyl group, a 3-furyl group, a 2-thienyl group, a 3-thienyl group, a 2-pyridyl group, a 3-pyridyl group, a 4-pyridyl group, a 2-pyrimidinyl group, a 2-pyrazinyl group, or a phenyl group.
[Aspect 36] The Present compound N, wherein
   the combination of X³ and Z represents:
   a combination wherein
   X³ represents CR³, and
   Z represents a C2-C6 alkyl group or a 5 membered aromatic heterocyclic group {wherein said 5 membered aromatic heterocyclic group is bound to the S(O)ₙ at a ring-constituting carbon atom, and optionally substituted with one or more substituent(s) selected from Group B2}; or, a combination wherein
   X³ represents a nitrogen atom, and
   Z represents a C2-C6 alkyl group, a 5 membered aromatic heterocyclic group {wherein said 5 membered aromatic heterocyclic group is bound to the S(O)ₙ at a ring-constituting carbon atom, and optionally substituted with one or more substituent(s) selected from Group B2}, or a phenyl group optionally substituted with one or more substituent(s) selected from Group B2.
[Aspect 37] The Present compound N, wherein
   the combination of X³ and Z represents:
   a combination wherein
      X³ represents CR³, and
      Z represents an ethyl group, a propyl group, a 2-pyridyl group, a 3-pyridyl group, a 4-pyridyl group, a 2-pyrimidinyl group, or a 2-pyrazinyl group;
   or, a combination wherein
      X³ represents a nitrogen atom, and
      Z represents an ethyl group, a propyl group, a 2-furyl group, a 3-furyl group, a 2-thienyl group, a 3-thienyl group, or a phenyl group.
[Aspect 38] The Present compound N, wherein
   the combination of X³ and Z represents:
   a combination wherein
      X³ represents CR³, and
      Z represents a 6 membered aromatic heterocyclic group {wherein said 6 membered aromatic heterocyclic group is bound to the S(O)ₙ at a ring-constituting carbon atom, and optionally substituted with one or more substituent(s) selected from Group B2};
   or, a combination wherein
      X³ represents a nitrogen atom, and
      Z represents a C2-C6 alkyl group, a 6 membered aromatic heterocyclic group {wherein said 6 membered aromatic heterocyclic group is bound to the S(O)ₙ at a ring-constituting carbon atom, and optionally substituted with one or more substituent(s) selected from Group B2}, or a phenyl group optionally substituted with one or more substituent(s) selected from Group B2.
[Aspect 39] The Present compound N, wherein
   the combination of X³ and Z represents:
   a combination wherein
      X³ represents CR³, and
      Z represents an ethyl group, a propyl group, a 2-pyridyl group, a 3-pyridyl group, a 4-pyridyl group, a 2-pyrimidinyl group, or a 2-pyrazinyl group;
   or, a combination wherein
      X³ represents a nitrogen atom, and
      Z represents an ethyl group, a propyl group, a 2-pyridyl group, a 3-pyridyl group, a 4-pyridyl group, a 2-pyrimidinyl group, a 2-pyrazinyl group, or a phenyl group.
[Aspect 40] The Present compound N, wherein
   the combination of X³ and Z represents:
   a combination wherein
      X³ represents CR³, and
      Z represents a 5 membered aromatic heterocyclic group {wherein said 5 membered aromatic heterocyclic group is bound to the S(O)ₙ at a ring-constituting carbon atom, and optionally substituted with one or more substituent(s) selected from Group B2};
   or, a combination wherein
      X³ represents a nitrogen atom, and
      Z represents a 5 membered aromatic heterocyclic group {wherein said 5 membered aromatic heterocyclic group is bound to the S(O)ₙ at a ring-constituting carbon atom, and optionally substituted with one or more substituent(s) selected from Group B2}, or a phenyl group optionally substituted with one or more substituent(s) selected from Group B2.
[Aspect 41] The Present compound N, wherein
   the combination of X³ and Z represents:
   a combination wherein
      X³ represents CR³, and
      Z represents a 2-pyridyl group, a 3-pyridyl group, a 4-pyridyl group, a 2-pyrimidinyl group, or a 2-pyrazinyl group;
   or, a combination wherein
      X³ represents a nitrogen atom, and
      Z represents a 2-furyl group, a 3-furyl group, a 2-thienyl group, a 3-thienyl group, or a phenyl group.
[Aspect 42] The Present compound N, wherein
   the combination of X³ and Z represents:
   a combination wherein
      X³ represents CR³, and
      Z represents a 6 membered aromatic heterocyclic group {wherein said 6 membered aromatic heterocyclic group is bound to the S(O)ₙ at a ring-constituting carbon atom, and optionally substituted with one or more substituent(s) selected from Group B2};
   or, a combination wherein
      X³ represents a nitrogen atom, and
      Z represents a C2-C6 alkyl group, a 6 membered aromatic heterocyclic group {wherein said 6 membered aromatic heterocyclic group is bound to the S(O)ₙ at a ring-constituting carbon atom, and optionally substituted with one or more substituent(s) selected from Group B2}, or a phenyl group optionally substituted with one or more substituent(s) selected from Group B2.
[Aspect 43] The Present compound N, wherein the combination of X³ and Z represents:
   a combination wherein
      X³ represents CR³, and
      Z represents a 2-pyridyl group, a 3-pyridyl group, a 4-pyridyl group, a 2-pyrimidinyl group, or a 2-pyrazinyl group;
   or, a combination wherein
      X³ represents a nitrogen atom, and
      Z represents a 2-pyridyl group, a 3-pyridyl group, a 4-pyridyl group, a 2-pyrimidinyl group, a 2-pyrazinyl group, or a phenyl group.
[Aspect 44] The Present compound N, wherein
   the combination of X³ and Z represents:
   a combination wherein
      X³ represents CR³, and
      Z represents a 5-6 membered aromatic heterocyclic group {wherein said 5-6 membered aromatic heterocyclic group is bound to the S(O)ₙ at a ring-constituting carbon atom, and optionally substituted with one or more substituent(s) selected from Group B2};
   or, a combination wherein
      X³ represents a nitrogen atom, and
      Z represents a 5-6 membered aromatic heterocyclic group {wherein said 5-6 membered aromatic heterocyclic group is bound to the S(O)ₙ at a ring-constituting carbon atom, and optionally substituted with one or more substituent(s) selected from Group B2}, or a phenyl group optionally substituted with one or more substituent(s) selected from Group B2.
[Aspect 45] The Present compound N, wherein
   the combination of X³ and Z represents:
   a combination wherein
      X³ represents CR³, and
      Z represents a 2-furyl group, a 3-furyl group, a 2-thienyl group, a 3-thienyl group, a 2-pyridyl group, a 3-pyridyl group, a 4-pyridyl group, a 2-pyrimidinyl group, or a 2-pyrazinyl group;
   or, a combination wherein
      X³ represents a nitrogen atom, and
      Z represents a 2-furyl group, a 3-furyl group, a 2-thienyl group, a 3-thienyl group, a 2-pyridyl group, a 3-pyridyl group, a 4-pyridyl group, a 2-pyrimidinyl group, a 2-pyrazinyl group, or a phenyl group.
[Aspect 46] The Present compound N, wherein
   X¹ represents CR¹;
   X² represents CR²;
   X³ represents CR³; and
   X⁴ represents CR⁴.
[Aspect 47] The Present compound N, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 48] The compound according to the Aspect 1, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 49] The compound according to the Aspect 2, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 50] The compound according to the Aspect 3, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 51] The compound according to the Aspect 4, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 52] The compound according to the Aspect 5, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 53] The compound according to the Aspect 6, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 54] The compound according to the Aspect 7, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 55] The compound according to the Aspect 8, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 56] The compound according to the Aspect 9, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 57] The compound according to the Aspect 10, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 58] The compound according to the Aspect 11, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 59] The compound according to the Aspect 12, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 60] The compound according to the Aspect 13, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 61] The compound according to the Aspect 14, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 62] The compound according to the Aspect 15, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 63] The compound according to the Aspect 16, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 64] The compound according to the Aspect 17, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 65] The compound according to the Aspect 18, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 66] The compound according to the Aspect 19, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 67] The compound according to the Aspect 20, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 68] The compound according to the Aspect 21, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 69] The compound according to the Aspect 22, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 70] The compound according to the Aspect 23, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 71] The compound according to the Aspect 24, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 72] The compound according to the Aspect 25, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 73] The compound according to the Aspect 26, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 74] The compound according to the Aspect 27, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 75] The compound according to the Aspect 28, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 76] The compound according to the Aspect 29, wherein
   X¹ represents CR¹;
   X² represents CR²;
   X³ represents CR³;
   X⁴ represents CR⁴; and
   R¹, R², R³, and R⁴ each represent a hydrogen atom.
[Aspect 77] The Present compound N, wherein
   X¹ represents a nitrogen atom;
   X² represents CR²;
   X³ represents CR³; and
   X⁴ represents CR⁴.
[Aspect 78] The Present compound N, wherein
   X¹ represents a nitrogen atom;
   X² represents CR²;
   X³ represents CR³;
   X⁴ represents CR⁴; and
   R², R³, and R⁴ each represent a hydrogen atom.
[Aspect 79] The Present compound N, wherein
   X¹ represents CR¹;
   X² represents a nitrogen atom;
   X³ represents CR³; and
   X⁴ represents CR⁴.
[Aspect 80] The Present compound N, wherein
   X¹ represents CR¹;
   X² represents a nitrogen atom;
   X³ represents CR³;
   X⁴ represents CR⁴; and
   R¹, R³, and R⁴ each represent a hydrogen atom.
[Aspect 81] The Present compound N, wherein
   X¹ represents CR¹;
   X² represents CR²;
   X³ represents a nitrogen atom; and
   X⁴ represents CR⁴.
[Aspect 82] The Present compound N, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 83] The compound according to the Aspect 1, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 84] The compound according to the Aspect 2, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 85] The compound according to the Aspect 3, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 86] The compound according to the Aspect 4, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 87] The compound according to the Aspect 5, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 88] The compound according to the Aspect 6, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 89] The compound according to the Aspect 7, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 90] The compound according to the Aspect 8, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 91] The compound according to the Aspect 9, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 92] The compound according to the Aspect 10, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 93] The compound according to the Aspect 11, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 94] The compound according to the Aspect 12, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 95] The compound according to the Aspect 13, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 96] The compound according to the Aspect 14, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 97] The compound according to the Aspect 15, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 98] The compound according to the Aspect 16, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 99] The compound according to the Aspect 17, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 100] The compound according to the Aspect 18, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 101] The compound according to the Aspect 19, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 102] The compound according to the Aspect 20, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 103] The compound according to the Aspect 21, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 104] The compound according to the Aspect 22, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 105] The compound according to the Aspect 23, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 106] The compound according to the Aspect 24, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 107] The compound according to the Aspect 25, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 108] The compound according to the Aspect 26, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 109] The compound according to the Aspect 27, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 110] The compound according to the Aspect 28, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 111] The compound according to the Aspect 29, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 112] The compound according to the Aspect 30, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 113] The compound according to the Aspect 31, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 114] The compound according to the Aspect 32, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 115] The compound according to the Aspect 33, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 116] The compound according to the Aspect 34, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 117] The compound according to the Aspect 35, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 118] The compound according to the Aspect 36, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 119] The compound according to the Aspect 37, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 120] The compound according to the Aspect 38, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 121] The compound according to the Aspect 39, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 122] The compound according to the Aspect 40, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 123] The compound according to the Aspect 41, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 124] The compound according to the Aspect 42, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 125] The compound according to the Aspect 43, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 126] The compound according to the Aspect 44, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 127] The compound according to the Aspect 45, wherein
   X¹ represents CR¹;
   X² represents CR²;
   X³ represents a nitrogen atom;
   X⁴ represents CR⁴; and
   R¹, R², and R⁴ each represent a hydrogen atom.
[Aspect 128] The Present compound N, wherein
   X¹ represents CR¹;
   X² represents CR²;
   X³ represents CR³; and
   X⁴ represents a nitrogen atom.
[Aspect 129] The Present compound N, wherein
   X¹ represents CR¹;
   X² represents CR²;
   X³ represents CR³;
   X⁴ represents a nitrogen atom; and
   R¹, R², and R³ each represent a hydrogen atom.
[Aspect 130] The Present compound N, wherein
   X¹ represents a nitrogen atom;
   X² represents a nitrogen atom;
   X³ represents CR³; and
   X⁴ represents CR⁴.
[Aspect 131] The Present compound N, wherein
   X¹ represents a nitrogen atom;
   X² represents a nitrogen atom;
   X³ represents CR³;
   X⁴ represents CR⁴; and
   R³ and R⁴ each represent a hydrogen atom.
[Aspect 132] The Present compound N, wherein
   X¹ represents a nitrogen atom;
   X² represents CR²;
   X³ represents a nitrogen atom; and
   X⁴ represents CR⁴.
[Aspect 133] The Present compound N, wherein
   X¹ represents a nitrogen atom;
   X² represents CR²;
   X³ represents a nitrogen atom;
   X⁴ represents CR⁴; and
   R² and R⁴ each represent a hydrogen atom.
[Aspect 134] The Present compound N, wherein
   X¹ represents a nitrogen atom;
   X² represents CR²;
   X³ represents CR³; and
   X⁴ represents a nitrogen atom.
[Aspect 135] The Present compound N, wherein
   X¹ represents a nitrogen atom;
   X² represents CR²;
   X³ represents CR³;
   X⁴ represents a nitrogen atom; and
   R² and R³ each represent a hydrogen atom.
[Aspect 136] The Present compound N, wherein
   X¹ represents CR¹;
   X² represents a nitrogen atom;
   X³ represents a nitrogen atom; and
   X⁴ represents CR⁴.
[Aspect 137] The Present compound N, wherein
   X¹ represents CR¹;
   X² represents a nitrogen atom;
   X³ represents a nitrogen atom;
   X⁴ represents CR⁴; and
   R¹ and R⁴ each represent a hydrogen atom.
[Aspect 138] The Present compound N, wherein
   X¹ represents CR¹;
   X² represents a nitrogen atom;
   X³ represents CR³; and
   X⁴ represents a nitrogen atom.
[Aspect 139] The Present compound N, wherein
   X¹ represents CR¹;
   X² represents a nitrogen atom;
   X³ represents CR³;
   X⁴ represents a nitrogen atom; and
   R¹ and R³ each represent a hydrogen atom.
[Aspect 140] The Present compound N, wherein
   X¹ represents CR¹;
   X² represents CR²;
   X³ represents a nitrogen atom; and
   X⁴ represents a nitrogen atom.
[Aspect 141] The Present compound N, wherein
   X¹ represents CR¹;
   X² represents CR²;
   X³ represents a nitrogen atom;
   X⁴ represents a nitrogen atom; and
   R¹ and R² each represent a hydrogen atom.
[Aspect 142] The Present compound N, wherein
   X² represents CR²; and
   X⁴ represents CR⁴.
[Aspect 143] The compound according to the Aspect 1, wherein
   X² represents CR²; and
   X⁴ represents CR⁴.
[Aspect 144] The compound according to the Aspect 2, wherein
   X² represents CR²; and
   X⁴ represents CR⁴.
[Aspect 145] The compound according to the Aspect 3, wherein
   X² represents CR²; and
   X⁴ represents CR⁴.
[Aspect 146] The compound according to the Aspect 4, wherein
   X² represents CR²; and
   X⁴ represents CR⁴.
[Aspect 147] The compound according to the Aspect 5, wherein
   X² represents CR²; and
   X⁴ represents CR⁴.
[Aspect 148] The compound according to the Aspect 6, wherein
   X² represents CR²; and
   X⁴ represents CR⁴.
[Aspect 149] The compound according to the Aspect 7, wherein
   X² represents CR²; and
   X⁴ represents CR⁴.
[Aspect 150] The compound according to the Aspect 8, wherein
   X² represents CR²; and
   X⁴ represents CR⁴.
[Aspect 151] The compound according to the Aspect 9, wherein
   X² represents CR²; and
   X⁴ represents CR⁴.
[Aspect 152] The compound according to the Aspect 10, wherein
   X² represents CR²; and
   X⁴ represents CR⁴.
[Aspect 153] The compound according to the Aspect 11, wherein
   X² represents CR²; and
   X⁴ represents CR⁴.
[Aspect 154] The compound according to the Aspect 12, wherein
   X² represents CR²; and
   X⁴ represents CR⁴.
[Aspect 155] The compound according to the Aspect 13, wherein
   X² represents CR²; and
   X⁴ represents CR⁴.
[Aspect 156] The compound according to the Aspect 14, wherein
   X² represents CR²; and
   X⁴ represents CR⁴.
[Aspect 157] The compound according to the Aspect 15, wherein
   X² represents CR²; and
   X⁴ represents CR⁴.
[Aspect 158] The compound according to the Aspect 16, wherein
   X² represents CR²; and
   X⁴ represents CR⁴.
[Aspect 159] The compound according to the Aspect 17, wherein
   X² represents CR²; and
   X⁴ represents CR⁴.
[Aspect 160] The compound according to the Aspect 18, wherein
   X² represents CR²; and
   X⁴ represents CR⁴.
[Aspect 161] The compound according to the Aspect 19, wherein
   X² represents CR²; and
   X⁴ represents CR⁴.
[Aspect 162] The compound according to the Aspect 20, wherein
   X² represents CR²; and
   X⁴ represents CR⁴.
[Aspect 163] The compound according to the Aspect 21, wherein
   X² represents CR²; and
   X⁴ represents CR⁴.
[Aspect 164] The compound according to the Aspect 22, wherein
   X² represents CR²; and
   X⁴ represents CR⁴.
[Aspect 165] The compound according to the Aspect 23, wherein
   X² represents CR²; and
   X⁴ represents CR⁴.
[Aspect 166] The compound according to the Aspect 24, wherein
   X² represents CR²; and
   X⁴ represents CR⁴.
[Aspect 167] The compound according to the Aspect 25, wherein
   X² represents CR²; and
   X⁴ represents CR⁴.
[Aspect 168] The compound according to the Aspect 26, wherein
   X² represents CR²; and
   X⁴ represents CR⁴.
[Aspect 169] The compound according to the Aspect 27, wherein
   X² represents CR²; and
   X⁴ represents CR⁴.
[Aspect 170] The compound according to the Aspect 28, wherein
   X² represents CR²; and
   X⁴ represents CR⁴.
[Aspect 171] The compound according to the Aspect 29, wherein
   X² represents CR²; and
   X⁴ represents CR⁴.
[Aspect 172] The compound according to the Aspect 33, wherein
   X² represents CR²; and
   X⁴ represents CR⁴.
[Aspect 173] The compound according to the Aspect 34, wherein
   X² represents CR²; and
   X⁴ represents CR⁴.
[Aspect 174] The compound according to the Aspect 35, wherein
   X² represents CR²; and
   X⁴ represents CR⁴.
[Aspect 175] The compound according to the Aspect 36, wherein
   X² represents CR²; and
   X⁴ represents CR⁴.
[Aspect 176] The compound according to the Aspect 37, wherein
   X² represents CR²; and
   X⁴ represents CR⁴.
[Aspect 177] The compound according to the Aspect 38, wherein
   X² represents CR²; and
   X⁴ represents CR⁴.
[Aspect 178] The compound according to the Aspect 39, wherein
   X² represents CR²; and
   X⁴ represents CR⁴.
[Aspect 179] The compound according to the Aspect 40, wherein
   X² represents CR²; and
   X⁴ represents CR⁴.
[Aspect 180] The compound according to the Aspect 41, wherein
   X² represents CR²; and
   X⁴ represents CR⁴.
[Aspect 181] The compound according to the Aspect 42, wherein
   X² represents CR²; and
   X⁴ represents CR⁴.
[Aspect 182] The compound according to the Aspect 43, wherein
   X² represents CR²; and
   X⁴ represents CR⁴.
[Aspect 183] The compound according to the Aspect 44, wherein
   X² represents CR²; and
   X⁴ represents CR⁴.
[Aspect 184] The compound according to the Aspect 45, wherein
   X² represents CR²; and
   X⁴ represents CR⁴.
[Aspect 185] The Present compound N, wherein
   X² represents CR²;
   X⁴ represents CR⁴; and
   R¹, R², R³, and R⁴ each represent a hydrogen atom.
[Aspect 186] The compound according to the Aspect 1, wherein
   X² represents CR²;
   X⁴ represents CR⁴; and
   R¹, R², R³, and R⁴ each represent a hydrogen atom.
[Aspect 187] The compound according to the Aspect 2, wherein
   X² represents CR²;
   X⁴ represents CR⁴; and
   R¹, R², R³, and R⁴ each represent a hydrogen atom.
[Aspect 188] The compound according to the Aspect 3, wherein
   X² represents CR²;
   X⁴ represents CR⁴; and
   R¹, R², R³, and R⁴ each represent a hydrogen atom.
[Aspect 189] The compound according to the Aspect 4, wherein
   X² represents CR²;
   X⁴ represents CR⁴; and
   R¹, R², R³, and R⁴ each represent a hydrogen atom.
[Aspect 190] The compound according to the Aspect 5, wherein
   X² represents CR²;
   X⁴ represents CR⁴; and
   R¹, R², R³, and R⁴ each represent a hydrogen atom.
[Aspect 191] The compound according to the Aspect 6, wherein
   X² represents CR²;
   X⁴ represents CR⁴; and
   R¹, R², R³, and R⁴ each represent a hydrogen atom.
[Aspect 192] The compound according to the Aspect 7, wherein
   X² represents CR²;
   X⁴ represents CR⁴; and
   R¹, R², R³, and R⁴ each represent a hydrogen atom.
[Aspect 193] The compound according to the Aspect 8, wherein
   X² represents CR²;
   X⁴ represents CR⁴; and
   R¹, R², R³, and R⁴ each represent a hydrogen atom.
[Aspect 194] The compound according to the Aspect 9, wherein
   X² represents CR²;
   X⁴ represents CR⁴; and
   R¹, R², R³, and R⁴ each represent a hydrogen atom.
[Aspect 195] The compound according to the Aspect 10, wherein
   X² represents CR²;
   X⁴ represents CR⁴; and
   R¹, R², R³, and R⁴ each represent a hydrogen atom.
[Aspect 196] The compound according to the Aspect 11, wherein
   X² represents CR²;
   X⁴ represents CR⁴; and
   R¹, R², R³, and R⁴ each represent a hydrogen atom.
[Aspect 197] The compound according to the Aspect 12, wherein
   X² represents CR²;
   X⁴ represents CR⁴; and
   R¹, R², R³, and R⁴ each represent a hydrogen atom.
[Aspect 198] The compound according to the Aspect 13, wherein
   X² represents CR²;
   X⁴ represents CR⁴; and
   R¹, R², R³, and R⁴ each represent a hydrogen atom.
[Aspect 199] The compound according to the Aspect 14, wherein
   X² represents CR²;
   X⁴ represents CR⁴; and
   R¹, R², R³, and R⁴ each represent a hydrogen atom.
[Aspect 200] The compound according to the Aspect 15, wherein
   X² represents CR²;
   X⁴ represents CR⁴; and
   R¹, R², R³, and R⁴ each represent a hydrogen atom.
[Aspect 201] The compound according to the Aspect 16, wherein
   X² represents CR²;
   X⁴ represents CR⁴; and
   R¹, R², R³, and R⁴ each represent a hydrogen atom.
[Aspect 202] The compound according to the Aspect 17, wherein
   X² represents CR²;
   X⁴ represents CR⁴; and
   R¹, R², R³, and R⁴ each represent a hydrogen atom.
[Aspect 203] The compound according to the Aspect 18, wherein
   X² represents CR²;
   X⁴ represents CR⁴; and
   R¹, R², R³, and R⁴ each represent a hydrogen atom.
[Aspect 204] The compound according to the Aspect 19, wherein
   X² represents CR²;
   X⁴ represents CR⁴; and
   R¹, R², R³, and R⁴ each represent a hydrogen atom.
[Aspect 205] The compound according to the Aspect 20, wherein
   X² represents CR²;
   X⁴ represents CR⁴; and
   R¹, R², R³, and R⁴ each represent a hydrogen atom.
[Aspect 206] The compound according to the Aspect 21, wherein
   X² represents CR²;
   X⁴ represents CR⁴; and
   R¹, R², R³, and R⁴ each represent a hydrogen atom.
[Aspect 207] The compound according to the Aspect 22, wherein
   X² represents CR²;
   X⁴ represents CR⁴; and
   R¹, R², R³, and R⁴ each represent a hydrogen atom.
[Aspect 208] The compound according to the Aspect 23, wherein
   X² represents CR²;
   X⁴ represents CR⁴; and
   R¹, R², R³, and R⁴ each represent a hydrogen atom.
[Aspect 209] The compound according to the Aspect 24, wherein
   X² represents CR²;
   X⁴ represents CR⁴; and
   R¹, R², R³, and R⁴ each represent a hydrogen atom.
[Aspect 210] The compound according to the Aspect 25, wherein
   X² represents CR²;
   X⁴ represents CR⁴; and
   R¹, R², R³, and R⁴ each represent a hydrogen atom.
[Aspect 211] The compound according to the Aspect 26, wherein
   X² represents CR²;
   X⁴ represents CR⁴; and
   R¹, R², R³, and R⁴ each represent a hydrogen atom.
[Aspect 212] The compound according to the Aspect 27, wherein
   X² represents CR²;
   X⁴ represents CR⁴; and
   R¹, R², R³, and R⁴ each represent a hydrogen atom.
[Aspect 213] The compound according to the Aspect 28, wherein
   X² represents CR²;
   X⁴ represents CR⁴; and
   R¹, R², R³, and R⁴ each represent a hydrogen atom.
[Aspect 214] The compound according to the Aspect 29, wherein
   X² represents CR²;
   X⁴ represents CR⁴; and
   R¹, R², R³, and R⁴ each represent a hydrogen atom.
[Aspect 215] The compound according to the Aspect 33, wherein
   X² represents CR²;
   X⁴ represents CR⁴; and
   R¹, R², R³, and R⁴ each represent a hydrogen atom.
[Aspect 216] The compound according to the Aspect 34, wherein
   X² represents CR²;
   X⁴ represents CR⁴; and
   R¹, R², R³, and R⁴ each represent a hydrogen atom.
[Aspect 217] The compound according to the Aspect 35, wherein
   X² represents CR²;
   X⁴ represents CR⁴; and
   R¹, R², R³, and R⁴ each represent a hydrogen atom.
[Aspect 218] The compound according to the Aspect 36, wherein
   X² represents CR²;
   X⁴ represents CR⁴; and
   R¹, R², R³, and R⁴ each represent a hydrogen atom.
[Aspect 219] The compound according to the Aspect 37, wherein
   X² represents CR²;
   X⁴ represents CR⁴; and
   R¹, R², R³, and R⁴ each represent a hydrogen atom.
[Aspect 220] The compound according to the Aspect 38, wherein
   X² represents CR²;
   X⁴ represents CR⁴; and
   R¹, R², R³, and R⁴ each represent a hydrogen atom.
[Aspect 221] The compound according to the Aspect 39, wherein
   X² represents CR²;
   X⁴ represents CR⁴; and
   R¹, R², R³, and R⁴ each represent a hydrogen atom.
[Aspect 222] The compound according to the Aspect 40, wherein
   X² represents CR²;
   X⁴ represents CR⁴; and
   R¹, R², R³, and R⁴ each represent a hydrogen atom.
[Aspect 223] The compound according to the Aspect 41, wherein
   X² represents CR²;
   X⁴ represents CR⁴; and
   R¹, R², R³, and R⁴ each represent a hydrogen atom.
[Aspect 224] The compound according to the Aspect 42, wherein
   X² represents CR²;
   X⁴ represents CR⁴; and
   R¹, R², R³, and R⁴ each represent a hydrogen atom.
[Aspect 225] The compound according to the Aspect 43, wherein
   X² represents CR²;
   X⁴ represents CR⁴; and
   R¹, R², R³, and R⁴ each represent a hydrogen atom.
[Aspect 226] The compound according to the Aspect 44, wherein
   X² represents CR²;
   X⁴ represents CR⁴; and
   R¹, R², R³, and R⁴ each represent a hydrogen atom.
[Aspect 227] The compound according to the Aspect 45, wherein
   X² represents CR²;
   X⁴ represents CR⁴; and
   R¹, R², R³, and R⁴ each represent a hydrogen atom.
[Aspect 228] The Present compound N, wherein
   X² represents CR²;
   X³ represents a nitrogen atom; and
   X⁴ represents CR⁴.
[Aspect 229] The compound according to the Aspect 1, wherein
   X² represents CR²;
   X³ represents a nitrogen atom; and
   X⁴ represents CR⁴.
[Aspect 230] The compound according to the Aspect 2, wherein
   X² represents CR²;
   X³ represents a nitrogen atom; and
   X⁴ represents CR⁴.
[Aspect 231] The compound according to the Aspect 3, wherein
   X² represents CR²;
   X³ represents a nitrogen atom; and
   X⁴ represents CR⁴.
[Aspect 232] The compound according to the Aspect 4, wherein
   X² represents CR²;
   X³ represents a nitrogen atom; and
   X⁴ represents CR⁴.
[Aspect 233] The compound according to the Aspect 5, wherein
   X² represents CR²;
   X³ represents a nitrogen atom; and
   X⁴ represents CR⁴.
[Aspect 234] The compound according to the Aspect 6, wherein
   X² represents CR²;
   X³ represents a nitrogen atom; and
   X⁴ represents CR⁴.
[Aspect 235] The compound according to the Aspect 7, wherein
   X² represents CR²;
   X³ represents a nitrogen atom; and
   X⁴ represents CR⁴.
[Aspect 236] The compound according to the Aspect 8, wherein
   X² represents CR²;
   X³ represents a nitrogen atom; and
   X⁴ represents CR⁴.
[Aspect 237] The compound according to the Aspect 9, wherein
   X² represents CR²;
   X³ represents a nitrogen atom; and
   X⁴ represents CR⁴.
[Aspect 238] The compound according to the Aspect 10, wherein
   X² represents CR²;
   X³ represents a nitrogen atom; and
   X⁴ represents CR⁴.
[Aspect 239] The compound according to the Aspect 11, wherein
   X² represents CR²;
   X³ represents a nitrogen atom; and
   X⁴ represents CR⁴.
[Aspect 240] The compound according to the Aspect 12, wherein
   X² represents CR²;
   X³ represents a nitrogen atom; and
   X⁴ represents CR⁴.
[Aspect 241] The compound according to the Aspect 13, wherein
   X² represents CR²;
   X³ represents a nitrogen atom; and
   X⁴ represents CR⁴.
[Aspect 242] The compound according to the Aspect 14, wherein
   X² represents CR²;
   X³ represents a nitrogen atom; and
   X⁴ represents CR⁴.
[Aspect 243] The compound according to the Aspect 15, wherein
   X² represents CR²;
   X³ represents a nitrogen atom; and
   X⁴ represents CR⁴.
[Aspect 244] The compound according to the Aspect 16, wherein
   X² represents CR²;
   X³ represents a nitrogen atom; and
   X⁴ represents CR⁴.
[Aspect 245] The compound according to the Aspect 17, wherein
   X² represents CR²;
   X³ represents a nitrogen atom; and
   X⁴ represents CR⁴.
[Aspect 246] The compound according to the Aspect 18, wherein
   X² represents CR²;
   X³ represents a nitrogen atom; and
   X⁴ represents CR⁴.
[Aspect 247] The compound according to the Aspect 19, wherein
   X² represents CR²;
   X³ represents a nitrogen atom; and
   X⁴ represents CR⁴.
[Aspect 248] The compound according to the Aspect 20, wherein
   X² represents CR²;
   X³ represents a nitrogen atom; and
   X⁴ represents CR⁴.
[Aspect 249] The compound according to the Aspect 21, wherein
   X² represents CR²;
   X³ represents a nitrogen atom; and
   X⁴ represents CR⁴.
[Aspect 250] The compound according to the Aspect 22, wherein
   X² represents CR²;
   X³ represents a nitrogen atom; and
   X⁴ represents CR⁴.
[Aspect 251] The compound according to the Aspect 23, wherein
   X² represents CR²;
   X³ represents a nitrogen atom; and
   X⁴ represents CR⁴.
[Aspect 252] The compound according to the Aspect 24, wherein
   X² represents CR²;
   X³ represents a nitrogen atom; and
   X⁴ represents CR⁴.
[Aspect 253] The compound according to the Aspect 25, wherein
   X² represents CR²;
   X³ represents a nitrogen atom; and
   X⁴ represents CR⁴.
[Aspect 254] The compound according to the Aspect 26, wherein
   X² represents CR²;
   X³ represents a nitrogen atom; and
   X⁴ represents CR⁴.
[Aspect 255] The compound according to the Aspect 27, wherein
   X² represents CR²;
   X³ represents a nitrogen atom; and
   X⁴ represents CR⁴.
[Aspect 256] The compound according to the Aspect 28, wherein
   X² represents CR²;
   X³ represents a nitrogen atom; and
   X⁴ represents CR⁴.
[Aspect 257] The compound according to the Aspect 29, wherein
   X² represents CR²;
   X³ represents a nitrogen atom; and
   X⁴ represents CR⁴.
[Aspect 258] The compound according to the Aspect 30, wherein
   X² represents CR²;
   X³ represents a nitrogen atom; and
   X⁴ represents CR⁴.
[Aspect 259] The compound according to the Aspect 31, wherein
   X² represents CR²;
   X³ represents a nitrogen atom; and
   X⁴ represents CR⁴.
[Aspect 260] The compound according to the Aspect 32, wherein
   X² represents CR²;
   X³ represents a nitrogen atom; and
   X⁴ represents CR⁴.
[Aspect 261] The compound according to the Aspect 33, wherein
   X² represents CR²;
   X³ represents a nitrogen atom; and
   X⁴ represents CR⁴.
[Aspect 262] The compound according to the Aspect 34, wherein
   X² represents CR²;
   X³ represents a nitrogen atom; and
   X⁴ represents CR⁴.
[Aspect 263] The compound according to the Aspect 35, wherein
   X² represents CR²;
   X³ represents a nitrogen atom; and
   X⁴ represents CR⁴.
[Aspect 264] The compound according to the Aspect 36, wherein
   X² represents CR²;
   X³ represents a nitrogen atom; and
   X⁴ represents CR⁴.
[Aspect 265] The compound according to the Aspect 37, wherein
   X² represents CR²;
   X³ represents a nitrogen atom; and
   X⁴ represents CR⁴.
[Aspect 266] The compound according to the Aspect 38, wherein
   X² represents CR²;
   X³ represents a nitrogen atom; and
   X⁴ represents CR⁴.
[Aspect 267] The compound according to the Aspect 39, wherein
   X² represents CR²;
   X³ represents a nitrogen atom; and
   X⁴ represents CR⁴.
[Aspect 268] The compound according to the Aspect 40, wherein
   X² represents CR²;
   X³ represents a nitrogen atom; and
   X⁴ represents CR⁴.
[Aspect 269] The compound according to the Aspect 41, wherein
   X² represents CR²;
   X³ represents a nitrogen atom; and
   X⁴ represents CR⁴.
[Aspect 270] The compound according to the Aspect 42, wherein
   X² represents CR²;
   X³ represents a nitrogen atom; and
   X⁴ represents CR⁴.
[Aspect 271] The compound according to the Aspect 43, wherein
   X² represents CR²;
   X³ represents a nitrogen atom; and
   X⁴ represents CR⁴.
[Aspect 272] The compound according to the Aspect 44, wherein
   X² represents CR²;
   X³ represents a nitrogen atom; and
   X⁴ represents CR⁴.
[Aspect 273] The compound according to the Aspect 45, wherein
   X² represents CR²;
   X³ represents a nitrogen atom; and
   X⁴ represents CR⁴.
[Aspect 274] The Present compound N, wherein
   X² represents CR²;
   X³ represents a nitrogen atom;
   X⁴ represents CR⁴; and
   R¹, R², and R⁴ each represent a hydrogen atom.
[Aspect 275] The compound according to the Aspect 1, wherein
   X² represents CR²;
   X³ represents a nitrogen atom;
   X⁴ represents CR⁴; and
   R¹, R², and R⁴ each represent a hydrogen atom.
[Aspect 276] The compound according to the Aspect 2, wherein
   X² represents CR²;
   X³ represents a nitrogen atom;
   X⁴ represents CR⁴; and
   R¹, R², and R⁴ each represent a hydrogen atom.
[Aspect 277] The compound according to the Aspect 3, wherein
   X² represents CR²;
   X³ represents a nitrogen atom;
   X⁴ represents CR⁴; and
   R¹, R², and R⁴ each represent a hydrogen atom.
[Aspect 278] The compound according to the Aspect 4, wherein
   X² represents CR²;
   X³ represents a nitrogen atom;
   X⁴ represents CR⁴; and
   R¹, R², and R⁴ each represent a hydrogen atom.
[Aspect 279] The compound according to the Aspect 5, wherein
   X² represents CR²;
   X³ represents a nitrogen atom;
   X⁴ represents CR⁴; and
   R¹, R², and R⁴ each represent a hydrogen atom.
[Aspect 280] The compound according to the Aspect 6, wherein
   X² represents CR²;
   X³ represents a nitrogen atom;
   X⁴ represents CR⁴; and
   R¹, R², and R⁴ each represent a hydrogen atom.
[Aspect 281] The compound according to the Aspect 7, wherein
   X² represents CR²;
   X³ represents a nitrogen atom;
   X⁴ represents CR⁴; and
   R¹, R², and R⁴ each represent a hydrogen atom.
[Aspect 282] The compound according to the Aspect 8, wherein
   X² represents CR²;
   X³ represents a nitrogen atom;
   X⁴ represents CR⁴; and
   R¹, R², and R⁴ each represent a hydrogen atom.
[Aspect 283] The compound according to the Aspect 9, wherein
   X² represents CR²;
   X³ represents a nitrogen atom;
   X⁴ represents CR⁴; and
   R¹, R², and R⁴ each represent a hydrogen atom.
[Aspect 284] The compound according to the Aspect 10, wherein
   X² represents CR²;
   X³ represents a nitrogen atom;
   X⁴ represents CR⁴; and
   R¹, R², and R⁴ each represent a hydrogen atom.
[Aspect 285] The compound according to the Aspect 11, wherein
   X² represents CR²;
   X³ represents a nitrogen atom;
   X⁴ represents CR⁴; and
   R¹, R², and R⁴ each represent a hydrogen atom.
[Aspect 286] The compound according to the Aspect 12, wherein
   X² represents CR²;
   X³ represents a nitrogen atom;
   X⁴ represents CR⁴; and
   R¹, R², and R⁴ each represent a hydrogen atom.
[Aspect 287] The compound according to the Aspect 13, wherein
   X² represents CR²;
   X³ represents a nitrogen atom;
   X⁴ represents CR⁴; and
   R¹, R², and R⁴ each represent a hydrogen atom.
[Aspect 288] The compound according to the Aspect 14, wherein
   X² represents CR²;
   X³ represents a nitrogen atom;
   X⁴ represents CR⁴; and
   R¹, R², and R⁴ each represent a hydrogen atom.
[Aspect 289] The compound according to the Aspect 15, wherein
   X² represents CR²;
   X³ represents a nitrogen atom;
   X⁴ represents CR⁴; and
   R¹, R², and R⁴ each represent a hydrogen atom.
[Aspect 290] The compound according to the Aspect 16, wherein
   X² represents CR²;
   X³ represents a nitrogen atom;
   X⁴ represents CR⁴; and
   R¹, R², and R⁴ each represent a hydrogen atom.
[Aspect 291] The compound according to the Aspect 17, wherein
   X² represents CR²;
   X³ represents a nitrogen atom;
   X⁴ represents CR⁴; and
   R¹, R², and R⁴ each represent a hydrogen atom.
[Aspect 292] The compound according to the Aspect 18, wherein
   X² represents CR²;
   X³ represents a nitrogen atom;
   X⁴ represents CR⁴; and
   R¹, R², and R⁴ each represent a hydrogen atom.
[Aspect 293] The compound according to the Aspect 19, wherein
   X² represents CR²;
   X³ represents a nitrogen atom;
   X⁴ represents CR⁴; and
   R¹, R², and R⁴ each represent a hydrogen atom.
[Aspect 294] The compound according to the Aspect 20, wherein
   X² represents CR²;
   X³ represents a nitrogen atom;
   X⁴ represents CR⁴; and
   R¹, R², and R⁴ each represent a hydrogen atom.
[Aspect 295] The compound according to the Aspect 21, wherein
   X² represents CR²;
   X³ represents a nitrogen atom;
   X⁴ represents CR⁴; and
   R¹, R², and R⁴ each represent a hydrogen atom.
[Aspect 296] The compound according to the Aspect 22, wherein
   X² represents CR²;
   X³ represents a nitrogen atom;
   X⁴ represents CR⁴; and
   R¹, R², and R⁴ each represent a hydrogen atom.
[Aspect 297] The compound according to the Aspect 23, wherein
   X² represents CR²;
   X³ represents a nitrogen atom;
   X⁴ represents CR⁴; and
   R¹, R², and R⁴ each represent a hydrogen atom.
[Aspect 298] The compound according to the Aspect 24, wherein
   X² represents CR²;
   X³ represents a nitrogen atom;
   X⁴ represents CR⁴; and
   R¹, R², and R⁴ each represent a hydrogen atom.
[Aspect 299] The compound according to the Aspect 25, wherein
   X² represents CR²;
   X³ represents a nitrogen atom;
   X⁴ represents CR⁴; and
   R¹, R², and R⁴ each represent a hydrogen atom.
[Aspect 300] The compound according to the Aspect 26, wherein
   X² represents CR²;
   X³ represents a nitrogen atom;
   X⁴ represents CR⁴; and
   R¹, R², and R⁴ each represent a hydrogen atom.
[Aspect 301] The compound according to the Aspect 27, wherein
   X² represents CR²;
   X³ represents a nitrogen atom;
   X⁴ represents CR⁴; and
   R¹, R², and R⁴ each represent a hydrogen atom.
[Aspect 302] The compound according to the Aspect 28, wherein
   X² represents CR²;
   X³ represents a nitrogen atom;
   X⁴ represents CR⁴; and
   R¹, R², and R⁴ each represent a hydrogen atom.
[Aspect 303] The compound according to the Aspect 29, wherein
   X² represents CR²;
   X³ represents a nitrogen atom;
   X⁴ represents CR⁴; and
   R¹, R², and R⁴ each represent a hydrogen atom.
[Aspect 304] The compound according to the Aspect 30, wherein
   X² represents CR²;
   X³ represents a nitrogen atom;
   X⁴ represents CR⁴; and
   R¹, R², and R⁴ each represent a hydrogen atom.
[Aspect 305] The compound according to the Aspect 31, wherein
   X² represents CR²;
   X³ represents a nitrogen atom;
   X⁴ represents CR⁴; and
   R¹, R², and R⁴ each represent a hydrogen atom.
[Aspect 306] The compound according to the Aspect 32, wherein
   X² represents CR²;
   X³ represents a nitrogen atom;
   X⁴ represents CR⁴; and
   R¹, R², and R⁴ each represent a hydrogen atom.
[Aspect 307] The compound according to the Aspect 33, wherein
   X² represents CR²;
   X³ represents a nitrogen atom;
   X⁴ represents CR⁴; and
   R¹, R², and R⁴ each represent a hydrogen atom.
[Aspect 308] The compound according to the Aspect 34, wherein
   X² represents CR²;
   X³ represents a nitrogen atom;
   X⁴ represents CR⁴; and
   R¹, R², and R⁴ each represent a hydrogen atom.
[Aspect 309] The compound according to the Aspect 35, wherein
   X² represents CR²;
   X³ represents a nitrogen atom;
   X⁴ represents CR⁴; and
   R¹, R², and R⁴ each represent a hydrogen atom.
[Aspect 310] The compound according to the Aspect 36, wherein
   X² represents CR²;
   X³ represents a nitrogen atom;
   X⁴ represents CR⁴; and
   R¹, R², and R⁴ each represent a hydrogen atom.
[Aspect 311] The compound according to the Aspect 37, wherein
   X² represents CR²;
   X³ represents a nitrogen atom;
   X⁴ represents CR⁴; and
   R¹, R², and R⁴ each represent a hydrogen atom.
[Aspect 312] The compound according to the Aspect 38, wherein
   X² represents CR²;
   X³ represents a nitrogen atom;
   X⁴ represents CR⁴; and
   R¹, R², and R⁴ each represent a hydrogen atom.
[Aspect 313] The compound according to the Aspect 39, wherein
   X² represents CR²;
   X³ represents a nitrogen atom;
   X⁴ represents CR⁴; and
   R¹, R², and R⁴ each represent a hydrogen atom.
[Aspect 314] The compound according to the Aspect 40, wherein
   X² represents CR²;
   X³ represents a nitrogen atom;
   X⁴ represents CR⁴; and
   R¹, R², and R⁴ each represent a hydrogen atom.
[Aspect 315] The compound according to the Aspect 41, wherein
   X² represents CR²;
   X³ represents a nitrogen atom;
   X⁴ represents CR⁴; and
   R¹, R², and R⁴ each represent a hydrogen atom.
[Aspect 316] The compound according to the Aspect 42, wherein
   X² represents CR²;
   X³ represents a nitrogen atom;
   X⁴ represents CR⁴; and
   R¹, R², and R⁴ each represent a hydrogen atom.
[Aspect 317] The compound according to the Aspect 43, wherein
   X² represents CR²;
   X³ represents a nitrogen atom;
   X⁴ represents CR⁴; and
   R¹, R², and R⁴ each represent a hydrogen atom.
[Aspect 318] The compound according to the Aspect 44, wherein
   X² represents CR²;
   X³ represents a nitrogen atom;
   X⁴ represents CR⁴; and
   R¹, R², and R⁴ each represent a hydrogen atom.
[Aspect 319] The compound according to the Aspect 45, wherein
   X² represents CR²;
   X³ represents a nitrogen atom;
   X⁴ represents CR⁴; and
   R¹, R², and R⁴ each represent a hydrogen atom.
[Aspect 320] The Present compound N, wherein
   X¹ represents CR¹;
   X² represents CR²; and
   X⁴ represents CR⁴.
[Aspect 321] The Present compound N, wherein
   X¹ represents CR¹;
   X² represents CR²;
   X⁴ represents CR⁴; and
   R¹, R², R³, and R⁴ each represent a hydrogen atom.
[Aspect 322] The compound according to the Aspect 1, wherein
   X¹ represents CR¹;
   X² represents CR²;
   X⁴ represents CR⁴; and
   R¹, R², R³, and R⁴ each represent a hydrogen atom.
[Aspect 323] The compound according to the Aspect 2, wherein
   X¹ represents CR¹;
   X² represents CR²;
   X⁴ represents CR⁴; and
   R¹, R², R³, and R⁴ each represent a hydrogen atom.
[Aspect 324] The compound according to the Aspect 3, wherein
   X¹ represents CR¹;
   X² represents CR²;
   X⁴ represents CR⁴; and
   R¹, R², R³, and R⁴ each represent a hydrogen atom.
[Aspect 325] The compound according to the Aspect 4, wherein
   X¹ represents CR¹;
   X² represents CR²;
   X⁴ represents CR⁴; and
   R¹, R², R³, and R⁴ each represent a hydrogen atom.
[Aspect 326] The compound according to the Aspect 5, wherein
   X¹ represents CR¹;
   X² represents CR²;
   X⁴ represents CR⁴; and
   R¹, R², R³, and R⁴ each represent a hydrogen atom.
[Aspect 327] The compound according to the Aspect 6, wherein
   X¹ represents CR¹;
   X² represents CR²;
   X⁴ represents CR⁴; and
   R¹, R², R³, and R⁴ each represent a hydrogen atom.
[Aspect 328] The compound according to the Aspect 7, wherein
   X¹ represents CR¹;
   X² represents CR²;
   X⁴ represents CR⁴; and
   R¹, R², R³, and R⁴ each represent a hydrogen atom.
[Aspect 329] The compound according to the Aspect 8, wherein
   X¹ represents CR¹;
   X² represents CR²;
   X⁴ represents CR⁴; and
   R¹, R², R³, and R⁴ each represent a hydrogen atom.
[Aspect 330] The compound according to the Aspect 9, wherein
   X¹ represents CR¹;
   X² represents CR²;
   X⁴ represents CR⁴; and
   R¹, R², R³, and R⁴ each represent a hydrogen atom.
[Aspect 331] The compound according to the Aspect 10, wherein
   X¹ represents CR¹;
   X² represents CR²;
   X⁴ represents CR⁴; and
   R¹, R², R³, and R⁴ each represent a hydrogen atom.
[Aspect 332] The compound according to the Aspect 11, wherein
   X¹ represents CR¹;
   X² represents CR²;
   X⁴ represents CR⁴; and
   R¹, R², R³, and R⁴ each represent a hydrogen atom.
[Aspect 333] The compound according to the Aspect 12, wherein
   X¹ represents CR¹;
   X² represents CR²;
   X⁴ represents CR⁴; and
   R¹, R², R³, and R⁴ each represent a hydrogen atom.
[Aspect 334] The compound according to the Aspect 13, wherein
   X¹ represents CR¹;
   X² represents CR²;
   X⁴ represents CR⁴; and
   R¹, R², R³, and R⁴ each represent a hydrogen atom.
[Aspect 335] The compound according to the Aspect 14, wherein
   X¹ represents CR¹;
   X² represents CR²;
   X⁴ represents CR⁴; and
   R¹, R², R³, and R⁴ each represent a hydrogen atom.
[Aspect 336] The compound according to the Aspect 15, wherein
   X¹ represents CR¹;
   X² represents CR²;
   X⁴ represents CR⁴; and
   R¹, R², R³, and R⁴ each represent a hydrogen atom.
[Aspect 337] The compound according to the Aspect 16, wherein
   X¹ represents CR¹;
   X² represents CR²;
   X⁴ represents CR⁴; and
   R¹, R², R³, and R⁴ each represent a hydrogen atom.
[Aspect 338] The compound according to the Aspect 17, wherein
   X¹ represents CR¹;
   X² represents CR²;
   X⁴ represents CR⁴; and
   R¹, R², R³, and R⁴ each represent a hydrogen atom.
[Aspect 339] The compound according to the Aspect 18, wherein
   X¹ represents CR¹;
   X² represents CR²;
   X⁴ represents CR⁴; and
   R¹, R², R³, and R⁴ each represent a hydrogen atom.
[Aspect 340] The compound according to the Aspect 19, wherein
   X¹ represents CR¹;
   X² represents CR²;
   X⁴ represents CR⁴; and
   R¹, R², R³, and R⁴ each represent a hydrogen atom.
[Aspect 341] The compound according to the Aspect 20, wherein
   X¹ represents CR¹;
   X² represents CR²;
   X⁴ represents CR⁴; and
   R¹, R², R³, and R⁴ each represent a hydrogen atom.
[Aspect 342] The compound according to the Aspect 21, wherein
   X¹ represents CR¹;
   X² represents CR²;
   X⁴ represents CR⁴; and
   R¹, R², R³, and R⁴ each represent a hydrogen atom.
[Aspect 343] The compound according to the Aspect 22, wherein
   X¹ represents CR¹;
   X² represents CR²;
   X⁴ represents CR⁴; and
   R¹, R², R³, and R⁴ each represent a hydrogen atom.
[Aspect 344] The compound according to the Aspect 23, wherein
   X¹ represents CR¹;
   X² represents CR²;
   X⁴ represents CR⁴; and
   R¹, R², R³, and R⁴ each represent a hydrogen atom.
[Aspect 345] The compound according to the Aspect 24, wherein
   X¹ represents CR¹;
   X² represents CR²;
   X⁴ represents CR⁴; and
   R¹, R², R³, and R⁴ each represent a hydrogen atom.
[Aspect 346] The compound according to the Aspect 25, wherein
   X¹ represents CR¹;
   X² represents CR²;
   X⁴ represents CR⁴; and
   R¹, R², R³, and R⁴ each represent a hydrogen atom.
[Aspect 347] The compound according to the Aspect 26, wherein
   X¹ represents CR¹;
   X² represents CR²;
   X⁴ represents CR⁴; and
   R¹, R², R³, and R⁴ each represent a hydrogen atom.
[Aspect 348] The compound according to the Aspect 27, wherein
   X¹ represents CR¹;
   X² represents CR²;
   X⁴ represents CR⁴; and
   R¹, R², R³, and R⁴ each represent a hydrogen atom.
[Aspect 349] The compound according to the Aspect 28, wherein
   X¹ represents CR¹;
   X² represents CR²;
   X⁴ represents CR⁴; and
   R¹, R², R³, and R⁴ each represent a hydrogen atom.
[Aspect 350] The compound according to the Aspect 29, wherein
   X¹ represents CR¹;
   X² represents CR²;
   X⁴ represents CR⁴; and
   R¹, R², R³, and R⁴ each represent a hydrogen atom.
[Aspect 351] The compound according to the Aspect 33, wherein
   X¹ represents CR¹;
   X² represents CR²;
   X⁴ represents CR⁴; and
   R¹, R², R³, and R⁴ each represent a hydrogen atom.
[Aspect 352] The compound according to the Aspect 34, wherein
   X¹ represents CR¹;
   X² represents CR²;
   X⁴ represents CR⁴; and
   R¹, R², R³, and R⁴ each represent a hydrogen atom.
[Aspect 353] The compound according to the Aspect 35, wherein
   X¹ represents CR¹;
   X² represents CR²;
   X⁴ represents CR⁴; and
   R¹, R², R³, and R⁴ each represent a hydrogen atom.
[Aspect 354] The compound according to the Aspect 36, wherein
   X¹ represents CR¹;
   X² represents CR²;
   X⁴ represents CR⁴; and
   R¹, R², R³, and R⁴ each represent a hydrogen atom.
[Aspect 355] The compound according to the Aspect 37, wherein
   X¹ represents CR¹;
   X² represents CR²;
   X⁴ represents CR⁴; and
   R¹, R², R³, and R⁴ each represent a hydrogen atom.
[Aspect 356] The compound according to the Aspect 38, wherein
   X¹ represents CR¹;
   X² represents CR²;
   X⁴ represents CR⁴; and
   R¹, R², R³, and R⁴ each represent a hydrogen atom.
[Aspect 357] The compound according to the Aspect 39, wherein
   X¹ represents CR¹;
   X² represents CR²;
   X⁴ represents CR⁴; and
   R¹, R², R³, and R⁴ each represent a hydrogen atom.
[Aspect 358] The compound according to the Aspect 40, wherein
   X¹ represents CR¹;
   X² represents CR²;
   X⁴ represents CR⁴; and
   R¹, R², R³, and R⁴ each represent a hydrogen atom.
[Aspect 359] The compound according to the Aspect 41, wherein
   X¹ represents CR¹;
   X² represents CR²;
   X⁴ represents CR⁴; and
   R¹, R², R³, and R⁴ each represent a hydrogen atom.
[Aspect 360] The compound according to the Aspect 42, wherein
   X¹ represents CR¹;
   X² represents CR²;
   X⁴ represents CR⁴; and
   R¹, R², R³, and R⁴ each represent a hydrogen atom.
[Aspect 361] The compound according to the Aspect 43, wherein
   X¹ represents CR¹;
   X² represents CR²;
   X⁴ represents CR⁴; and
   R¹, R², R³, and R⁴ each represent a hydrogen atom.
[Aspect 362] The compound according to the Aspect 44, wherein
   X¹ represents CR¹;
   X² represents CR²;
   X⁴ represents CR⁴; and
   R¹, R², R³, and R⁴ each represent a hydrogen atom.
[Aspect 363] The compound according to the Aspect 45, wherein
   X¹ represents CR¹;
   X² represents CR²;
   X⁴ represents CR⁴; and
   R¹, R², R³, and R⁴ each represent a hydrogen atom.
[Aspect 364] The Present compound N, wherein n represents 0.
[Aspect 365] The Present compound N, wherein n represents 1.
[Aspect 366] The Present compound N, wherein n represents 2.
[Aspect 367] The Present compound N, wherein n represents 1 or 2.
[Aspect 368] The compound according to the Aspect 1, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 369] The compound according to the Aspect 2, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 370] The compound according to the Aspect 3, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 371] The compound according to the Aspect 4, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 372] The compound according to the Aspect 5, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 373] The compound according to the Aspect 6, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 374] The compound according to the Aspect 7, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 375] The compound according to the Aspect 8, wherein
   X¹ represents CR¹;
   X² represents CR²;
   X³ represents a nitrogen atom;
   X⁴ represents CR⁴;
   R¹, R², and R⁴ each represent a hydrogen atom; and n represents 2.
[Aspect 376] The compound according to the Aspect 9, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 377] The compound according to the Aspect 10, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 378] The compound according to the Aspect 11, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 379] The compound according to the Aspect 12, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 380] The compound according to the Aspect 13, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 381] The compound according to the Aspect 14, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 382] The compound according to the Aspect 15, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 383] The compound according to the Aspect 16, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 384] The compound according to the Aspect 17, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 385] The compound according to the Aspect 18, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 386] The compound according to the Aspect 19, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 387] The compound according to the Aspect 20, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 388] The compound according to the Aspect 21, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 389] The compound according to the Aspect 22, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 390] The compound according to the Aspect 23, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 391] The compound according to the Aspect 24, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 392] The compound according to the Aspect 25, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 393] The compound according to the Aspect 26, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 394] The compound according to the Aspect 27, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 395] The compound according to the Aspect 28, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 396] The compound according to the Aspect 29, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 397] The compound according to the Aspect 30, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 398] The compound according to the Aspect 31, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 399] The compound according to the Aspect 32, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 400] The compound according to the Aspect 33, wherein
   X¹ represents CR¹;
   X² represents CR²;
   X³ represents a nitrogen atom;
   X⁴ represents CR⁴;
   R¹, R², and R⁴ each represent a hydrogen atom; and n represents 2.
[Aspect 401] The compound according to the Aspect 34, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 402] The compound according to the Aspect 35, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 403] The compound according to the Aspect 36, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 404] The compound according to the Aspect 37, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 405] The compound according to the Aspect 38, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 406] The compound according to the Aspect 39, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 407] The compound according to the Aspect 40, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 408] The compound according to the Aspect 41, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 409] The compound according to the Aspect 42, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 410] The compound according to the Aspect 43, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 411] The compound according to the Aspect 44, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 412] The compound according to the Aspect 45, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 413] The compound according to the Aspect 82, wherein
   X¹ represents CR¹;
   X² represents CR²;
   X³ represents a nitrogen atom;
   X⁴ represents CR⁴;
   R¹, R², and R⁴ each represent a hydrogen atom; and
   n represents 2.
[Aspect 414] The compound according to the Aspect 1, wherein
   X² represents CR²;
   X⁴ represents CR⁴;
   R¹, R², R³, and R⁴ each represent a hydrogen atom; and
   n represents 2.
[Aspect 415] The compound according to the Aspect 2, wherein
   X² represents CR²;
   X⁴ represents CR⁴;
   R¹, R², R³, and R⁴ each represent a hydrogen atom; and
   n represents 2.
[Aspect 416] The compound according to the Aspect 3, wherein
   X² represents CR²;
   X⁴ represents CR⁴;
   R¹, R², R³, and R⁴ each represent a hydrogen atom; and
   n represents 2.
[Aspect 417] The compound according to the Aspect 4, wherein
   X² represents CR²;
   X⁴ represents CR⁴;
   R¹, R², R³, and R⁴ each represent a hydrogen atom; and
   n represents 2.
[Aspect 418] The compound according to the Aspect 5, wherein
   X² represents CR²;
   X⁴ represents CR⁴;
   R¹, R², R³, and R⁴ each represent a hydrogen atom; and
   n represents 2.
[Aspect 419] The compound according to the Aspect 6, wherein
   X² represents CR²;
   X⁴ represents CR⁴;
   R¹, R², R³, and R⁴ each represent a hydrogen atom; and
   n represents 2.
[Aspect 420] The compound according to the Aspect 7, wherein
   X² represents CR²;
   X⁴ represents CR⁴;
   R¹, R², R³, and R⁴ each represent a hydrogen atom; and
   n represents 2.
[Aspect 421] The compound according to the Aspect 8, wherein
   X² represents CR²;
   X⁴ represents CR⁴;
   R¹, R², R³, and R⁴ each represent a hydrogen atom; and
   n represents 2.
[Aspect 422] The compound according to the Aspect 9, wherein
   X² represents CR²;
   X⁴ represents CR⁴;
   R¹, R², R³, and R⁴ each represent a hydrogen atom; and
   n represents 2.
[Aspect 423] The compound according to the Aspect 10, wherein
   X² represents CR²;
   X⁴ represents CR⁴;
   R¹, R², R³, and R⁴ each represent a hydrogen atom; and
   n represents 2.
[Aspect 424] The compound according to the Aspect 11, wherein
   X² represents CR²;
   X⁴ represents CR⁴;
   R¹, R², R³, and R⁴ each represent a hydrogen atom; and
   n represents 2.
[Aspect 425] The compound according to the Aspect 12, wherein
   X² represents CR²;
   X⁴ represents CR⁴;
   R¹, R², R³, and R⁴ each represent a hydrogen atom; and
   n represents 2.
[Aspect 426] The compound according to the Aspect 13, wherein
   X² represents CR²;
   X⁴ represents CR⁴;
   R¹, R², R³, and R⁴ each represent a hydrogen atom; and
   n represents 2.
[Aspect 427] The compound according to the Aspect 14, wherein
   X² represents CR²;
   X⁴ represents CR⁴;
   R¹, R², R³, and R⁴ each represent a hydrogen atom; and
   n represents 2.
[Aspect 428] The compound according to the Aspect 15, wherein
   X² represents CR²;
   X⁴ represents CR⁴;
   R¹, R², R³, and R⁴ each represent a hydrogen atom; and
   n represents 2.
[Aspect 429] The compound according to the Aspect 16, wherein
   X² represents CR²;
   X⁴ represents CR⁴;
   R¹, R², R³, and R⁴ each represent a hydrogen atom; and
   n represents 2.
[Aspect 430] The compound according to the Aspect 17, wherein
   X² represents CR²;
   X⁴ represents CR⁴;
   R¹, R², R³, and R⁴ each represent a hydrogen atom; and
   n represents 2.
[Aspect 431] The compound according to the Aspect 18, wherein
   X² represents CR²;
   X⁴ represents CR⁴;
   R¹, R², R³, and R⁴ each represent a hydrogen atom; and
   n represents 2.
[Aspect 432] The compound according to the Aspect 19, wherein
   X² represents CR²;
   X⁴ represents CR⁴;
   R¹, R², R³, and R⁴ each represent a hydrogen atom; and
   n represents 2.
[Aspect 433] The compound according to the Aspect 20, wherein
   X² represents CR²;
   X⁴ represents CR⁴;
   R¹, R², R³, and R⁴ each represent a hydrogen atom; and
   n represents 2.
[Aspect 434] The compound according to the Aspect 21, wherein
   X² represents CR²;
   X⁴ represents CR⁴;
   R¹, R², R³, and R⁴ each represent a hydrogen atom; and
   n represents 2.
[Aspect 435] The compound according to the Aspect 22, wherein
   X² represents CR²;
   X⁴ represents CR⁴;
   R¹, R², R³, and R⁴ each represent a hydrogen atom; and
   n represents 2.
[Aspect 436] The compound according to the Aspect 23, wherein
   X² represents CR²;
   X⁴ represents CR⁴;
   R¹, R², R³, and R⁴ each represent a hydrogen atom; and
   n represents 2.
[Aspect 437] The compound according to the Aspect 24, wherein
   X² represents CR²;
   X⁴ represents CR⁴;
   R¹, R², R³, and R⁴ each represent a hydrogen atom; and
   n represents 2.
[Aspect 438] The compound according to the Aspect 25, wherein
   X² represents CR²;
   X⁴ represents CR⁴;
   R¹, R², R³, and R⁴ each represent a hydrogen atom; and
   n represents 2.
[Aspect 439] The compound according to the Aspect 26, wherein
   X² represents CR²;
   X⁴ represents CR⁴;
   R¹, R², R³, and R⁴ each represent a hydrogen atom; and
   n represents 2.
[Aspect 440] The compound according to the Aspect 27, wherein
   X² represents CR²;
   X⁴ represents CR⁴;
   R¹, R², R³, and R⁴ each represent a hydrogen atom; and
   n represents 2.
[Aspect 441] The compound according to the Aspect 28, wherein
   X² represents CR²;
   X⁴ represents CR⁴;
   R¹, R², R³, and R⁴ each represent a hydrogen atom; and
   n represents 2.
[Aspect 442] The compound according to the Aspect 29, wherein
   X² represents CR²;
   X⁴ represents CR⁴;
   R¹, R², R³, and R⁴ each represent a hydrogen atom; and
   n represents 2.
[Aspect 443] The compound according to the Aspect 33, wherein
   X² represents CR²;
   X⁴ represents CR⁴;
   R¹, R², R³, and R⁴ each represent a hydrogen atom; and
   n represents 2.
[Aspect 444] The compound according to the Aspect 34, wherein
   X² represents CR²;
   X⁴ represents CR⁴;
   R¹, R², R³, and R⁴ each represent a hydrogen atom; and
   n represents 2.
[Aspect 445] The compound according to the Aspect 35, wherein
   X² represents CR²;
   X⁴ represents CR⁴;
   R¹, R², R³, and R⁴ each represent a hydrogen atom; and
   n represents 2.
[Aspect 446] The compound according to the Aspect 36, wherein
   X² represents CR²;
   X⁴ represents CR⁴;
   R¹, R², R³, and R⁴ each represent a hydrogen atom; and
   n represents 2.
[Aspect 447] The compound according to the Aspect 37, wherein
   X² represents CR²;
   X⁴ represents CR⁴;
   R¹, R², R³, and R⁴ each represent a hydrogen atom; and
   n represents 2.
[Aspect 448] The compound according to the Aspect 38, wherein
   X² represents CR²;
   X⁴ represents CR⁴;
   R¹, R², R³, and R⁴ each represent a hydrogen atom; and
   n represents 2.
[Aspect 449] The compound according to the Aspect 39, wherein
   X² represents CR²;
   X⁴ represents CR⁴;
   R¹, R², R³, and R⁴ each represent a hydrogen atom; and
   n represents 2.
[Aspect 450] The compound according to the Aspect 40, wherein
   X² represents CR²;
   X⁴ represents CR⁴;
   R¹, R², R³, and R⁴ each represent a hydrogen atom; and
   n represents 2.
[Aspect 451] The compound according to the Aspect 41, wherein
   X² represents CR²;
   X⁴ represents CR⁴;
   R¹, R², R³, and R⁴ each represent a hydrogen atom; and
   n represents 2.
[Aspect 452] The compound according to the Aspect 42, wherein
   X² represents CR²;
   X⁴ represents CR⁴;
   R¹, R², R³, and R⁴ each represent a hydrogen atom; and
   n represents 2.
[Aspect 453] The compound according to the Aspect 43, wherein
   X² represents CR²;
   X⁴ represents CR⁴;
   R¹, R², R³, and R⁴ each represent a hydrogen atom; and
   n represents 2.
[Aspect 454] The compound according to the Aspect 44, wherein
   X² represents CR²;
   X⁴ represents CR⁴;
   R¹, R², R³, and R⁴ each represent a hydrogen atom; and
   n represents 2.
[Aspect 455] The compound according to the Aspect 45, wherein
   X² represents CR²;
   X⁴ represents CR⁴;
   R¹, R², R³, and R⁴ each represent a hydrogen atom; and
   n represents 2.
[Aspect 456] The compound according to the Aspect 185, wherein
   X² represents CR²;
   X⁴ represents CR⁴;
   R¹, R², R³, and R⁴ each represent a hydrogen atom; and
   n represents 2.
[Aspect 457] The compound according to the Aspect 1, wherein
   X² represents CR²;
   X³ represents a nitrogen atom;
   X⁴ represents CR⁴;
   R¹, R², and R⁴ each represent a hydrogen atom; and
   n represents 2.
[Aspect 458] The compound according to the Aspect 2, wherein
   X² represents CR²;
   X³ represents a nitrogen atom;
   X⁴ represents CR⁴;
   R¹, R², and R⁴ each represent a hydrogen atom; and
   n represents 2.
[Aspect 459] The compound according to the Aspect 3, wherein
   X² represents CR²;
   X³ represents a nitrogen atom;
   X⁴ represents CR⁴;
   R¹, R², and R⁴ each represent a hydrogen atom; and
   n represents 2.
[Aspect 460] The compound according to the Aspect 4, wherein
   X² represents CR²;
   X³ represents a nitrogen atom;
   X⁴ represents CR⁴;
   R¹, R², and R⁴ each represent a hydrogen atom; and
   n represents 2.
[Aspect 461] The compound according to the Aspect 5, wherein
   X² represents CR²;
   X³ represents a nitrogen atom;
   X⁴ represents CR⁴;
   R¹, R², and R⁴ each represent a hydrogen atom; and
   n represents 2.
[Aspect 462] The compound according to the Aspect 6, wherein
   X² represents CR²;
   X³ represents a nitrogen atom;
   X⁴ represents CR⁴;
   R¹, R², and R⁴ each represent a hydrogen atom; and
   n represents 2.
[Aspect 463] The compound according to the Aspect 7, wherein
   X² represents CR²;
   X³ represents a nitrogen atom;
   X⁴ represents CR⁴;
   R¹, R², and R⁴ each represent a hydrogen atom; and
   n represents 2.
[Aspect 464] The compound according to the Aspect 8, wherein
   X² represents CR²;
   X³ represents a nitrogen atom;
   X⁴ represents CR⁴;
   R¹, R², and R⁴ each represent a hydrogen atom; and
   n represents 2.
[Aspect 465] The compound according to the Aspect 9, wherein
   X² represents CR²;
   X³ represents a nitrogen atom;
   X⁴ represents CR⁴;
   R¹, R², and R⁴ each represent a hydrogen atom; and
   n represents 2.
[Aspect 466] The compound according to the Aspect 10, wherein
   X² represents CR²;
   X³ represents a nitrogen atom;
   X⁴ represents CR⁴;
   R¹, R², and R⁴ each represent a hydrogen atom; and
   n represents 2.
[Aspect 467] The compound according to the Aspect 11, wherein
   X² represents CR²;
   X³ represents a nitrogen atom;
   X⁴ represents CR⁴;
   R¹, R², and R⁴ each represent a hydrogen atom; and
   n represents 2.
[Aspect 468] The compound according to the Aspect 12, wherein
   X² represents CR²;
   X³ represents a nitrogen atom;
   X⁴ represents CR⁴;
   R¹, R², and R⁴ each represent a hydrogen atom; and
   n represents 2.
[Aspect 469] The compound according to the Aspect 13, wherein
   X² represents CR²;
   X³ represents a nitrogen atom;
   X⁴ represents CR⁴;
   R¹, R², and R⁴ each represent a hydrogen atom; and
   n represents 2.
[Aspect 470] The compound according to the Aspect 14, wherein
   X² represents CR²;
   X³ represents a nitrogen atom;
   X⁴ represents CR⁴;
   R¹, R², and R⁴ each represent a hydrogen atom; and
   n represents 2.
[Aspect 471] The compound according to the Aspect 15, wherein
   X² represents CR²;
   X³ represents a nitrogen atom;
   X⁴ represents CR⁴;
   R¹, R², and R⁴ each represent a hydrogen atom; and
   n represents 2.
[Aspect 472] The compound according to the Aspect 16, wherein
   X² represents CR²;
   X³ represents a nitrogen atom;
   X⁴ represents CR⁴;
   R¹, R², and R⁴ each represent a hydrogen atom; and
   n represents 2.
[Aspect 473] The compound according to the Aspect 17, wherein
   X² represents CR²;
   X³ represents a nitrogen atom;
   X⁴ represents CR⁴;
   R¹, R², and R⁴ each represent a hydrogen atom; and
   n represents 2.
[Aspect 474] The compound according to the Aspect 18, wherein
   X² represents CR²;
   X³ represents a nitrogen atom;
   X⁴ represents CR⁴;
   R¹, R², and R⁴ each represent a hydrogen atom; and
   n represents 2.
[Aspect 475] The compound according to the Aspect 19, wherein
   X² represents CR²;
   X³ represents a nitrogen atom;
   X⁴ represents CR⁴;
   R¹, R², and R⁴ each represent a hydrogen atom; and
   n represents 2.
[Aspect 476] The compound according to the Aspect 20, wherein
   X² represents CR²;
   X³ represents a nitrogen atom;
   X⁴ represents CR⁴;
   R¹, R², and R⁴ each represent a hydrogen atom; and
   n represents 2.
[Aspect 477] The compound according to the Aspect 21, wherein
   X² represents CR²;
   X³ represents a nitrogen atom;
   X⁴ represents CR⁴;
   R¹, R², and R⁴ each represent a hydrogen atom; and
   n represents 2.
[Aspect 478] The compound according to the Aspect 22, wherein
   X² represents CR²;
   X³ represents a nitrogen atom;
   X⁴ represents CR⁴;
   R¹, R², and R⁴ each represent a hydrogen atom; and
   n represents 2.
[Aspect 479] The compound according to the Aspect 23, wherein
   X² represents CR²;
   X³ represents a nitrogen atom;
   X⁴ represents CR⁴;
   R¹, R², and R⁴ each represent a hydrogen atom; and
   n represents 2.
[Aspect 480] The compound according to the Aspect 24, wherein
   X² represents CR²;
   X³ represents a nitrogen atom;
   X⁴ represents CR⁴;
   R¹, R², and R⁴ each represent a hydrogen atom; and
   n represents 2.
[Aspect 481] The compound according to the Aspect 25, wherein
   X² represents CR²;
   X³ represents a nitrogen atom;
   X⁴ represents CR⁴;
   R¹, R², and R⁴ each represent a hydrogen atom; and
   n represents 2.
[Aspect 482] The compound according to the Aspect 26, wherein
   X² represents CR²;
   X³ represents a nitrogen atom;
   X⁴ represents CR⁴;
   R¹, R², and R⁴ each represent a hydrogen atom; and
   n represents 2.
[Aspect 483] The compound according to the Aspect 27, wherein
   X² represents CR²;
   X³ represents a nitrogen atom;
   X⁴ represents CR⁴;
   R¹, R², and R⁴ each represent a hydrogen atom; and
   n represents 2.
[Aspect 484] The compound according to the Aspect 28, wherein
   X² represents CR²;
   X³ represents a nitrogen atom;
   X⁴ represents CR⁴;
   R¹, R², and R⁴ each represent a hydrogen atom; and
   n represents 2.
[Aspect 485] The compound according to the Aspect 29, wherein
   X² represents CR²;
   X³ represents a nitrogen atom;
   X⁴ represents CR⁴;
   R¹, R², and R⁴ each represent a hydrogen atom; and
   n represents 2.
[Aspect 486] The compound according to the Aspect 30, wherein
   X² represents CR²;
   X³ represents a nitrogen atom;
   X⁴ represents CR⁴;
   R¹, R², and R⁴ each represent a hydrogen atom; and
   n represents 2.
[Aspect 487] The compound according to the Aspect 31, wherein
   X² represents CR²;
   X³ represents a nitrogen atom;
   X⁴ represents CR⁴;
   R¹, R², and R⁴ each represent a hydrogen atom; and
   n represents 2.
[Aspect 488] The compound according to the Aspect 32, wherein
   X² represents CR²;
   X³ represents a nitrogen atom;
   X⁴ represents CR⁴;
   R¹, R², and R⁴ each represent a hydrogen atom; and
   n represents 2.
[Aspect 489] The compound according to the Aspect 33, wherein
   X² represents CR²;
   X³ represents a nitrogen atom;
   X⁴ represents CR⁴;
   R¹, R², and R⁴ each represent a hydrogen atom; and
   n represents 2.
[Aspect 490] The compound according to the Aspect 34, wherein
   X² represents CR²;
   X³ represents a nitrogen atom;
   X⁴ represents CR⁴;
   R¹, R², and R⁴ each represent a hydrogen atom; and
   n represents 2.
[Aspect 491] The compound according to the Aspect 35, wherein
   X² represents CR²;
   X³ represents a nitrogen atom;
   X⁴ represents CR⁴;
   R¹, R², and R⁴ each represent a hydrogen atom; and
   n represents 2.
[Aspect 492] The compound according to the Aspect 36, wherein
   X² represents CR²;
   X³ represents a nitrogen atom;
   X⁴ represents CR⁴;
   R¹, R², and R⁴ each represent a hydrogen atom; and
   n represents 2.
[Aspect 493] The compound according to the Aspect 37, wherein
   X² represents CR²;
   X³ represents a nitrogen atom;
   X⁴ represents CR⁴;
   R¹, R², and R⁴ each represent a hydrogen atom; and
   n represents 2.
[Aspect 494] The compound according to the Aspect 38, wherein
   X² represents CR²;
   X³ represents a nitrogen atom;
   X⁴ represents CR⁴;
   R¹, R², and R⁴ each represent a hydrogen atom; and
   n represents 2.
[Aspect 495] The compound according to the Aspect 39, wherein
   X² represents CR²;
   X³ represents a nitrogen atom;
   X⁴ represents CR⁴;
   R¹, R², and R⁴ each represent a hydrogen atom; and
   n represents 2.
[Aspect 496] The compound according to the Aspect 40, wherein
   X² represents CR²;
   X³ represents a nitrogen atom;
   X⁴ represents CR⁴;
   R¹, R², and R⁴ each represent a hydrogen atom; and
   n represents 2.
[Aspect 497] The compound according to the Aspect 41, wherein
   X² represents CR²;
   X³ represents a nitrogen atom;
   X⁴ represents CR⁴;
   R¹, R², and R⁴ each represent a hydrogen atom; and
   n represents 2.
[Aspect 498] The compound according to the Aspect 42, wherein
   X² represents CR²;
   X³ represents a nitrogen atom;
   X⁴ represents CR⁴;
   R¹, R², and R⁴ each represent a hydrogen atom; and
   n represents 2.
[Aspect 499] The compound according to the Aspect 43, wherein
   X² represents CR²;
   X³ represents a nitrogen atom;
   X⁴ represents CR⁴;
   R¹, R², and R⁴ each represent a hydrogen atom; and
   n represents 2.
[Aspect 500] The compound according to the Aspect 44, wherein
   X² represents CR²;
   X³ represents a nitrogen atom;
   X⁴ represents CR⁴;
   R¹, R², and R⁴ each represent a hydrogen atom; and
   n represents 2.
[Aspect 501] The compound according to the Aspect 45, wherein
   X² represents CR²;
   X³ represents a nitrogen atom;
   X⁴ represents CR⁴;
   R¹, R², and R⁴ each represent a hydrogen atom; and
   n represents 2.
[Aspect 502] The compound according to the Aspect 274, wherein
   X² represents CR²;
   X³ represents a nitrogen atom;
   X⁴ represents CR⁴;
   R¹, R², and R⁴ each represent a hydrogen atom; and
   n represents 2.
[Aspect 503] The Present compound N, wherein X³ represents CR³.
[Aspect 504] The Present compound N, wherein X³ represents a nitrogen atom.
Aspects of the Compound N of the present invention include the following compounds.
[Aspect C1] The Compound N of the present invention, wherein Z^{a} represents a C2-C6 chain hydrocarbon group.
[Aspect C2] The Compound N of the present invention, wherein Z^{a} represents a C2-C6 alkyl group.
[Aspect C3] The Compound N of the present invention, wherein Z^{a} represents an ethyl group or a propyl group.
[Aspect C4] The Compound N of the present invention, wherein Z^{a} represents a C1-C2 alkyl group {wherein said C1-C2 alkyl group is substituted with a methoxy group or an ethoxy group}.
[Aspect C5] The Compound N of the present invention, wherein Z^{a} represents a 2-methoxyethyl group or a 2-ethoxyethyl group.
[Aspect C6] The Compound N of the present invention, wherein Z^{a} represents a C3-C8 alicyclic hydrocarbon group optionally substituted with one or more substituent(s) selected from Group A^{a}.
[Aspect C7] The Compound N of the present invention, wherein Z^{a} represents a C3-C8 alicyclic hydrocarbon group.
[Aspect C8] The Compound N of the present invention, wherein Z^{a} represents a cyclopropyl group, a cyclopentyl group, or a cyclohexyl group.
[Aspect C9] The Compound N of the present invention, wherein Z^{a} represents a 3-8 membered nonaromatic heterocyclic group {wherein said 3-8 membered nonaromatic heterocyclic group is bound to the S(O)ₙₐ at a ring-constituting carbon atom, and optionally substituted with one or more substituent(s) selected from Group A^{a}}.
[Aspect C10] The Compound N of the present invention, wherein Z^{a} represents a 3-8 membered nonaromatic heterocyclic group in which a ring-constituting carbon atom is bound to the S(O)ₙₐ.
[Aspect C11] The Compound N of the present invention, wherein Z^{a} represents a 3-tetrahydrofuranyl group or a 3-tetrahydrothienyl group.
[Aspect C12] The Compound N of the present invention, wherein Z^{a} represents a 5-10 membered aromatic heterocyclic group {wherein said 5-10 membered aromatic heterocyclic group is bound to the S(O)ₙₐ at a ring-constituting carbon atom, and optionally substituted with one or more substituent(s) selected from Group B^{a}}.
[Aspect C13] The Compound N of the present invention, wherein Z^{a} represents a 5 membered aromatic heterocyclic group {wherein said 5 membered aromatic heterocyclic group is bound to the S(O)ₙₐ at a ring-constituting carbon atom, and optionally substituted with one or more substituent(s) selected from Group B^{a}2};
   Group B^{a}2: a group consisting of a C1-C6 chain hydrocarbon group and a halogen atom.
[Aspect C14] The Compound N of the present invention, wherein Z^{a} represents a 2-furyl group, a 3-furyl group, a 2-thienyl group, or a 3-thienyl group.
[Aspect C15] The Compound N of the present invention, wherein Z^{a} represents a 6 membered aromatic heterocyclic group {wherein said 6 membered aromatic heterocyclic group is bound to the S(O)ₙₐ at a ring-constituting carbon atom, and optionally substituted with one or more substituent(s) selected from Group B^{a}2}.
[Aspect C16] The Compound N of the present invention, wherein Z^{a} represents a 2-pyridyl group, a 3-pyridyl group, a 4-pyridyl group, a 2-pyrimidinyl group, or a 2-pyrazinyl group.
[Aspect C17] The Compound N of the present invention, wherein Z^{a} represents a 5-6 membered aromatic heterocyclic group {wherein said 5-6 membered aromatic heterocyclic group is bound to the S(O)ₙₐ at a ring-constituting carbon atom, and optionally substituted with one or more substituent(s) selected from Group B^{a}2}.
[Aspect C18] The Compound N of the present invention, wherein Z^{a} represents a 2-furyl group, a 3-furyl group, a 2-thienyl group, a 3-thienyl group, a 2-pyridyl group, a 3-pyridyl group, a 4-pyridyl group, a 2-pyrimidinyl group, or a 2-pyrazinyl group.
[Aspect C19] The Compound N of the present invention, wherein Z^{a} represents a C2-C6 chain hydrocarbon group or a 5-10 membered aromatic heterocyclic group {wherein said 5-10 membered aromatic heterocyclic group is bound to the S(O)ₙₐ at a ring-constituting carbon atom, and optionally substituted with one or more substituent(s) selected from Group B^{a}}.
[Aspect C20] The Compound N of the present invention, wherein Z^{a} represents a C2-C6 alkyl group or a 5-6 membered aromatic heterocyclic group {wherein said 5-6 membered aromatic heterocyclic group is bound to the S(O)ₙₐ at a ring-constituting carbon atom, and optionally substituted with one or more substituent(s) selected from Group B^{a}2}.
[Aspect C21] The Compound N of the present invention, wherein Z^{a} represents an ethyl group, a propyl group, a 2-furyl group, a 3-furyl group, a 2-thienyl group, a 3-thienyl group, a 2-pyridyl group, a 3-pyridyl group, a 4-pyridyl group, a 2-pyrimidinyl group, or a 2-pyrazinyl group.
[Aspect C22] The Compound N of the present invention, wherein Z^{a} represents a C2-C6 alkyl group or a 5 membered aromatic heterocyclic group {wherein said 5 membered aromatic heterocyclic group is bound to the S(O)ₙₐ at a ring-constituting carbon atom, and optionally substituted with one or more substituent(s) selected from Group B^{a}2}.
[Aspect C23] The Compound N of the present invention, wherein Z^{a} represents an ethyl group, a propyl group, a 2-furyl group, a 3-furyl group, a 2-thienyl group, or a 3-thienyl group.
[Aspect C24] The Compound N of the present invention, wherein Z^{a} represents a C2-C6 alkyl group or a 6 membered aromatic heterocyclic group {wherein said 6 membered aromatic heterocyclic group is bound to the S(O)ₙₐ at a ring-constituting carbon atom, and optionally substituted with one or more substituent(s) selected from Group B^{a}2}.
[Aspect C25] The Compound N of the present invention, wherein Z^{a} represents an ethyl group, a propyl group, a 2-pyridyl group, a 3-pyridyl group, a 4-pyridyl group, a 2-pyrimidinyl group, or a 2-pyrazinyl group.
[Aspect C26] The Compound N of the present invention, wherein Z^{a} represents a C2-C6 alkyl group, a C3-C8 alicyclic hydrocarbon group, or a 5-6 membered aromatic heterocyclic group {wherein said 5-6 membered aromatic heterocyclic group is bound to the S(O)ₙₐ at a ring-constituting carbon atom, and optionally substituted with one or more substituent(s) selected from Group B^{a}2}.
[Aspect C27] The Compound N of the present invention, wherein Z^{a} represents an ethyl group, a propyl group, a cyclopropyl group, a cyclopentyl group, a cyclohexyl group, a 2-furyl group, a 3-furyl group, a 2-thienyl group, a 3-thienyl group, a 2-pyridyl group, a 3-pyridyl group, a 4-pyridyl group, a 2-pyrimidinyl group, or a 2-pyrazinyl group.
[Aspect C28] The Compound N of the present invention, wherein
   Z^{a} represents a phenyl group optionally substituted with one or more substituent(s) selected from Group B^{a}; and
   X^{3a} represents a nitrogen atom.
[Aspect C29] The Compound N of the present invention, wherein
   Z^{a} represents a phenyl group optionally substituted with one or more substituent(s) selected from Group B^{a}2; and
   X^{3a} represents a nitrogen atom.
[Aspect C30] The Compound N of the present invention, wherein
   Z^{a} represents a phenyl group; and
   X^{3a} represents a nitrogen atom.
[Aspect C31] The Compound N of the present invention, wherein
   the combination of X^{3a} and Z^{a} represents:
   a combination wherein
      X^{3a} represents CR^{3a}, and
      Z^{a} represents a C2-C6 chain hydrocarbon group or a 5-10 membered aromatic heterocyclic group {wherein said 5-10 membered aromatic heterocyclic group is bound to the S(O)ₙₐ at a ring-constituting carbon atom, and optionally substituted with one or more substituent(s) selected from Group B^{a}};
   or, a combination wherein
      X^{3a} represents a nitrogen atom, and
      Z^{a} represents a C2-C6 chain hydrocarbon group, a 5-10 membered aromatic heterocyclic group {wherein said 5-10 membered aromatic heterocyclic group is bound to the S(O)ₙₐ at a ring-constituting carbon atom, and optionally substituted with one or more substituent(s) selected from Group B^{a}}, or a phenyl group optionally substituted with one or more substituent(s) selected from Group B^{a}.
[Aspect C32] The Compound N of the present invention, wherein
   the combination of X^{3a} and Z^{a} represents:
   a combination wherein
   X^{3a} represents CR^{3a}, and
   Z^{a} represents a C2-C6 alkyl group or a 5-6 membered aromatic heterocyclic group {wherein said 5-6 membered aromatic heterocyclic group is bound to the S(O)ₙₐ at a ring-constituting carbon atom, and optionally substituted with one or more substituent(s) selected from Group B^{a}2}; or, a combination wherein
   X^{3a} represents a nitrogen atom, and
   Z^{a} represents a C2-C6 alkyl group, a 5-6 membered aromatic heterocyclic group {wherein said 5-6 membered aromatic heterocyclic group is bound to the S(O)ₙₐ at a ring-constituting carbon atom, and optionally substituted with one or more substituent(s) selected from Group B^{a}2}, or a phenyl group optionally substituted with one or more substituent(s) selected from Group B^{a}2.
[Aspect C33] The Compound N of the present invention, wherein
   the combination of X^{3a} and Z^{a} represents:
   a combination wherein
      X^{3a} represents CR^{3a}, and
      Z^{a} represents an ethyl group, a propyl group, a 2-furyl group, a 3-furyl group, a 2-thienyl group, a 3-thienyl group, a 2-pyridyl group, a 3-pyridyl group, a 4-pyridyl group, a 2-pyrimidinyl group, or a 2-pyrazinyl group;
   or, a combination wherein
      X^{3a} represents a nitrogen atom, and
      Z^{a} represents an ethyl group, a propyl group, a 2-furyl group, a 3-furyl group, a 2-thienyl group, a 3-thienyl group, a 2-pyridyl group, a 3-pyridyl group, a 4-pyridyl group, a 2-pyrimidinyl group, a 2-pyrazinyl group, or a phenyl group.
[Aspect C34] The Compound N of the present invention, wherein
   the combination of X^{3a} and Z^{a} represents:
   a combination wherein
   X^{3a} represents CR^{3a}, and
   Z^{a} represents a C2-C6 alkyl group or a 5 membered aromatic heterocyclic group {wherein said 5 membered aromatic heterocyclic group is bound to the S(O)ₙₐ at a ring-constituting carbon atom, and optionally substituted with one or more substituent(s) selected from Group B^{a}2}; or, a combination wherein
   X^{3a} represents a nitrogen atom, and
   Z^{a} represents a C2-C6 alkyl group, a 5 membered aromatic heterocyclic group {wherein said 5 membered aromatic heterocyclic group is bound to the S(O)ₙₐ at a ring-constituting carbon atom, and optionally substituted with one or more substituent(s) selected from Group B^{a}2}, or a phenyl group optionally substituted with one or more substituent(s) selected from Group B^{a}2.
[Aspect C35] The Compound N of the present invention, wherein
   the combination of X^{3a} and Z^{a} represents:
   a combination wherein
      X^{3a} represents CR^{3a}, and
      Z^{a} represents an ethyl group, a propyl group, a 2-furyl group, a 3-furyl group, a 2-thienyl group, or a 3-thienyl group;
   or, a combination wherein
      X^{3a} represents a nitrogen atom, and
      Z^{a} represents an ethyl group, a propyl group, a 2-furyl group, a 3-furyl group, a 2-thienyl group, a 3-thienyl group, or a phenyl group.
[Aspect C36] The Compound N of the present invention, wherein
   the combination of X^{3 a} and Z^{a} represents:
   a combination wherein
      X^{3 a} represents CR^{3 a}, and
      Z^{a} represents a C2-C6 alkyl group or a 6 membered aromatic heterocyclic group {wherein said 6 membered aromatic heterocyclic group is bound to the S(O)_{n a} at a ring-constituting carbon atom, and optionally substituted with one or more substituent(s) selected from Group B^{a}2};
   or, a combination wherein
      X^{3 a} represents a nitrogen atom, and
      Z^{a} represents a C2-C6 alkyl group, a 6 membered aromatic heterocyclic group {wherein said 6 membered aromatic heterocyclic group is bound to the S(O)_{n a} at a ring-constituting carbon atom, and optionally substituted with one or more substituent(s) selected from Group B^{a} 2}, or a phenyl group optionally substituted with one or more substituent(s) selected from Group B^{a} 2.
[Aspect C37] The Compound N of the present invention, wherein
   the combination of X^{3 a} and Z^{a} represents:
   a combination wherein
      X^{3 a} represents CR^{3 a}, and
      Z^{a} represents an ethyl group, a propyl group, a 2-pyridyl group, a 3-pyridyl group, a 4-pyridyl group, a 2-pyrimidinyl group, or a 2-pyrazinyl group;
   or, a combination wherein
      X^{3 a} represents a nitrogen atom, and
      Z^{a} represents an ethyl group, a propyl group, a 2-pyridyl group, a 3-pyridyl group, a 4-pyridyl group, a 2-pyrimidinyl group, a 2-pyrazinyl group, or a phenyl group.
[Aspect C38] The Compound N of the present invention, wherein
   the combination of X^{3 a} and Z^{a} represents:
   a combination wherein
      X^{3 a} represents CR^{3 a}, and
      Z^{a} represents a 5-6 membered aromatic heterocyclic group {wherein said 5-6 membered aromatic heterocyclic group is bound to the S(O)_{n a} at a ring-constituting carbon atom, and optionally substituted with one or more substituent(s) selected from Group B^{a} 2};
   or, a combination wherein
      X^{3 a} represents a nitrogen atom, and
      Z² represents a 5-6 membered aromatic heterocyclic group {wherein said 5-6 membered aromatic heterocyclic group is bound to the S(O)_{n a} at a ring-constituting carbon atom, and optionally substituted with one or more substituent(s) selected from Group B^{a} 2}, or a phenyl group optionally substituted with one or more substituent(s) selected from Group B^{a} 2.
[Aspect C39] The Compound N of the present invention, wherein
   the combination of X^{3 a} and Z^{a} represents:
   a combination wherein
      X^{3 a} represents CR^{3 a}, and
      Z^{a} represents a 2-furyl group, a 3-furyl group, a 2-thienyl group, a 3-thienyl group, a 2-pyridyl group, a 3-pyridyl group, a 4-pyridyl group, a 2-pyrimidinyl group, or a 2-pyrazinyl group;
   or, a combination wherein
      X^{3 a} represents a nitrogen atom, and
      Z^{a} represents a 2-furyl group, a 3-furyl group, a 2-thienyl group, a 3-thienyl group, a 2-pyridyl group, a 3-pyridyl group, a 4-pyridyl group, a 2-pyrimidinyl group, a 2-pyrazinyl group, or a phenyl group.
[Aspect C40] The Compound N of the present invention, wherein
   the combination of X^{3 a} and Z^{a} represents:
   a combination wherein
      X^{3 a} represents CR^{3 a}, and
      Z^{a} represents a 5 membered aromatic heterocyclic group {wherein said 5 membered aromatic heterocyclic group is bound to the S(O)_{n a} at a ring-constituting carbon atom, and optionally substituted with one or more substituent(s) selected from Group B^{a} 2};
   or, a combination wherein
      X^{3 a} represents a nitrogen atom, and
      Z^{a} represents a 5 membered aromatic heterocyclic group {wherein said 5 membered aromatic heterocyclic group is bound to the S(O)_{n a} at a ring-constituting carbon atom, and optionally substituted with one or more substituent(s) selected from Group B^{a} 2}, or a phenyl group optionally substituted with one or more substituent(s) selected from Group B^{a} 2.
[Aspect C41] The Compound N of the present invention, wherein
   the combination of X^{3 a} and Z^{a} represents:
   a combination wherein
      X^{3 a} represents CR^{3 a}, and
      Z^{a} represents a 2-furyl group, a 3-furyl group, a 2-thienyl group, or a 3-thienyl group;
   or, a combination wherein
      X^{3 a} represents a nitrogen atom, and
      Z^{a} represents a 2-furyl group, a 3-furyl group, a 2-thienyl group, a 3-thienyl group, or a phenyl group.
[Aspect C42] The Compound N of the present invention, wherein
   the combination of X^{3a} and Z^{a} represents:
   a combination wherein
      X^{3a} represents CR^{3a}, and
      Z^{a} represents a 6 membered aromatic heterocyclic group {wherein said 6 membered aromatic heterocyclic group is bound to the S(O)ₙₐ at a ring-constituting carbon atom, and optionally substituted with one or more substituent(s) selected from Group B^{a}2};
   or, a combination wherein
      X^{3a} represents a nitrogen atom, and
      Z^{a} represents a 6 membered aromatic heterocyclic group {wherein said 6 membered aromatic heterocyclic group is bound to the S(O)ₙₐ at a ring-constituting carbon atom, and optionally substituted with one or more substituent(s) selected from Group B^{a}2}, or a phenyl group optionally substituted with one or more substituent(s) selected from Group B^{a}2.
[Aspect C43] The Compound N of the present invention, wherein
   the combination of X^{3a} and Z^{a} represents:
   a combination wherein
      X^{3a} represents CR^{3a}, and
      Z^{a} represents a 2-pyridyl group, a 3-pyridyl group, a 4-pyridyl group, a 2-pyrimidinyl group, or a 2-pyrazinyl group;
   or, a combination wherein
      X^{3a} represents a nitrogen atom, and
      Z^{a} represents a 2-pyridyl group, a 3-pyridyl group, a 4-pyridyl group, a 2-pyrimidinyl group, a 2-pyrazinyl group, or a phenyl group.
[Aspect C44] The Compound N of the present invention, wherein
   X^{1 a} represents CR^{1 a}; and
   X^{3 a} represents CR^{3 a}.
[Aspect C45] The Compound N of the present invention, wherein
   X^{1 a} represents CR^{1 a};
   X^{3 a} represents CR^{3 a}; and
   R^{1 a} and R^{3 a} each represent a hydrogen atom.
[Aspect C46] The Compound N of the present invention, wherein
   X^{1 a} represents a nitrogen atom; and
   X^{3 a} represents CR^{3 a}.
[Aspect C47] The Compound N of the present invention, wherein
   X^{1 a} represents a nitrogen atom;
   X^{3 a} represents CR^{3 a}; and
   R^{3 a} represents a hydrogen atom.
[Aspect C48] The Compound N of the present invention, wherein
   X^{1 a} represents CR^{1 a}; and
   X^{3 a} represents a nitrogen atom.
[Aspect C49] The compound according to the Aspect C1, wherein
   X^{1 a} represents CR^{1 a}; and
   X^{3 a} represents a nitrogen atom.
[Aspect C50] The compound according to the Aspect C2, wherein
   X^{1 a} represents CR^{1 a}; and
   X^{3 a} represents a nitrogen atom.
[Aspect C51] The compound according to the Aspect C3, wherein
   X^{1 a} represents CR^{1 a}; and
   X^{3 a} represents a nitrogen atom.
[Aspect C52] The compound according to the Aspect C12, wherein
   X^{1 a} represents CR^{1 a}; and
   X^{3 a} represents a nitrogen atom.
[Aspect C53] The compound according to the Aspect C13, wherein
   X^{1 a} represents CR^{1 a}; and
   X^{3 a} represents a nitrogen atom.
[Aspect C54] The compound according to the Aspect C14, wherein
   X^{1 a} represents CR^{1 a}; and
   X^{3 a} represents a nitrogen atom.
[Aspect C55] The compound according to the Aspect C15, wherein
   X^{1 a} represents CR^{1 a}; and
   X^{3 a} represents a nitrogen atom.
[Aspect C56] The compound according to the Aspect C16, wherein
   X^{1 a} represents CR^{1 a}; and
   X^{3 a} represents a nitrogen atom.
[Aspect C57] The compound according to the Aspect C17, wherein
   X^{1 a} represents CR^{1 a}; and
   X^{3 a} represents a nitrogen atom.
[Aspect C58] The compound according to the Aspect C18, wherein
   X^{1 a} represents CR^{1 a}; and
   X^{3 a} represents a nitrogen atom.
[Aspect C59] The compound according to the Aspect C19, wherein
   X^{1 a} represents CR^{1 a}; and
   X^{3 a} represents a nitrogen atom.
[Aspect C60] The compound according to the Aspect C20, wherein
   X^{1 a} represents CR^{1 a}; and
   X^{3 a} represents a nitrogen atom.
[Aspect C61] The compound according to the Aspect C21, wherein
   X^{1 a} represents CR^{1 a}; and
   X^{3 a} represents a nitrogen atom.
[Aspect C62] The compound according to the Aspect C22, wherein
   X^{1 a} represents CR^{1 a}; and
   X^{3 a} represents a nitrogen atom.
[Aspect C63] The compound according to the Aspect C23, wherein
   X^{1 a} represents CR^{1 a}; and
   X^{3 a} represents a nitrogen atom.
[Aspect C64] The compound according to the Aspect C24, wherein
   X^{1 a} represents CR^{1 a}; and
   X^{3 a} represents a nitrogen atom.
[Aspect C65] The compound according to the Aspect C25, wherein
   X^{1 a} represents CR^{1a}; and
   X^{3 a} represents a nitrogen atom.
[Aspect C66] The compound according to the Aspect C26, wherein
   X^{1 a} represents CR^{1 a}; and
   X^{3 a} represents a nitrogen atom.
[Aspect C67] The compound according to the Aspect C27, wherein
   X^{1 a} represents CR^{1 a}; and
   X^{3 a} represents a nitrogen atom.
[Aspect C68] The compound according to the Aspect C28, wherein
   X^{1 a} represents CR^{1 a}; and
   X^{3 a} represents a nitrogen atom.
[Aspect C69] The compound according to the Aspect C29, wherein
   X^{1 a} represents CR^{1 a}; and
   X^{3 a} represents a nitrogen atom.
[Aspect C70] The compound according to the Aspect C30, wherein
   X^{1 a} represents CR^{1 a}; and
   X^{3 a} represents a nitrogen atom.
[Aspect C71] The compound according to the Aspect C31, wherein
   X^{1 a} represents CR^{1 a}; and
   X^{3 a} represents a nitrogen atom.
[Aspect C72] The compound according to the Aspect C32, wherein
   X^{1 a} represents CR^{1 a}; and
   X^{3 a} represents a nitrogen atom.
[Aspect C73] The compound according to the Aspect C33, wherein
   X^{1 a} represents CR^{1a}; and
   X^{3 a} represents a nitrogen atom.
[Aspect C74] The compound according to the Aspect C34, wherein
   X^{1 a} represents CR^{1 a}; and
   X^{3 a} represents a nitrogen atom.
[Aspect C75] The compound according to the Aspect C35, wherein
   X^{1 a} represents CR^{1 a}; and
   X^{3 a} represents a nitrogen atom.
[Aspect C76] The compound according to the Aspect C36, wherein
   X^{1 a} represents CR^{1 a}; and
   X^{3 a} represents a nitrogen atom.
[Aspect C77] The compound according to the Aspect C37, wherein
   X^{1 a} represents CR^{1 a}; and
   X^{3 a} represents a nitrogen atom.
[Aspect C78] The compound according to the Aspect C38, wherein
   X^{1 a} represents CR^{1 a}; and
   X^{3 a} represents a nitrogen atom.
[Aspect C79] The compound according to the Aspect C39, wherein
   X^{1 a} represents CR^{1 a}; and
   X^{3 a} represents a nitrogen atom.
[Aspect C80] The compound according to the Aspect C40, wherein
   X^{1 a} represents CR^{1 a}; and
   X^{3 a} represents a nitrogen atom.
[Aspect C81] The compound according to the Aspect C41, wherein
   X^{1 a} represents CR^{1 a}; and
   X^{3 a} represents a nitrogen atom.
[Aspect C82] The compound according to the Aspect C42, wherein
   X^{1 a} represents CR^{1 a}; and
   X^{3 a} represents a nitrogen atom.
[Aspect C83] The compound according to the Aspect C43, wherein
   X^{1 a} represents CR^{1 a}; and
   X^{3 a} represents a nitrogen atom.
[Aspect C84] The Compound N of the present invention, wherein
   X^{1 a} represents CR^{1 a};
   X^{3 a} represents a nitrogen atom; and
   R^{1 a} represents a hydrogen atom.
[Aspect C85] The compound according to the Aspect C1, wherein
   X^{1 a} represents CR^{1 a};
   X^{3 a} represents a nitrogen atom; and
   R^{1 a} represents a hydrogen atom.
[Aspect C86] The compound according to the Aspect C2, wherein
   X^{1 a} represents CR^{1 a};
   X^{3 a} represents a nitrogen atom; and
   R^{1 a} represents a hydrogen atom.
[Aspect C87] The compound according to the Aspect C3, wherein
   X^{1 a} represents CR^{1 a};
   X^{3 a} represents a nitrogen atom; and
   R^{1 a} represents a hydrogen atom.
[Aspect C88] The compound according to the Aspect C12, wherein
   X^{1 a} represents CR^{1 a};
   X^{3 a} represents a nitrogen atom; and
   R^{1 a} represents a hydrogen atom.
[Aspect C89] The compound according to the Aspect C13, wherein
   X^{1 a} represents CR^{1 a};
   X^{3 a} represents a nitrogen atom; and
   R^{1 a} represents a hydrogen atom.
[Aspect C90] The compound according to the Aspect C14, wherein
   X^{1 a} represents CR^{1 a};
   X^{3 a} represents a nitrogen atom; and
   R^{1 a} represents a hydrogen atom.
[Aspect C91] The compound according to the Aspect C15, wherein
   X^{1 a} represents CR^{1 a};
   X^{3 a} represents a nitrogen atom; and
   R^{1 a} represents a hydrogen atom.
[Aspect C92] The compound according to the Aspect C16, wherein
   X^{1 a} represents CR^{1 a};
   X^{3 a} represents a nitrogen atom; and
   R^{1 a} represents a hydrogen atom.
[Aspect C93] The compound according to the Aspect C17, wherein
   X^{1 a} represents CR^{1 a};
   X^{3 a} represents a nitrogen atom; and
   R^{1 a} represents a hydrogen atom.
[Aspect C94] The compound according to the Aspect C18, wherein
   X^{1 a} represents CR^{1 a};
   X^{3 a} represents a nitrogen atom; and
   R^{1 a} represents a hydrogen atom.
[Aspect C95] The compound according to the Aspect C19, wherein
   X^{1 a} represents CR^{1 a};
   X^{3 a} represents a nitrogen atom; and
   R^{1 a} represents a hydrogen atom.
[Aspect C96] The compound according to the Aspect C20, wherein
   X^{1 a} represents CR^{1 a};
   X^{3 a} represents a nitrogen atom; and
   R^{1 a} represents a hydrogen atom.
[Aspect C97] The compound according to the Aspect C21, wherein
   X^{1 a} represents CR^{1 a};
   X^{3 a} represents a nitrogen atom; and
   R^{1 a} represents a hydrogen atom.
[Aspect C98] The compound according to the Aspect C22, wherein
   X^{1 a} represents CR^{1 a};
   X^{3 a} represents a nitrogen atom; and
   R^{1 a} represents a hydrogen atom.
[Aspect C99] The compound according to the Aspect C23, wherein
   X^{1 a} represents CR^{1 a};
   X^{3 a} represents a nitrogen atom; and
   R^{1 a} represents a hydrogen atom.
[Aspect C100] The compound according to the Aspect C24, wherein
   X^{1 a} represents CR^{1 a};
   X^{3 a} represents a nitrogen atom; and
   R^{1 a} represents a hydrogen atom.
[Aspect C101] The compound according to the Aspect C25, wherein
   X^{1 a} represents CR^{1 a};
   X^{3 a} represents a nitrogen atom; and
   R^{1 a} represents a hydrogen atom.
[Aspect C102] The compound according to the Aspect C26, wherein
   X^{1 a} represents CR^{1 a};
   X^{3 a} represents a nitrogen atom; and
   R^{1 a} represents a hydrogen atom.
[Aspect C103] The compound according to the Aspect C27, wherein
   X^{1 a} represents CR^{1 a};
   X^{3 a} represents a nitrogen atom; and
   R^{1 a} represents a hydrogen atom.
[Aspect C104] The compound according to the Aspect C28, wherein
   X^{1 a} represents CR^{1 a};
   X^{3 a} represents a nitrogen atom; and
   R^{1 a} represents a hydrogen atom.
[Aspect C105] The compound according to the Aspect C29, wherein
   X^{1 a} represents CR^{1 a};
   X^{3 a} represents a nitrogen atom; and
   R^{1 a} represents a hydrogen atom.
[Aspect C106] The compound according to the Aspect C30, wherein
   X^{1 a} represents CR^{1 a};
   X^{3 a} represents a nitrogen atom; and
   R^{1 a} represents a hydrogen atom.
[Aspect C107] The compound according to the Aspect C31, wherein
   X^{1 a} represents CR^{1 a};
   X^{3 a} represents a nitrogen atom; and
   R^{1 a} represents a hydrogen atom.
[Aspect C108] The compound according to the Aspect C32, wherein
   X^{1 a} represents CR^{1 a};
   X^{3 a} represents a nitrogen atom; and
   R^{1 a} represents a hydrogen atom.
[Aspect C109] The compound according to the Aspect C33, wherein
   X^{1 a} represents CR^{1 a};
   X^{3 a} represents a nitrogen atom; and
   R^{1 a} represents a hydrogen atom.
[Aspect C110] The compound according to the Aspect C34, wherein
   X^{1 a} represents CR^{1 a};
   X^{3 a} represents a nitrogen atom; and
   R^{1 a} represents a hydrogen atom.
[Aspect C111] The compound according to the Aspect C35, wherein
   X^{1 a} represents CR^{1 a};
   X^{3 a} represents a nitrogen atom; and
   R^{1 a} represents a hydrogen atom.
[Aspect C112] The compound according to the Aspect C36, wherein
   X^{1 a} represents CR^{1 a};
   X^{3 a} represents a nitrogen atom; and
   R^{1 a} represents a hydrogen atom.
[Aspect C113] The compound according to the Aspect C37, wherein
   X^{1 a} represents CR^{1 a};
   X^{3 a} represents a nitrogen atom; and
   R^{1 a} represents a hydrogen atom.
[Aspect C114] The compound according to the Aspect C38, wherein
   X^{1 a} represents CR^{1 a};
   X^{3 a} represents a nitrogen atom; and
   R^{1 a} represents a hydrogen atom.
[Aspect C115] The compound according to the Aspect C39, wherein
   X^{1 a} represents CR^{1 a};
   X^{3 a} represents a nitrogen atom; and
   R^{1 a} represents a hydrogen atom.
[Aspect C116] The compound according to the Aspect C40, wherein
   X^{1 a} represents CR^{1 a};
   X^{3 a} represents a nitrogen atom; and
   R^{1 a} represents a hydrogen atom.
[Aspect C117] The compound according to the Aspect C41, wherein
   X^{1 a} represents CR^{1 a};
   X^{3 a} represents a nitrogen atom; and
   R^{1 a} represents a hydrogen atom.
[Aspect C118] The compound according to the Aspect C42, wherein
   X^{1 a} represents CR^{1 a};
   X^{3 a} represents a nitrogen atom; and
   R^{1 a} represents a hydrogen atom.
[Aspect C119] The compound according to the Aspect C43, wherein
   X^{1 a} represents CR^{1 a};
   X^{3 a} represents a nitrogen atom; and
   R^{1 a} represents a hydrogen atom.
[Aspect C120] The Compound N of the present invention, wherein
   X^{1 a} represents a nitrogen atom; and
   X^{3 a} represents a nitrogen atom.
[Aspect C121] The Compound N of the present invention, wherein X^{1 a} represents CR^{1 a}.
[Aspect C122] The compound according to the Aspect C1, wherein X^{1 a} represents CR^{1 a}.
[Aspect C123] The compound according to the Aspect C2, wherein X^{1 a} represents CR^{1 a}.
[Aspect C124] The compound according to the Aspect C3, wherein X^{1 a} represents CR^{1 a}.
[Aspect C125] The compound according to the Aspect C12, wherein X^{1 a} represents CR^{1 a}.
[Aspect C126] The compound according to the Aspect C13, wherein X^{1 a} represents CR^{1 a}.
[Aspect C127] The compound according to the Aspect C14, wherein X^{1 a} represents CR^{1 a}.
[Aspect C128] The compound according to the Aspect C15, wherein X^{1 a} represents CR^{1 a}.
[Aspect C129] The compound according to the Aspect C16, wherein X^{1 a} represents CR^{1 a}.
[Aspect C130] The compound according to the Aspect C17, wherein X^{1 a} represents CR^{1 a}.
[Aspect C131] The compound according to the Aspect C18, wherein X^{1 a} represents CR^{1 a}.
[Aspect C132] The compound according to the Aspect C19, wherein X^{1 a} represents CR^{1 a}.
[Aspect C133] The compound according to the Aspect C20, wherein X^{1 a} represents CR^{1 a}.
[Aspect C134] The compound according to the Aspect C21, wherein X^{1 a} represents CR^{1 a}.
[Aspect C135] The compound according to the Aspect C22, wherein X^{1 a} represents CR^{1 a}.
[Aspect C136] The compound according to the Aspect C23, wherein X^{1 a} represents CR^{1 a}.
[Aspect C137] The compound according to the Aspect C24, wherein X^{1 a} represents CR^{1 a}.
[Aspect C138] The compound according to the Aspect C25, wherein X^{1 a} represents CR^{1 a}.
[Aspect C139] The compound according to the Aspect C26, wherein X^{1 a} represents CR^{1 a}.
[Aspect C140] The compound according to the Aspect C27, wherein X^{1 a} represents CR^{1 a}.
[Aspect C141] The compound according to the Aspect C28, wherein X^{1 a} represents CR^{1 a}.
[Aspect C142] The compound according to the Aspect C29, wherein X^{1 a} represents CR^{1 a}.
[Aspect C143] The compound according to the Aspect C30, wherein X^{1 a} represents CR^{1 a}.
[Aspect C144] The compound according to the Aspect C31, wherein X^{1 a} represents CR^{1 a}.
[Aspect C145] The compound according to the Aspect C32, wherein X^{1 a} represents CR^{1 a}.
[Aspect C146] The compound according to the Aspect C33, wherein X^{1 a} represents CR^{1 a}.
[Aspect C147] The compound according to the Aspect C34, wherein X^{1 a} represents CR^{1 a}.
[Aspect C148] The compound according to the Aspect C35, wherein X^{1 a} represents CR^{1 a}.
[Aspect C149] The compound according to the Aspect C36, wherein X^{1 a} represents CR^{1 a}.
[Aspect C150] The compound according to the Aspect C37, wherein X^{1 a} represents CR^{1 a}.
[Aspect C151] The compound according to the Aspect C38, wherein X^{1 a} represents CR^{1 a}.
[Aspect C152] The compound according to the Aspect C39, wherein X^{1 a} represents CR^{1 a}.
[Aspect C153] The compound according to the Aspect C40, wherein X^{1 a} represents CR^{1 a}.
[Aspect C154] The compound according to the Aspect C41, wherein X^{1 a} represents CR^{1 a}.
[Aspect C155] The compound according to the Aspect C42, wherein X^{1 a} represents CR^{1 a}.
[Aspect C156] The compound according to the Aspect C43, wherein X^{1 a} represents CR^{1 a}.
[Aspect C157] The Compound N of the present invention, wherein
   X^{1 a} represents CR^{1 a}; and
   R^{1 a} represents a hydrogen atom.
[Aspect C158] The compound according to the Aspect C1, wherein
   X^{1 a} represents CR^{1 a}; and
   R^{1 a} represents a hydrogen atom.
[Aspect C159] The compound according to the Aspect C2, wherein
   X^{1 a} represents CR^{1 a}; and
   R^{1 a} represents a hydrogen atom.
[Aspect C160] The compound according to the Aspect C3, wherein
   X^{1 a} represents CR^{1 a}; and
   R^{1 a} represents a hydrogen atom.
[Aspect C161] The compound according to the Aspect C12, wherein
   X^{1 a} represents CR^{1 a}; and
   R^{1 a} represents a hydrogen atom.
[Aspect C162] The compound according to the Aspect C13, wherein
   X^{1 a} represents CR^{1 a}; and
   R^{1 a} represents a hydrogen atom.
[Aspect C163] The compound according to the Aspect C14, wherein
   X^{1 a} represents CR^{1 a}; and
   R^{1 a} represents a hydrogen atom.
[Aspect C164] The compound according to the Aspect C15, wherein
   X^{1 a} represents CR^{1 a}; and
   R^{1 a} represents a hydrogen atom.
[Aspect C165] The compound according to the Aspect C16, wherein
   X^{1 a} represents CR^{1 a}; and
   R^{1 a} represents a hydrogen atom.
[Aspect C166] The compound according to the Aspect C17, wherein
   X^{1 a} represents CR^{1 a}; and
   R^{1 a} represents a hydrogen atom.
[Aspect C167] The compound according to the Aspect C18, wherein
   X^{1 a} represents CR^{1 a}; and
   R^{1 a} represents a hydrogen atom.
[Aspect C168] The compound according to the Aspect C19, wherein
   X^{1 a} represents CR^{1 a}; and
   R^{1 a} represents a hydrogen atom.
[Aspect C169] The compound according to the Aspect C20, wherein
   X^{1 a} represents CR^{1 a}; and
   R^{1 a} represents a hydrogen atom.
[Aspect C170] The compound according to the Aspect C21, wherein
   X^{1 a} represents CR^{1 a}; and
   R^{1 a} represents a hydrogen atom.
[Aspect C171] The compound according to the Aspect C22, wherein
   X^{1 a} represents CR^{1 a}; and
   R^{1 a} represents a hydrogen atom.
[Aspect C172] The compound according to the Aspect C23, wherein
   X^{1 a} represents CR^{1 a}; and
   R^{1 a} represents a hydrogen atom.
[Aspect C173] The compound according to the Aspect C24, wherein
   X^{1 a} represents CR^{1 a}; and
   R^{1 a} represents a hydrogen atom.
[Aspect C174] The compound according to the Aspect C25, wherein
   X^{1 a} represents CR^{1 a}; and
   R^{1 a} represents a hydrogen atom.
[Aspect C175] The compound according to the Aspect C26, wherein
   X^{1 a} represents CR^{1 a}; and
   R^{1 a} represents a hydrogen atom.
[Aspect C176] The compound according to the Aspect C27, wherein
   X^{1 a} represents CR^{1 a}; and
   R^{1 a} represents a hydrogen atom.
[Aspect C177] The compound according to the Aspect C28, wherein
   X^{1 a} represents CR^{1 a}; and
   R^{1 a} represents a hydrogen atom.
[Aspect C178] The compound according to the Aspect C29, wherein
   X^{1 a} represents CR^{1 a}; and
   R^{1 a} represents a hydrogen atom.
[Aspect C179] The compound according to the Aspect C30, wherein
   X^{1 a} represents CR^{1 a}; and
   R^{1 a} represents a hydrogen atom.
[Aspect C180] The compound according to the Aspect C31, wherein
   X^{1 a} represents CR^{1 a}; and
   R^{1 a} represents a hydrogen atom.
[Aspect C181] The compound according to the Aspect C32, wherein
   X^{1 a} represents CR^{1 a}; and
   R^{1 a} represents a hydrogen atom.
[Aspect C182] The compound according to the Aspect C33, wherein
   X^{1 a} represents CR^{1 a}; and
   R^{1 a} represents a hydrogen atom.
[Aspect C183] The compound according to the Aspect C34, wherein
   X^{1 a} represents CR^{1 a}; and
   R^{1 a} represents a hydrogen atom.
[Aspect C184] The compound according to the Aspect C35, wherein
   X^{1 a} represents CR^{1 a}; and
   R^{1 a} represents a hydrogen atom.
[Aspect C185] The compound according to the Aspect C36, wherein
   X^{1 a} represents CR^{1 a}; and
   R^{1 a} represents a hydrogen atom.
[Aspect C186] The compound according to the Aspect C37, wherein
   X^{1 a} represents CR^{1 a}; and
   R^{1 a} represents a hydrogen atom.
[Aspect C187] The compound according to the Aspect C38, wherein
   X^{1 a} represents CR^{1 a}; and
   R^{1 a} represents a hydrogen atom.
[Aspect C188] The compound according to the Aspect C39, wherein
   X^{1 a} represents CR^{1 a}; and
   R^{1 a} represents a hydrogen atom.
[Aspect C189] The compound according to the Aspect C40, wherein
   X^{1 a} represents CR^{1 a}; and
   R^{1 a} represents a hydrogen atom.
[Aspect C190] The compound according to the Aspect C41, wherein
   X^{1 a} represents CR^{1 a}; and
   R^{1 a} represents a hydrogen atom.
[Aspect C191] The compound according to the Aspect C42, wherein
   X^{1 a} represents CR^{1 a}; and
   R^{1 a} represents a hydrogen atom.
[Aspect C192] The compound according to the Aspect C43, wherein
   X^{1 a} represents CR^{1 a}; and
   R^{1 a} represents a hydrogen atom.
[Aspect C193] The Compound N of the present invention, wherein X^{3 a} represents a nitrogen atom.
[Aspect C194] The compound according to the Aspect C1, wherein X^{3 a} represents a nitrogen atom.
[Aspect C195] The compound according to the Aspect C2, wherein X^{3 a} represents a nitrogen atom.
[Aspect C196] The compound according to the Aspect C3, wherein X^{3 a} represents a nitrogen atom.
[Aspect C197] The compound according to the Aspect C12, wherein X^{3 a} represents a nitrogen atom.
[Aspect C198] The compound according to the Aspect C13, wherein X^{3 a} represents a nitrogen atom.
[Aspect C199] The compound according to the Aspect C14, wherein X^{3 a} represents a nitrogen atom.
[Aspect C200] The compound according to the Aspect C15, wherein X^{3 a} represents a nitrogen atom.
[Aspect C201] The compound according to the Aspect C16, wherein X^{3 a} represents a nitrogen atom.
[Aspect C202] The compound according to the Aspect C17, wherein X^{3 a} represents a nitrogen atom.
[Aspect C203] The compound according to the Aspect C18, wherein X^{3 a} represents a nitrogen atom.
[Aspect C204] The compound according to the Aspect C19, wherein X^{3 a} represents a nitrogen atom.
[Aspect C205] The compound according to the Aspect C20, wherein X^{3 a} represents a nitrogen atom.
[Aspect C206] The compound according to the Aspect C21, wherein X^{3 a} represents a nitrogen atom.
[Aspect C207] The compound according to the Aspect C22, wherein X^{3 a} represents a nitrogen atom.
[Aspect C208] The compound according to the Aspect C23, wherein X^{3 a} represents a nitrogen atom.
[Aspect C209] The compound according to the Aspect C24, wherein X^{3 a} represents a nitrogen atom.
[Aspect C210] The compound according to the Aspect C25, wherein X^{3 a} represents a nitrogen atom.
[Aspect C211] The compound according to the Aspect C26, wherein X^{3 a} represents a nitrogen atom.
[Aspect C212] The compound according to the Aspect C27, wherein X^{3 a} represents a nitrogen atom.
[Aspect C213] The compound according to the Aspect C31, wherein X^{3 a} represents a nitrogen atom.
[Aspect C214] The compound according to the Aspect C32, wherein X^{3 a} represents a nitrogen atom.
[Aspect C215] The compound according to the Aspect C33, wherein X^{3 a} represents a nitrogen atom.
[Aspect C216] The compound according to the Aspect C34, wherein X^{3 a} represents a nitrogen atom.
[Aspect C217] The compound according to the Aspect C35, wherein X^{3 a} represents a nitrogen atom.
[Aspect C218] The compound according to the Aspect C36, wherein X^{3 a} represents a nitrogen atom.
[Aspect C219] The compound according to the Aspect C37, wherein X^{3 a} represents a nitrogen atom.
[Aspect C220] The compound according to the Aspect C38, wherein X^{3 a} represents a nitrogen atom.
[Aspect C221] The compound according to the Aspect C39, wherein X^{3 a} represents a nitrogen atom.
[Aspect C222] The compound according to the Aspect C40, wherein X^{3 a} represents a nitrogen atom.
[Aspect C223] The compound according to the Aspect C41, wherein X^{3 a} represents a nitrogen atom.
[Aspect C224] The compound according to the Aspect C42, wherein X^{3 a} represents a nitrogen atom.
[Aspect C225] The compound according to the Aspect C43, wherein X^{3 a} represents a nitrogen atom.
[Aspect C226] The Compound N of the present invention, wherein
   X^{3 a} represents a nitrogen atom; and
   R^{1 a} represents a hydrogen atom.
[Aspect C227] The compound according to the Aspect C1, wherein
   X^{3 a} represents a nitrogen atom; and
   R^{1 a} represents a hydrogen atom.
[Aspect C228] The compound according to the Aspect C2, wherein
   X^{3 a} represents a nitrogen atom; and
   R^{1 a} represents a hydrogen atom.
[Aspect C229] The compound according to the Aspect C3, wherein
   X^{3 a} represents a nitrogen atom; and
   R^{1 a} represents a hydrogen atom.
[Aspect C230] The compound according to the Aspect C12, wherein
   X^{3 a} represents a nitrogen atom; and
   R^{1 a} represents a hydrogen atom.
[Aspect C231] The compound according to the Aspect C13, wherein
   X^{3 a} represents a nitrogen atom; and
   R^{1 a} represents a hydrogen atom.
[Aspect C232] The compound according to the Aspect C14, wherein
   X^{3 a} represents a nitrogen atom; and
   R^{1 a} represents a hydrogen atom.
[Aspect C233] The compound according to the Aspect C15, wherein
   X^{3 a} represents a nitrogen atom; and
   R^{1 a} represents a hydrogen atom.
[Aspect C234] The compound according to the Aspect C16, wherein
   X^{3 a} represents a nitrogen atom; and
   R^{1 a} represents a hydrogen atom.
[Aspect C235] The compound according to the Aspect C17, wherein
   X^{3 a} represents a nitrogen atom; and
   R^{1 a} represents a hydrogen atom.
[Aspect C236] The compound according to the Aspect C18, wherein
   X^{3 a} represents a nitrogen atom; and
   R^{1 a} represents a hydrogen atom.
[Aspect C237] The compound according to the Aspect C19, wherein
   X^{3 a} represents a nitrogen atom; and
   R^{1 a} represents a hydrogen atom.
[Aspect C238] The compound according to the Aspect C20, wherein
   X^{3 a} represents a nitrogen atom; and
   R^{1 a} represents a hydrogen atom.
[Aspect C239] The compound according to the Aspect C21, wherein
   X^{3 a} represents a nitrogen atom; and
   R^{1 a} represents a hydrogen atom.
[Aspect C240] The compound according to the Aspect C22, wherein
   X^{3 a} represents a nitrogen atom; and
   R^{1 a} represents a hydrogen atom.
[Aspect C241] The compound according to the Aspect C23, wherein
   X^{3 a} represents a nitrogen atom; and
   R^{1 a} represents a hydrogen atom.
[Aspect C242] The compound according to the Aspect C24, wherein
   X^{3 a} represents a nitrogen atom; and
   R^{1 a} represents a hydrogen atom.
[Aspect C243] The compound according to the Aspect C25, wherein
   X^{3 a} represents a nitrogen atom; and
   R^{1 a} represents a hydrogen atom.
[Aspect C244] The compound according to the Aspect C26, wherein
   X^{3 a} represents a nitrogen atom; and
   R^{1 a} represents a hydrogen atom.
[Aspect C245] The compound according to the Aspect C27, wherein
   X^{3 a} represents a nitrogen atom; and
   R^{1 a} represents a hydrogen atom.
[Aspect C246] The compound according to the Aspect C31, wherein
   X^{3 a} represents a nitrogen atom; and
   R^{1 a} represents a hydrogen atom.
[Aspect C247] The compound according to the Aspect C32, wherein
   X^{3 a} represents a nitrogen atom; and
   R^{1 a} represents a hydrogen atom.
[Aspect C248] The compound according to the Aspect C33, wherein
   X^{3 a} represents a nitrogen atom; and
   R^{1 a} represents a hydrogen atom.
[Aspect C249] The compound according to the Aspect C34, wherein
   X^{3 a} represents a nitrogen atom; and
   R^{1 a} represents a hydrogen atom.
[Aspect C250] The compound according to the Aspect C35, wherein
   X^{3 a} represents a nitrogen atom; and
   R^{1 a} represents a hydrogen atom.
[Aspect C251] The compound according to the Aspect C36, wherein
   X^{3 a} represents a nitrogen atom; and
   R^{1 a} represents a hydrogen atom.
[Aspect C252] The compound according to the Aspect C37, wherein
   X^{3 a} represents a nitrogen atom; and
   R^{1 a} represents a hydrogen atom.
[Aspect C253] The compound according to the Aspect C38, wherein
   X^{3 a} represents a nitrogen atom; and
   R^{1 a} represents a hydrogen atom.
[Aspect C254] The compound according to the Aspect C39, wherein
   X^{3 a} represents a nitrogen atom; and
   R^{1 a} represents a hydrogen atom.
[Aspect C255] The compound according to the Aspect C40, wherein
   X^{3 a} represents a nitrogen atom; and
   R^{1 a} represents a hydrogen atom.
[Aspect C256] The compound according to the Aspect C41, wherein
   X^{3 a} represents a nitrogen atom; and
   R^{1 a} represents a hydrogen atom.
[Aspect C257] The compound according to the Aspect C42, wherein
   X^{3 a} represents a nitrogen atom; and
   R^{1 a} represents a hydrogen atom.
[Aspect C258] The compound according to the Aspect C43, wherein
   X^{3 a} represents a nitrogen atom; and
   R^{1 a} represents a hydrogen atom.
[Aspect C259] The Compound N of the present invention, wherein na represents 1.
[Aspect C260] The Compound N of the present invention, wherein na represents 2.
[Aspect C261] The Compound N of the present invention, wherein
   X^{1 a} represents CR^{1 a};
   X^{3 a} represents a nitrogen atom;
   R^{1 a} represents a hydrogen atom; and
   na represents 2.
[Aspect C262] The compound according to the Aspect C1, wherein
   X^{1 a} represents CR^{1 a};
   X^{3 a} represents a nitrogen atom;
   R^{1 a} represents a hydrogen atom; and
   na represents 2.
[Aspect C263] The compound according to the Aspect C2, wherein
   X^{1 a} represents CR^{1 a};
   X^{3 a} represents a nitrogen atom;
   R^{1 a} represents a hydrogen atom; and
   na represents 2.
[Aspect C264] The compound according to the Aspect C3, wherein
   X^{1 a} represents CR^{1 a};
   X^{3 a} represents a nitrogen atom;
   R^{1 a} represents a hydrogen atom; and
   na represents 2.
[Aspect C265] The compound according to the Aspect C12, wherein
   X^{1 a} represents CR^{1 a};
   X^{3 a} represents a nitrogen atom;
   R^{1 a} represents a hydrogen atom; and na represents 2.
[Aspect C266] The compound according to the Aspect C13, wherein
   X^{1 a} represents CR^{1 a};
   X^{3 a} represents a nitrogen atom;
   R^{1 a} represents a hydrogen atom; and
   na represents 2.
[Aspect C267] The compound according to the Aspect C14, wherein
   X^{1 a} represents CR^{1 a};
   X^{3 a} represents a nitrogen atom;
   R^{1 a} represents a hydrogen atom; and
   na represents 2.
[Aspect C268] The compound according to the Aspect C15, wherein
   X^{1 a} represents CR^{1 a};
   X^{3 a} represents a nitrogen atom;
   R^{1 a} represents a hydrogen atom; and
   na represents 2.
[Aspect C269] The compound according to the Aspect C16, wherein
   X^{1 a} represents CR^{1 a};
   X^{3 a} represents a nitrogen atom;
   R^{1 a} represents a hydrogen atom; and
   na represents 2.
[Aspect C270] The compound according to the Aspect C17, wherein
   X^{1 a} represents CR^{1 a};
   X^{3 a} represents a nitrogen atom;
   R^{1 a} represents a hydrogen atom; and
   na represents 2.
[Aspect C271] The compound according to the Aspect C18, wherein
   X^{1 a} represents CR^{1 a};
   X^{3 a} represents a nitrogen atom;
   R^{1 a} represents a hydrogen atom; and
   na represents 2.
[Aspect C272] The compound according to the Aspect C19, wherein
   X^{1 a} represents CR^{1 a};
   X^{3 a} represents a nitrogen atom;
   R^{1 a} represents a hydrogen atom; and
   na represents 2.
[Aspect C273] The compound according to the Aspect C20, wherein
   X^{1 a} represents CR^{1 a};
   X^{3 a} represents a nitrogen atom;
   R^{1 a} represents a hydrogen atom; and
   na represents 2.
[Aspect C274] The compound according to the Aspect C21, wherein
   X^{1 a} represents CR^{1 a};
   X^{3 a} represents a nitrogen atom;
   R^{1 a} represents a hydrogen atom; and
   na represents 2.
[Aspect C275] The compound according to the Aspect C22, wherein
   X^{1 a} represents CR^{1 a};
   X^{3 a} represents a nitrogen atom;
   R^{1 a} represents a hydrogen atom; and
   na represents 2.
[Aspect C276] The compound according to the Aspect C23, wherein
   X^{1 a} represents CR^{1 a};
   X^{3 a} represents a nitrogen atom;
   R^{1 a} represents a hydrogen atom; and
   na represents 2.
[Aspect C277] The compound according to the Aspect C24, wherein
   X^{1 a} represents CR^{1 a};
   X^{3 a} represents a nitrogen atom;
   R^{1 a} represents a hydrogen atom; and
   na represents 2.
[Aspect C278] The compound according to the Aspect C25, wherein
   X^{1 a} represents CR^{1 a};
   X^{3 a} represents a nitrogen atom;
   R^{1 a} represents a hydrogen atom; and
   na represents 2.
[Aspect C279] The compound according to the Aspect C26, wherein
   X^{1 a} represents CR^{1 a};
   X^{3 a} represents a nitrogen atom;
   R^{1 a} represents a hydrogen atom; and
   na represents 2.
[Aspect C280] The compound according to the Aspect C27, wherein
   X^{1 a} represents CR^{1 a};
   X^{3 a} represents a nitrogen atom;
   R^{1 a} represents a hydrogen atom; and
   na represents 2.
[Aspect C281] The compound according to the Aspect C28, wherein
   X^{1 a} represents CR^{1 a};
   X^{3 a} represents a nitrogen atom;
   R^{1 a} represents a hydrogen atom; and
   na represents 2.
[Aspect C282] The compound according to the Aspect C29, wherein
   X^{1 a} represents CR^{1 a};
   X^{3 a} represents a nitrogen atom;
   R^{1 a} represents a hydrogen atom; and
   na represents 2.
[Aspect C283] The compound according to the Aspect C30, wherein
   X^{1 a} represents CR^{1 a};
   X^{3 a} represents a nitrogen atom;
   R^{1 a} represents a hydrogen atom; and
   na represents 2.
[Aspect C284] The compound according to the Aspect C31, wherein
   X^{1 a} represents CR^{1 a};
   X^{3 a} represents a nitrogen atom;
   R^{1 a} represents a hydrogen atom; and
   na represents 2.
[Aspect C285] The compound according to the Aspect C32, wherein
   X^{1 a} represents CR^{1 a};
   X^{3 a} represents a nitrogen atom;
   R^{1 a} represents a hydrogen atom; and
   na represents 2.
[Aspect C286] The compound according to the Aspect C33, wherein
   X^{1 a} represents CR^{1 a};
   X^{3 a} represents a nitrogen atom;
   R^{1 a} represents a hydrogen atom; and
   na represents 2.
[Aspect C287] The compound according to the Aspect C34, wherein
   X^{1 a} represents CR^{1 a};
   X^{3 a} represents a nitrogen atom;
   R^{1 a} represents a hydrogen atom; and
   na represents 2.
[Aspect C288] The compound according to the Aspect C35, wherein
   X^{1 a} represents CR^{1 a};
   X^{3 a} represents a nitrogen atom;
   R^{1 a} represents a hydrogen atom; and
   na represents 2.
[Aspect C289] The compound according to the Aspect C36, wherein
   X^{1 a} represents CR^{1 a};
   X^{3 a} represents a nitrogen atom;
   R^{1 a} represents a hydrogen atom; and
   na represents 2.
[Aspect C290] The compound according to the Aspect C37, wherein
   X^{1 a} represents CR^{1 a};
   X^{3 a} represents a nitrogen atom;
   R^{1 a} represents a hydrogen atom; and
   na represents 2.
[Aspect C291] The compound according to the Aspect C38, wherein
   X^{1 a} represents CR^{1 a};
   X^{3 a} represents a nitrogen atom;
   R^{1 a} represents a hydrogen atom; and
   na represents 2.
[Aspect C292] The compound according to the Aspect C39, wherein
   X^{1 a} represents CR^{1 a};
   X^{3 a} represents a nitrogen atom;
   R^{1 a} represents a hydrogen atom; and
   na represents 2.
[Aspect C293] The compound according to the Aspect C40, wherein
   X^{1 a} represents CR^{1 a};
   X^{3 a} represents a nitrogen atom;
   R^{1 a} represents a hydrogen atom; and
   na represents 2.
[Aspect C294] The compound according to the Aspect C41, wherein
   X^{1 a} represents CR^{1 a};
   X^{3 a} represents a nitrogen atom;
   R^{1 a} represents a hydrogen atom; and
   na represents 2.
[Aspect C295] The compound according to the Aspect C42, wherein
   X^{1 a} represents CR^{1 a};
   X^{3 a} represents a nitrogen atom;
   R^{1 a} represents a hydrogen atom; and
   na represents 2.
[Aspect C296] The compound according to the Aspect C43, wherein
   X^{1 a} represents CR^{1 a};
   X^{3 a} represents a nitrogen atom;
   R^{1 a} represents a hydrogen atom; and
   na represents 2.
[Aspect C297] The Compound N of the present invention, wherein
   X^{1 a} represents CR^{1 a};
   R^{1 a} represents a hydrogen atom; and
   na represents 2.
[Aspect C298] The compound according to the Aspect C1, wherein
   X^{1 a} represents CR^{1 a};
   R^{1 a} represents a hydrogen atom; and
   na represents 2.
[Aspect C299] The compound according to the Aspect C2, wherein
   X^{1 a} represents CR^{1a};
   R^{1 a} represents a hydrogen atom; and
   na represents 2.
[Aspect C300] The compound according to the Aspect C3, wherein
   X^{1 a} represents CR^{1 a};
   R^{1 a} represents a hydrogen atom; and
   na represents 2.
[Aspect C301] The compound according to the Aspect C12, wherein
   X^{1 a} represents CR^{1 a};
   R^{1 a} represents a hydrogen atom; and
   na represents 2.
[Aspect C302] The compound according to the Aspect C13, wherein
   X^{1 a} represents CR^{1 a};
   R^{1 a} represents a hydrogen atom; and
   na represents 2.
[Aspect C303] The compound according to the Aspect C14, wherein
   X^{1 a} represents CR^{1 a};
   R^{1 a} represents a hydrogen atom; and
   na represents 2.
[Aspect C304] The compound according to the Aspect C15, wherein
   X^{1 a} represents CR^{1 a};
   R^{1 a} represents a hydrogen atom; and
   na represents 2.
[Aspect C305] The compound according to the Aspect C16, wherein
   X^{1 a} represents CR^{1 a};
   R^{1 a} represents a hydrogen atom; and
   na represents 2.
[Aspect C306] The compound according to the Aspect C17, wherein
   X^{1 a} represents CR^{1 a};
   R^{1 a} represents a hydrogen atom; and
   na represents 2.
[Aspect C307] The compound according to the Aspect C18, wherein
   X^{1 a} represents CR^{1 a};
   R^{1 a} represents a hydrogen atom; and
   na represents 2.
[Aspect C308] The compound according to the Aspect C19, wherein
   X^{1 a} represents CR^{1a};
   R^{1 a} represents a hydrogen atom; and
   na represents 2.
[Aspect C309] The compound according to the Aspect C20, wherein
   X^{1 a} represents CR^{1 a};
   R^{1 a} represents a hydrogen atom; and
   na represents 2.
[Aspect C310] The compound according to the Aspect C21, wherein
   X^{1 a} represents CR^{1 a};
   R^{1 a} represents a hydrogen atom; and
   na represents 2.
[Aspect C311] The compound according to the Aspect C22, wherein
   X^{1 a} represents CR^{1 a};
   R^{1 a} represents a hydrogen atom; and
   na represents 2.
[Aspect C312] The compound according to the Aspect C23, wherein
   X^{1 a} represents CR^{1 a};
   R^{1 a} represents a hydrogen atom; and
   na represents 2.
[Aspect C313] The compound according to the Aspect C24, wherein
   X^{1 a} represents CR^{1 a};
   R^{1 a} represents a hydrogen atom; and
   na represents 2.
[Aspect C314] The compound according to the Aspect C25, wherein
   X^{1 a} represents CR^{1 a};
   R^{1 a} represents a hydrogen atom; and
   na represents 2.
[Aspect C315] The compound according to the Aspect C26, wherein
   X^{1 a} represents CR^{1a};
   R^{1a} represents a hydrogen atom; and
   na represents 2.
[Aspect C316] The compound according to the Aspect C27, wherein
   X^{1 a} represents CR^{1 a};
   R^{1 a} represents a hydrogen atom; and
   na represents 2.
[Aspect C317] The compound according to the Aspect C28, wherein
   X^{1 a} represents CR^{1 a};
   R^{1 a} represents a hydrogen atom; and
   na represents 2.
[Aspect C318] The compound according to the Aspect C29, wherein
   X^{1 a} represents CR^{1 a};
   R^{1 a} represents a hydrogen atom; and
   na represents 2.
[Aspect C319] The compound according to the Aspect C30, wherein
   X^{1 a} represents CR^{1a};
   R^{1 a} represents a hydrogen atom; and
   na represents 2.
[Aspect C320] The compound according to the Aspect C31, wherein
   X^{1 a} represents CR^{1 a};
   R^{1 a} represents a hydrogen atom; and
   na represents 2.
[Aspect C321] The compound according to the Aspect C32, wherein
   X^{1 a} represents CR^{1 a};
   R^{1 a} represents a hydrogen atom; and
   na represents 2.
[Aspect C322] The compound according to the Aspect C33, wherein
   X^{1 a} represents CR^{1 a};
   R^{1 a} represents a hydrogen atom; and
   na represents 2.
[Aspect C323] The compound according to the Aspect C34, wherein
   X^{1 a} represents CR^{1 a};
   R^{1 a} represents a hydrogen atom; and
   na represents 2.
[Aspect C324] The compound according to the Aspect C35, wherein
   X^{1 a} represents CR^{1 a};
   R^{1 a} represents a hydrogen atom; and
   na represents 2.
[Aspect C325] The compound according to the Aspect C36, wherein
   X^{1 a} represents CR^{1 a};
   R^{1 a} represents a hydrogen atom; and
   na represents 2.
[Aspect C326] The compound according to the Aspect C37, wherein
   X^{1 a} represents CR^{1 a};
   R^{1 a} represents a hydrogen atom; and
   na represents 2.
[Aspect C327] The compound according to the Aspect C38, wherein
   X^{1 a} represents CR^{1 a};
   R^{1 a} represents a hydrogen atom; and
   na represents 2.
[Aspect C328] The compound according to the Aspect C39, wherein
   X^{1 a} represents CR^{1 a};
   R^{1 a} represents a hydrogen atom; and
   na represents 2.
[Aspect C329] The compound according to the Aspect C40, wherein
   X^{1 a} represents CR^{1 a};
   R^{1 a} represents a hydrogen atom; and
   na represents 2.
[Aspect C330] The compound according to the Aspect C41, wherein
   X^{1 a} represents CR^{1 a};
   R^{1 a} represents a hydrogen atom; and
   na represents 2.
[Aspect C331] The compound according to the Aspect C42, wherein
   X^{1 a} represents CR^{1 a};
   R^{1 a} represents a hydrogen atom; and
   na represents 2.
[Aspect C332] The compound according to the Aspect C43, wherein
   X^{1 a} represents CR^{1 a};
   R^{1 a} represents a hydrogen atom; and
   na represents 2.
[Aspect C333] The Compound N of the present invention, wherein X^{3 a} represents CR^{3 a}.
[Aspect C334] The Compound N of the present invention, wherein X^{3 a} represents a nitrogen atom.
[Aspect A1] A method for controlling Phakopsora pachyrhizi which comprises applying the Present compound to a soybean or soil for cultivating a soybean.
[Aspect A2] A seed or a vegetative reproductive organ holding an effective amount of the Present compound.
[Aspect AC1] A method for controlling Phakopsora pachyrhizi which comprises applying the Compound of the present invention to a soybean or soil for cultivating a soybean.

Next, production methods of the Present compounds (including Compounds of the present invention) are described.

### Production method A

A compound represented by formula (I-b) (hereinafter referred to as "Compound (I-b)") or a compound represented by formula (I-c) (hereinafter referred to as "Compound (I-c)") may be prepared by reacting a compound represented by formula (I-a) (hereinafter referred to as "Compound (I-a)") with an oxidizing agent. 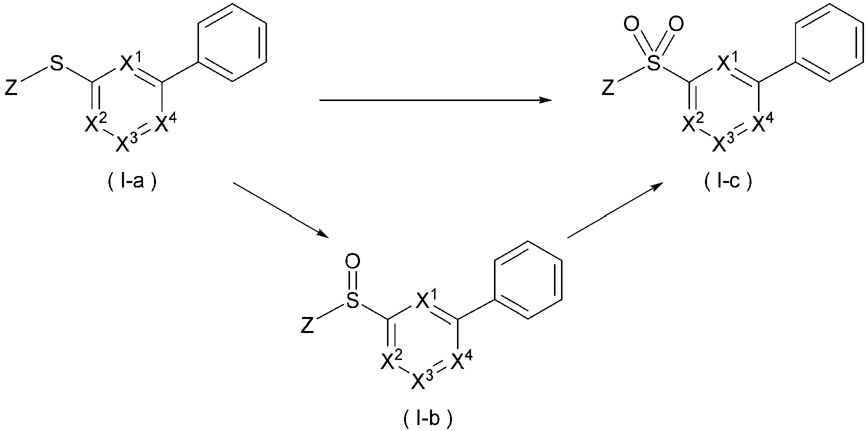 [wherein the symbols are the same as defined above.]

First, a method for producing the Compound (I-b) from the Compound (I-a) is described.

The reaction is usually carried out in a solvent. Examples of the solvent to be used in the reaction include halogenated hydrocarbons such as dichloromethane, chloroform, and chlorobenzene (hereinafter collectively referred to as "halogenated hydrocarbons"); nitriles such as acetonitrile and propionitrile (hereinafter collectively referred to as "nitriles"); alcohols such as methanol and ethanol (hereinafter collectively referred to as "alcohols"); acetic acid; water; and mixtures of two or more of them.

Examples of the oxidizing agent to be used in the reaction include sodium periodate, m-chloroperbenzoic acid (hereinafter referred to as "mCPBA"), and hydrogen peroxide.

When hydrogen peroxide is used as the oxidizing agent, a base or a catalyst may be used as needed.

Examples of the base to be used in the reaction include sodium carbonate. When a base is used in the reaction, the base is usually used at a ratio of 0.01 to 1 mol relative to 1 mol of the Compound (I-a).

Examples of the catalyst to be used in the reaction include sodium tungstate. When a catalyst is used in the reaction, the catalyst is usually used at a ratio of 0.01 to 0.5 mol relative to 1 mol of the Compound (I-a).

In the reaction, the oxidizing agent is usually used at a ratio of 1 to 1.2 mol relative to 1 mol of the Compound (I-a).

The reaction temperature is usually within the range of -20 to 80°C. The reaction time is usually within the range of 0.1 to 12 hour(s).

When the reaction is completed, to the reaction mixture is added water, the resulting mixture is subjected to extraction with organic solvent(s), and the resulting organic layer is washed with an aqueous solution of a reducing agent (for example, sodium sulfite or sodium thiosulfate) and an aqueous solution of a base (for example, sodium hydrogen carbonate) as needed. The resulting organic layer may be dried and/or concentrated to give the Compound (I-b).

Next, a method for producing the Compound (I-c) from the Compound (I-b) is described.

The reaction is usually carried out in a solvent. Examples of the solvent to be used in the reaction include halogenated hydrocarbons, nitriles, alcohols, acetic acid, water, and mixtures of two or more of them.

Examples of the oxidizing agent to be used in the reaction include mCPBA and hydrogen peroxide.

When hydrogen peroxide is used as the oxidizing agent, a base or a catalyst may be used as needed.

Examples of the base to be used in the reaction include sodium carbonate. When a base is used in the reaction, the base is usually used at a ratio of 0.01 to 1 mol relative to 1 mol of the Compound (I-b).

Examples of the catalyst to be used in the reaction include sodium tungstate. When a catalyst is used in the reaction, the catalyst is usually used at a ratio of 0.01 to 0.5 mol relative to 1 mol of the Compound (I-b).

In the reaction, the oxidizing agent is usually used at a ratio of 1 to 2 mol relative to 1 mol of the Compound (I-b).

The reaction temperature is usually within the range of -20 to 120°C. The reaction time is usually within the range of 0.1 to 12 hour(s).

When the reaction is completed, to the reaction mixture is added water, the resulting mixture is subjected to extraction with organic solvent(s), and the resulting organic layer is washed with an aqueous solution of a reducing agent (for example, sodium sulfite or sodium thiosulfate) and an aqueous solution of a base (for example, sodium hydrogen carbonate) as needed. The resulting organic layer may be dried and/or concentrated to give the Compound (I-c).

Also, the Compound (I-c) may be prepared in one step reaction (one-pot) by reacting the Compound (I-a) with an oxidizing agent.

The reaction may be carried out according to the method for producing the Compound (I-c) from the Compound (I-b) by usually using the oxidizing agent at a ratio of 2 to 5 mol relative to 1 mol of the Compound (I-a).

### Production method B

The Compound (I-a) may be prepared by reacting a compound represented by formula (B1) (hereinafter referred to as "Compound (B1)") with a compound represented by formula (M1) (hereinafter referred to as "Compound (M1)") in the presence of a base. 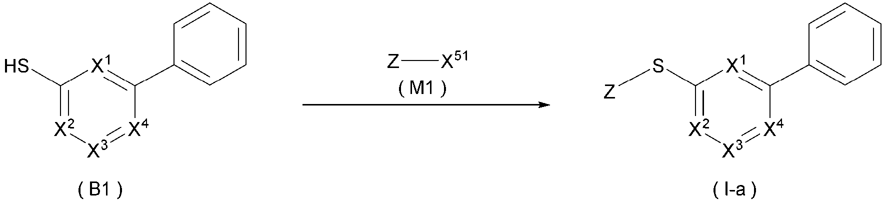 [wherein:
X^{5 1} represents a chlorine atom, a bromine atom, an iodine atom, a mesyloxy group, or a trifuryloxy group; and
the other symbols are the same as defined above.]

The reaction is usually carried out in a solvent. Examples of the solvent to be used in the reaction include hydrocarbons such as hexane, toluene, and xylene (hereinafter collectively referred to as "hydrocarbons"); ethers such as methyl tert-butyl ether (hereinafter referred to as "MTBE"), tetrahydrofuran (hereinafter referred to as "THF"), and dimethoxyethane (hereinafter referred to as "DME") (hereinafter collectively referred to as "ethers"); halogenated hydrocarbons; amides such as dimethylformamide (hereinafter referred to as "DMF") and N-methylpyrrolidone (hereinafter referred to as "NMP") (hereinafter collectively referred to as "amides"); esters such as methyl acetate and ethyl acetate (hereinafter collectively referred to as "esters"); nitriles; water; and mixtures of two or more of them.

Examples of the base include organic bases such as triethylamine and pyridine (hereinafter collectively referred to as "organic bases"); alkali metal carbonates such as sodium carbonate and potassium carbonate (hereinafter collectively referred to as "alkali metal carbonates"); alkali metal hydrogen carbonates such as sodium hydrogen carbonate and potassium hydrogen carbonate (hereinafter collectively referred to as "alkali metal hydrogen carbonates"); sodium hydride, and tripotassium phosphate.

In the reaction, a metal catalyst and/or a ligand may be used as needed .

Examples of the metal catalyst include copper catalysts such as copper(I) iodide, copper(I) bromide, copper(I) chloride, copper(I) oxide, copper(I) trifluoromethanesulfonate benzene complex, tetrakis(acetonitrile)copper(I) hexafluorophosphate, and copper(I) thiophene-2-carboxylate; nickel catalysts such as bis(cyclooctadiene)nickel(0) and nickel(II) chloride; and palladium catalysts such as palladium acetate, tris(dibenzylideneaceton)dipalladium(0), tetrakis(triphenylphosphine)palladium(0), and [1,1'-bis(diphenylphosphino)ferrocene]palladium(II) dichloride. When a metal catalyst is used in the reaction, the metal catalyst is usually used at a ratio of 0.01 to 1 mol relative to 1 mol of the Compound (B1).

Examples of the ligand include triphenylphosphine, Xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, 1,10-phenanthroline, trans-1,2-cyclohexanediamine, trans-N,N'-dimethylcyclohexane-1,2-diamine, N,N'-dimethylethylenediamine, and N,N-dimethylglycine hydrochloride. When a ligand is used in the reaction, the ligand is usually used at a ratio of 0.01 to 1 mol relative to 1 mol of the Compound (B1).

In the reaction, the Compound (M1) is usually used at a ratio of 1 to 10 mol, and the base is usually used at a ratio of 1 to 10 mol, relative to 1 mol of the Compound (B1).

The reaction temperature is usually within the range of 0 to 150°C. The reaction time is usually within the range of 0.1 to 48 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to isolate the Compound (I-a).

The Compound (M1) is known or may be prepared according to known method(s).

### Production method C

The compound represented by formula (I) may be prepared by reacting a compound represented by formula (B2) (hereinafter referred to as "Compound (B2)") with a compound represented by formula (M2) (hereinafter referred to as "Compound (M2)") in the presence of a palladium catalyst and a base. 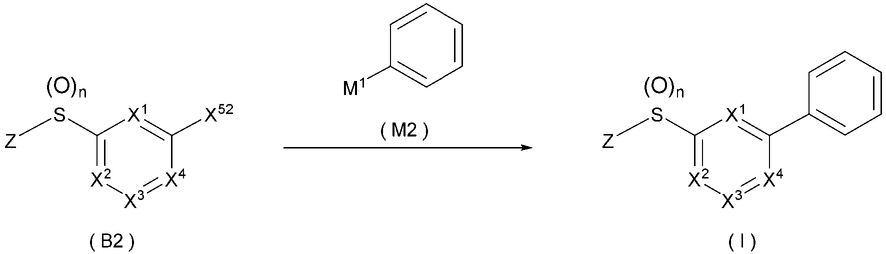 [wherein:
X^{5 2} represents a chlorine atom, a bromine atom, an iodine atom, or a trifuryloxy group;
M¹ represents B(OH)₂ or a 4,4,5,5-tetramethyl-1,3,2-dioxaborolan-2-yl group; and
the other symbols are the same as defined above.]

The reaction is usually carried out in a solvent. Examples of the solvent to be used in the reaction include hydrocarbons, ethers, halogenated hydrocarbons, amides, esters, nitriles, dimethyl sulfoxide (hereinafter referred to as "DMSO"), water, and mixtures of two or more of them.

Examples of the palladium catalyst to be used in the reaction include palladium(II) acetate and [1,1'-bis(diphenylphosphino)ferrocene]palladium(II) dichloride.

Examples of the base to be used in the reaction include organic bases, alkali metal carbonates, alkali metal hydrogen carbonates, sodium fluoride, and tripotassium phosphate.

In the reaction, the Compound (M2) is usually used at a ratio of 0.5 to 2 mol, the palladium catalyst is usually used at a ratio of 0.01 to 0.3 mol, and the base is usually used at a ratio of 1 to 10 mol, relative to 1 mol of the Compound (B2).

The reaction temperature is usually within the range of 0 to 150°C. The reaction time is usually within the range of 0.1 to 120 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to isolate the compound represented by formula (I).

The Compound (M2) is a commercially available compound or may also be prepared according to known method(s).

### Production method D

The compound represented by formula (I) may also be prepared by reacting a compound represented by formula (B3) (hereinafter referred to as "Compound (B3)") with a compound represented by formula (M3) (hereinafter referred to as "Compound (M3)") in the presence of a palladium catalyst and a base. 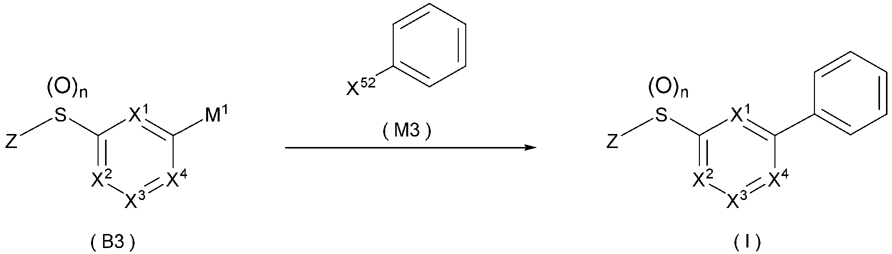 [wherein the symbols are the same as defined above.]

The reaction may be carried out according to the Production method C by using the Compound (M3) instead of the Compound (B2), and using the Compound (B3) instead of the Compound (M2).

The Compound (M3) is known or may be prepared according to known method(s).

### Production method E

The Compound (I-a) may also be prepared by reacting a compound represented by formula (B4) (hereinafter referred to as "Compound (B4)") with a compound represented by formula (M4) (hereinafter referred to as "Compound (M4)") in the presence of a base. 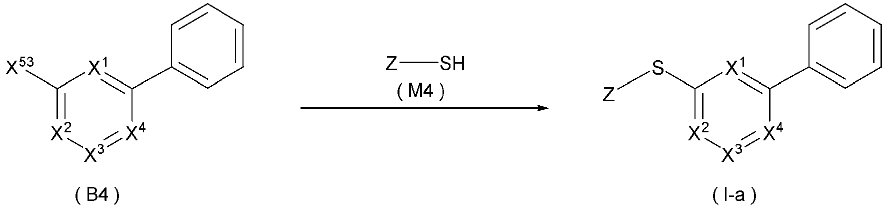 [wherein:
X^{5 3} represents a halogen atom, a mesyloxy group, or a trifuryloxy group; and
the other symbols are the same as defined above.]

The reaction is usually carried out in a solvent. Examples of the solvent to be used in the reaction include alcohols, nitriles, ethers, aromatic hydrocarbons, amides, water, and mixtures of two or more of them.

Examples of the base to be used in the reaction include alkali metal hydrides such as lithium hydride and sodium hydride, alkali metal carbonates, organic bases, sodium hydride, and tripotassium phosphate.

When X^{5 3} represents a bromine atom, an iodine atom, or a trifuryloxy group, a metal catalyst and a ligand may be used as needed.

Examples of the metal catalyst to be used in the reaction include palladium catalysts such as palladium(II) acetate and [1,1'-bis(diphenylphosphino)ferrocene]palladium(II) dichloride; nickel catalysts such as bis(cyclooctadiene)nickel(0) and nickel(II) chloride; and copper catalysts such as copper(I) iodide and copper(I) chloride. When a metal catalyst is used in the reaction, the metal catalyst is usually used at a ratio of 0.01 to 1 mol relative to 1 mol of the Compound (B4).

Examples of the ligand to be used in the reaction include triphenylphosphine, Xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, and 1,10-phenanthroline. When a ligand is used in the reaction, the ligand is usually used at a ratio of 0.01 to 1 mol relative to 1 mol of the Compound (B4).

In the reaction, the Compound (M4) is usually used at a ratio of 1 to 10 mol, and the base is usually used at a ratio of 1 to 10 mol, relative to 1 mol of the Compound (B4).

The reaction temperature is usually within the range of -20°C to 200°C. The reaction time is usually within the range of 0.1 to 72 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-a).

The Compound (M4) is known or may be prepared according to known method(s).

### Production method F

The Compound (I-c) may be prepared by reacting the Compound (B4) with a compound represented by formula (M5) (hereinafter referred to as "Compound (M5)"). 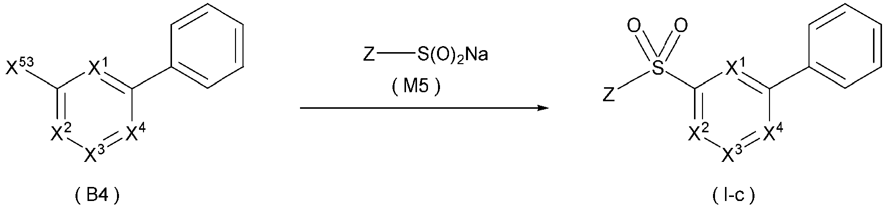 [wherein the symbols are the same as defined above.]

The reaction is usually carried out in a solvent. Examples of the solvent to be used in the reaction include hydrocarbons, ethers, halogenated hydrocarbons, amides, nitriles, DMSO, water, and mixtures of two or more of them.

In the reaction, the Compound (M5) is usually used at a ratio of 1 to 3 mol relative to 1 mol of the Compound (B4).

The reaction temperature is usually within the range of 0 to 150°C. The reaction time is usually within the range of 0.1 to 48 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to isolate the Compound (I-c).

The Compound (M5) is known or may be prepared according to known method(s).

### Reference Production method A

The Compound (B1) may be prepared by reacting the Compound (B4) with a sulfating agent. 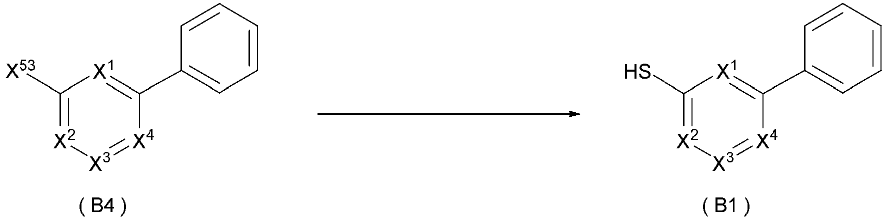 [wherein the symbols are the same as defined above.]

The reaction may be carried out according to the Production method E by using a sulfating agent such as hydrogen sulfide instead of the Compound (M4).

### Reference Production method B

The Compound (B4) may be prepared by reacting a compound represented by formula (C1) (hereinafter referred to as "Compound (C1)") with the Compound (M2) in the presence of a palladium catalyst and a base. 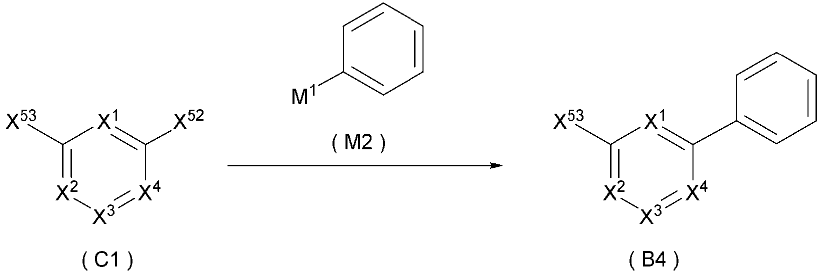 [wherein the symbols are the same as defined above.]

The reaction may be carried out according to the Production method C by using the Compound (C1) instead of the Compound (B2).

The Compound (C1) is known or may be prepared according to known method(s).

### Reference Production method C

A compound represented by formula (C3) (hereinafter referred to as "Compound (C3)") may be prepared by reacting a compound represented by formula (C2) (hereinafter referred to as "Compound (C2)") with the Compound (M1) in the presence of a base. 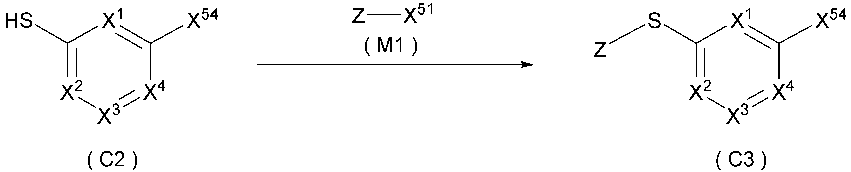 [wherein:
X^{5 4} represents a chlorine atom, a bromine atom, an iodine atom, a trifuryloxy group, or a 4,4,5,5-tetramethyl-1,3,2-dioxaborolan-2-yl group; and
the other symbols are the same as defined above.]

The reaction may be carried out according to the Production method B by using the Compound (C2) instead of the Compound (B1).

The Compound (C2) is known or may be prepared according to known method(s).

### Reference Production method D

A compound represented by formula (C4) (hereinafter referred to as "Compound (C4)") or a compound represented by formula (C5) (hereinafter referred to as "Compound (C5)") may be prepared by reacting the Compound (C3) with an oxidizing agent. 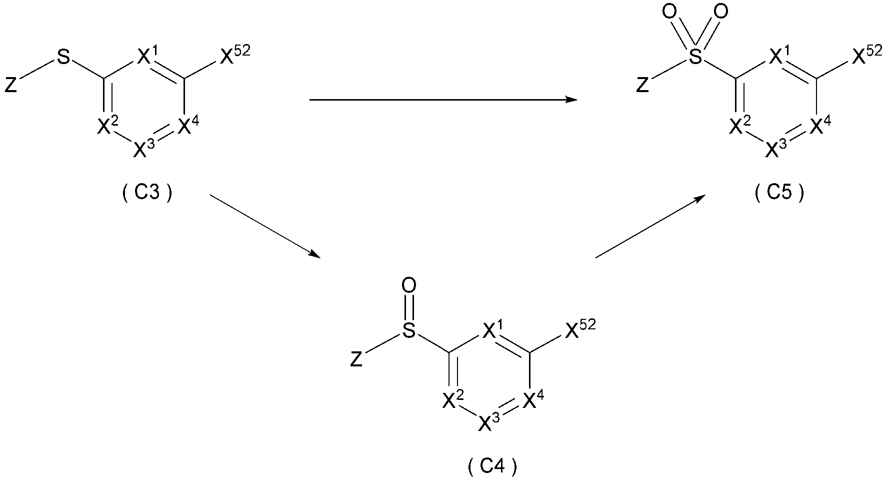 [wherein the symbols are the same as defined above.]

The reaction may be carried out according to the Production method A by using the Compound (C3) instead of the Compound (I-a), and using the Compound (C4) instead of the Compound (I-b).

### Reference Production method E

A compound represented by formula (C6) (hereinafter referred to as "Compound (C6)") may be prepared by reacting the Compound (B2) with bis(pinacolato)diboron in the presence of a base and a palladium catalyst. 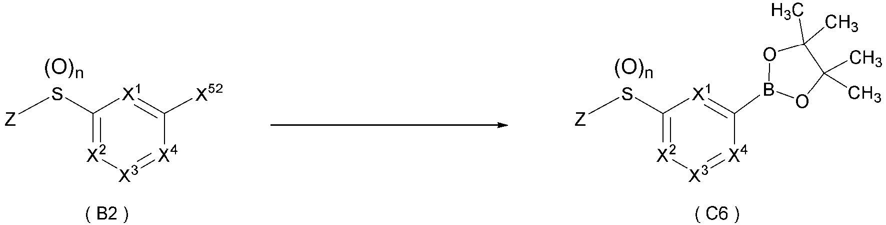 [wherein the symbols are the same as defined above.]

The reaction may be carried out according to the Production method C by using bis(pinacolato)diboron instead of the Compound (M2).

The Compound of the present invention may be mixed with or used in combination with one or more ingredient(s) selected from the group consisting of the following Group (a), Group (b), Group (c), and Group (d) (hereinafter referred to as "Present ingredient").

When the Compound of the present invention is mixed with or used in combination with the Present ingredient, they are used simultaneously, separately, or at time intervals with each other.

When the Compound of the present invention is used simultaneously with the Present ingredient, the Compound of the present invention and the Present ingredient may be contained in separate formulations or contained in one formulation.

One aspect of the present invention provides a composition comprising one or more ingredient(s) selected from the group consisting of Group (a), Group (b), Group (c), and Group (d) (i.e., Present ingredient), and the Compound of the present invention (hereinafter referred to as "Composition A").

Group (a) is a group consisting of acetylcholinesterase inhibitors (for example, carbamate insecticides and organophosphate insecticides), GABA-gated chloride channel blockers (for example, phenylpyrazole insecticides), sodium channel modulators (for example, pyrethroid insecticides), nicotinic acetylcholine receptor competitive modulators (for example, neonicotinoid insecticides), nicotinic acetylcholine receptor allosteric modulators, glutamate-gated chloride channel allosteric modulators (for example, macrolide insecticides), juvenile hormone mimics, multisite inhibitors, chordotonal organ TRPV channel modulators, mite growth inhibitors, microbial disruptors of insect midgut membranes, inhibitors of mitochondrial ATP synthase, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, mitochondrial complexes I, II, III, and IV electron transport inhibitors, voltage-dependent sodium channel blockers, inhibitors of acetyl CoA carboxylase, ryanodine receptor modulators (for example, diamide insecticides), chordotonal organ modulators, and microbial insecticides, and other insecticidal active ingredients, miticidal active ingredients, and nematicidal active ingredients. These ingredients are described in the classification on the basis of action mechanism by IRAC.

Group (b) is a group consisting of nucleic acids synthesis inhibitors (for example, phenylamide fungicides and acylamino acid fungicides), cell division and cytoskeleton inhibitors (for example, MBC fungicides), respiration inhibitors (for example, QoI fungicides and QiI fungicides), amino acids synthesis and protein synthesis inhibitors (for example, anilino-pyrimidine fungicides), signal transduction inhibitors, lipid synthesis and membrane synthesis inhibitors, sterol biosynthesis inhibitors (for example, DMI fungicides such as triazole fungicides), cell wall biosynthesis inhibitors, melanin synthesis inhibitors, plant defense inducers, fungicides with multi-site contact activity, microbial fungicides, and other fungicidal active ingredients. These ingredients are described in the classification on the basis of action mechanism by FRAC.

Group (c) is a group of plant growth regulatory ingredients (including mycorrhizal fungi and root nodule bacteria).

Group (d) is a group of repellent ingredients.

Hereinafter, examples of the combination of the Present ingredient and the Compound of the present invention are described. For example, "alanycarb + SX" indicates a combination of alanycarb and SX.

The abbreviation of "SX" indicates any one of the Compound of the present invention selected from the Compound groups SX1 to SX21. Also, all of the following Present ingredient are known ingredients, and may be obtained from commercially available formulations, or may be prepared by known methods. When the Present ingredient is a microorganism, it may also be available from a bacterial authority depository. Further, the number in parentheses represents the CAS RN (registered trademark).

Combinations of the Present ingredient in the above Group (a) and the Compound of the present invention:
abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alphacypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, bark of Celastrus angulatus + SX, bendiocarb + SX, benfluthrin + SX, benfuracarb + SX, bensultap + SX, bentioflumin + SX, benzoximate + SX, benzpyrimoxan + SX, beta-cyfluthrin + SX, beta-cypermethrin + SX, bifenazate + SX, bifenthrin + SX, bioallethrin + SX, bioresmethrin + SX, bistrifluron + SX, bisulflufen + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cybenzoxasulfyl + SX, cyclaniliprole + SX, cyclobutrifluram + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC(2-methyl-4,6-dinitrophenol) + SX, doramectin + SX, dried leaves of Dryopteris filix-mas + SX, emamectinbenzoate + SX, empenthrin + SX, endosulfan + SX, EPN(O-ethyl O-(4-nitrophenyl) phenylphosphonothioate) + SX, epsilon-metofluthrin + SX, epsilon-momfluorothrin + SX, esfenvalerate + SX, ethiofencarb + SX, ethion + SX, ethiprole + SX, ethoprophos + SX, etofenprox + SX, etoxazole + SX, extract of Artemisia absinthium + SX, extract of Azadirachta indica + SX, extract of Cassia nigricans + SX, extract of clitoria ternatea + SX, extract of Symphytum officinale + SX, extract of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, flupyroxystrobin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hv1a peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoflualanam + SX, isoprocarb + SX, isopropyl-O-(methoxyaminothiophosphoryl) salicylate + SX, isoxathion + SX, ivermectin + SX, kadethrin + SX, kappa-tefluthrin + SX, kappa-bifenthrin + SX, kinoprene + SX, lambda-cyhalothrin + SX, ledprona + SX, lenoremycin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, mivorilaner + SX, modoflaner + SX, momfluorothrin + SX, monocrotophos + SX, moxidectin + SX, naled + SX, nicofluprole + SX, nicotine + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pioxaniliprole + SX, piperflanilide + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirobudifen + SX, spirodiclofen + SX, spiromesifen + SX, spiropidion + SX, spirotetramat + SX, sulfiflumin + SX, sulfluramid + SX, sulfotep + SX, sulfoxaflor + SX, sulfur + SX, sulfuryl fluoride + SX, tartar emetic + SX, taufluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin + SX, tetramethylfluthrin + SX, tetraniliprole + SX, theta-cypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tiapyrachlor + SX, tigolaner + SX, tiorantraniliprole + SX, tioxazafen + SX, tolfenpyrad + SX, tralomethrin + SX, transfluthrin + SX, triazamate + SX, triazophos + SX, trichlorfon + SX, trifluenfuronate + SX, triflumezopyrim + SX, triflumuron + SX, trimethacarb + SX, tyclopyrazoflor + SX, umifoxolaner + SX, vadescana + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl)phenyl]-4,5-dihydro-1,2-oxazol-3-yl}indan-1-yl)propanamide (1118626-57-5) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, N-[4-chloro-2-(pyridin-3-yl)-1,3-thiazol-5-yl]-N-ethyl-3-(methanesulfonyl)propanamide + SX, 1,4-dimethyl-2-[2-(pyridin-3-yl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboxamide + SX, 7-fluoro-N-[1-(methylsulfanyl)-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido[2,3-b][1,4]oxazepin-10-one (2607927-97-7) + SX, Bacillus thuringiensis Cry1Ab protein + SX, Bacillus thuringiensis Cry1Ac protein + SX, Bacillus thuringiensis Cry1Fa protein + SX, Bacillus thuringiensis Cry1A.105 protein + SX, Bacillus thuringiensis Cry2Ab protein + SX, Bacillus thuringiensis Vip3A protein + SX, Bacillus thuringiensis mCry3A protein + SX, Bacillus thuringiensis Cry3Ab protein + SX, Bacillus thuringiensis Cry3Bb protein + SX, Bacillus thuringiensis Cry34Ab1/Cry35Ab1 protein + SX, Adoxophyes orana GV(granulovirus) strain BV-0001 + SX, Anticarsia gemmatalis MNPV(multiple nucleocapsid nucleopolyhedrovirus) + SX, Autographa californica MNPV + SX, Cydia pomonella GV strain V15 + SX, Cydia pomonella GV strain V22 + SX, Cryptophlebia leucotreta GV + SX, Dendrolimus punctatus cypovirus + SX, Helicoverpa armigera NPV(nucleopolyhedrovirus) strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua MNPV + SX, Spodoptera littoralis MNPV + SX, Spodoptera litura NPV + SX, Arthrobotrys dactyloides + SX, Bacillus firmus strain GB-126 + SX, Bacillus firmus strain I-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus thuringiensis strain AQ52 + SX, Bacillus thuringiensis strain BD#32 + SX, Bacillus thuringiensis strain CR-371 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306 + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis var. 7216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, Wolbachia pipientis + SX.

Combination of the Present ingredient in the above Group (b) and the Compound of the present invention:
acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper(II) acetate + SX, copper(II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Allium sativum + SX, extract of the cotyledons of lupine plantlets ("BLAD") + SX, extract of Equisetum arvense + SX, extract of Melaleuca alternifolia + SX, extract of Reynoutria sachalinensis + SX, extract of Tropaeolum majus + SX, famoxadone + SX, fenamidone + SX, fenaminstrobin + SX, fenarimol + SX, fenbuconazole + SX, feneptamidoquin + SX, fenfuram + SX, fenhexamid + SX, fenopyramid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin + SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, galquin + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder + SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R, 2S, 5S)-5-(4-chlorobenzyl)-2-(chloromethyl)-2-methyl-1-(1H-1,2,4-triazol-1-ylmethyl)cyclopentan-1-ol (1801930-06-2) + SX, (1S, 2R, 5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R, 2S, 5S)-2-(chloromethyl)-5-(4-fluorobenzyl)-2-methyl-1-(1H-1,2,4-triazol-1-ylmethyl)cyclopentan-1-ol (1801930-08-4) + SX, (1S, 2R, 5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl]ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, 4,4-dimethyl-2-({4-[5-(trifluoromethyl)-1,2,4-oxadiazol-3-yl]phenyl}methyl)isoxazolidin-3-one (2098918-25-1) + SX, 5,5-dimethyl-2-({4-[5-(trifluoromethyl)-1,2,4-oxadiazol-3-yl]phenyl}methyl)isoxazolidin-3-one (2098918-26-2) + SX, N-ethyl-2-methyl-N-({4-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl}methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl)butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl)carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, N-({4-[5-(trifluoromethyl)-1,2,4-oxadiazol-3-yl]phenyl}methyl)cyclopropanecarboxamide + SX, N-allyl-N-({4-[5-(trifluoromethyl)-1,2,4-oxadiazol-3-yl]phenyl}methyl)acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl}methyl)propanamide + SX, 3,3,3-trifluoro-N-({2-fluoro-4-[5-(trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({ 4-[5-(trifluoromethyl)-1,2,4-oxadiazol-3-yl]phenyl}methyl)urea + SX, N,N-diethyl-N'-({ 4-[5-(trifluoromethyl)-1,2,4-oxadiazol-3-yl]phenyl}methyl)urea + SX, N-methyl-N'-({ 4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl)pyrrolidin-2-one + SX, 1-({4-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl]phenyl}methyl)isoxazolidin-3-one + SX, 3,3-dimethyl-1-({4-[5-(trifluoromethyl)-1,2,4-oxadiazol-3-yl]phenyl}methyl)piperidin-2-one + SX, 2-({4-[5-(trifluoromethyl)-1,2,4-oxadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl)pyrrolidin-2-one + SX, 5-methyl-1-({4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-5-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl)(2,6-difluoropyridin-4-yl)amino]-N-(2,2-dimethylcyclobutyl)-5-methylthiazole-4-carboxamide + SX, 2-[(2-methylpropanoyl)(2,6-difluoropyridin-4-yl)amino]-N-(2,2-dimethylcyclobutyl)-5-methylthiazole-4-carboxamide + SX, 2-[(2,6-difluoropyridin-4-yl)amino]-N-(2,2-dimethylcyclobutyl)-5-methylthiazole-4-carboxamide + SX, 2-{[(oxetan-3-yl)carbonyl](2,6-difluoropyridin-4-yl)amino}-N-(2,2-dimethylcyclobutyl)-5-methylthiazole-4-carboxamide + SX, 2-{[(oxolan-3-yl)carbonyl](2,6-difluoropyridin-4-yl)amino}-N-(2,2-dimethylcyclobutyl)-5-methylthiazole-4-carboxamide + SX, 2-{[(oxan-4-yl)carbonyl](2,6-difluoropyridin-4-yl)amino}-N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl]pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2-methylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2,2-dimethylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(5-fluoro-2-methoxyphenyl)-2-(2-cyanoethoxy)ethyl]-5-[1-(isopropoxyimino)ethyl]-2,6-dioxo-3,6-dihydropyrimidin-1(2H)-yl}propan-2-yl)-2-methylpropanamide + SX, methyl ({5-[1-(2,6-difluoro-4-isopropylphenyl)-1H-pyrazol-3-yl]-2-methylphenyl}methyl)carbamate + SX, methyl ({5-[1-(2,6-difluoro-4-cyclopropylphenyl)-1H-pyrazol-3-yl]-2-methylphenyl}methyl)carbamate + SX, methyl ({5-[1-(2,6-difluoro-4-methoxyphenyl)-1H-pyrazol-3-yl]-2-methylphenyl}methyl)carbamate + SX, methyl (Z)-2-(5-cyclopentyl-2-methylphenoxy)-3-methoxyprop-2-enoate + SX, methyl (Z)-2-(5-cyclohexyl-2-methylphenoxy)-3-methoxyprop-2-enoate + SX, methyl (Z)-2-[(3-isopropyl-1H-pyrazol-1-yl)-2-methylphenoxy]-3-methoxyprop-2-enoate + SX, 1-(4,5-dimethyl-1H-benzimidazol-1-yl)-4,4-difluoro-3,3-dimethyl-3,4-dihydroisoquinoline + SX, 1-(4,5-dimethyl-1H-benzimidazol-1-yl)-4,4,5-trifluoro-3,3-dimethyl-3,4-dihydroisoquinoline + SX, 1-(pyrazolo[1,5-a]pyridin-3-yl)-4,4-difluoro-3,3-dimethyl-3,4-dihydroisoquinoline + SX, 1-(6,7-dimethylpyrazolo[1,5-a]pyridin-3-yl)-6-fluoro-3,3-dimethylisoquinolin-4(3H)-one + SX, methyl (2Z)-3-methoxy-2-[(4-methyl[1,1'-biphenyl]-3-yl)oxy]prop-2-enoate + SX, Agrobacterium radiobactor strain K1026 + SX, Agrobacterium radiobactor strain K84 + SX, Bacillus amyloliquefaciens strain PTA-4838 (Aveo (registered trademark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerans strain E325 + SX, Phlebiopsis gigantea strain VRA1992 + SX, Pseudomonas aureofaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G-41 + SX, Trichoderma virens strain GL-21 + SX, Trichoderma viride + SX, Variovorax paradoxus strain CGF4526 + SX, Harpin protein + SX.

Combination of the Present ingredient in the above Group (c) and the Compound of the present invention:
1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadione-calcium + SX, prohydrojasmon + SX, pyraflufenethyl + SX, sintofen + SX, sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butylate + SX, methyl 5-(trifluoromethyl)benzo[b]thiophene-2-carboxylate + SX, 3-[(6-chloro-4-phenylquinazolin-2-yl)amino]propan-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Zucchini Yellow Mosaik Virus weak strain + SX.

Combination of the Present ingredient in the above Group (d) and the Compound of the present invention:
anthraquinone + SX, deet + SX, icaridin + SX.

The ratio of the Compound of the present invention to the Present ingredient includes, but not limited thereto, as a ratio by weight (the Compound of the present invention : the Present ingredient) 1,000 : 1 to 1 : 1,000, 500 : 1 to 1 : 500, 100 : 1 to 1 : 100, 50 : 1, 20 : 1, 10 : 1, 9 : 1, 8 : 1, 7 : 1, 6 : 1, 5 : 1, 4 : 1, 3 : 1, 2 : 1, 1 : 1, 1 : 2, 1 : 3, 1 : 4, 1 : 5, 1 : 6, 1 : 7, 1 : 8, 1 : 9, 1 : 10, 1 : 20, 1 : 50, and the others.

The Present compound, the Compound of the present invention, or the Composition A can control plant diseases caused by plant pathogenic microorganisms such as fungi, Oomycete, Phytomyxea, and bacteria. Examples of the fungi include Ascomycota, Basidiomycota, Blastocladiomycota, Chytridiomycota, Mucoromycota, and Olpidiomycota. Specific examples thereof include the followings. The scientific name of plant pathogenic microorganism which causes each disease is shown in parentheses.

Rice diseases:
   blast (Pyricularia oryzae), brown spot (Cochliobolus miyabeanus), sheath blight (Rhizoctonia solani), bakanae disease (Gibberella fujikuroi), downy mildew (Sclerophthora macrospora), false blast and head blight (Epicoccum nigrum), seedling blight (Trichoderma viride and Rhizopus oryzae), pseudo sheath blight (Waitea circinate, Ceratobasidium setariae, and Thanatephorus cucumeris), Rice false smut (Ustilaginoidea virens), and Kernel smut (Tilletia horrida and Tilletia barclayana);
Wheat diseases:
   powdery mildew (Blumeria graminis), fusarium blight (Fusarium graminearum, Fusarium avenaceum, Fusarium culmorum, and Microdochium nivale), stripe rust (Puccinia striiformis), stem rust (Puccinia graminis), leaf rust (Puccinia recondita), snow mould (Microdochium nivale and Microdochium majus), typhula snow blight (Typhula incarnata and Typhula ishikariensis), Sclerotinia snow blight (Sclerotinia borealis), Browning root rot (Pythium spp.), loose smut (Ustilago tritici), stinking smut (Tilletia caries and Tilletia controversa), eyespot (Pseudocercosporella herpotrichoides), leaf blotch (Septoria tritici), glume blotch (Stagonospora nodorum), tan spot (Pyrenophora tritici-repentis), rhizoctonia seeding blight (Rhizoctonia solani), take-all disease (Gaeumannomyces graminis), and blast (Pyricularia graministritici) ;
Barley diseases:
   powdery mildew (Blumeria graminis), fusarium head blight (Fusarium graminearum, Fusarium avenaceum, Fusarium culmorum, and Microdochium nivale), stripe rust (Puccinia striiformis), stem rust (Puccinia graminis), dwarf leaf rust (Puccinia hordei), loose smut (Ustilago nuda), scald (Rhynchosporium secalis), net blotch (Pyrenophora teres), spot blotch (Cochliobolus sativus), stripe (Pyrenophora graminea), Ramularia disease (Ramularia collo-cygni), and rhizoctonia seeding blight (Rhizoctonia solani);
Corn diseases:
   rust (Puccinia sorghi), southern rust (Puccinia polysora), northern leaf blight (Setosphaeria turcica), tropical rust (Physopella zeae), southern leaf blight (Cochliobolus heterostrophus), anthracnose (Colletotrichum graminicola), gray leaf spot (Cercospora zeae-maydis), eyespot (Kabatiella zeae), phaeosphaeria leaf spot (Phaeosphaeria maydis), diplodia rot (Stenocarpella maydis and Stenocarpella macrospora), stalk rot (Fusarium graminearum, Fusarium verticilioides, and Colletotrichum graminicola), smut (Ustilago maydis), Physoderma brown spot and Physoderma stalk rot (Physoderma maydis), and tar spot disease (Phyllachora maydis);
Cotton diseases:
   anthracnose (Colletotrichum gossypii), grey mildew (Ramularia areola), alternaria leaf spot (Alternaria macrospora and Alternaria gossypii), and black root rot (Thielaviopsis basicola);
Coffee diseases:
   rust (Hemileia vastatrix) and leaf spot (Cercospora coffeicola);
Rape seed diseases:
   sclerotinia rot (Sclerotinia sclerotiorum), gray leaf spot (Alternaria brassicae), root rot (Phoma lingam), and light leaf spot (Pyrenopeziza brassicae);
Sugar cane diseases:
   rust (Puccinia melanocephela and Puccinia kuehnii), and smut (Ustilago scitaminea);
Sunflower diseases:
   rust (Puccinia helianthi) and downy mildew (Plasmopara halstedii) ;
Citrus diseases:
   melanose (Diaporthe citri), scab (Elsinoe fawcetti), green mold (Penicillium digitatum), blue mold (Penicillium italicum), Phytophthora rot (Phytophthora parasitica and Phytophthora citrophthora), aspergillus rot (Aspergillus niger), sour rot (Geotrichum candidum var. citri-aurantii), and sooty spot (Cladosporium cladosporioides);
Apple diseases:
   blossom blight (Monilinia mali), valsa canker (Valsa ceratosperma), powdery mildew (Podosphaera leucotricha), alternaria leaf spot (Alternaria alternata apple pathotype), scab (Venturia inaequalis), bitter rot (Glomerella cingulata and Colletotrichum acutatum), blotch (Diplocarpon mali), ring rot (Botryosphaeria berengeriana), crown rot (Phytophtora cactorum), and rust (Gymnosporangium juniperivirginianae and Gymnosporangium yamadae);
Pear diseases:
   scab (Venturia nashicola and Venturia pirina), black spot (Alternaria alternata Japanese pear pathotype), and rust (Gymnosporangium haraeanum);
Peach diseases:
   brown rot (Monilinia fructicola), scab (Cladosporium carpophilum), Phomopsis rot (Phomopsis sp.), leaf curl (Taphrina deformans), and powdery mildew (Podosphaera leucotricha);
Grapes diseases:
   anthracnose (Elsinoe ampelina), ripe rot (Glomerella cingulata and Colletotrichum acutatum), powdery mildew (Uncinula necator), rust (Neophysopella sp.), black rot (Guignardia bidwellii), downy mildew (Plasmopara viticola), leaf blight (Pseudocercospora vitis), and white rot (Coniella castaneicola);
Japanese persimmon diseases:
   anthracnose (Gloeosporium kaki and Colletotrichum acutatum), leaf spot (Cercospora kaki and Mycosphaerella nawae), and powdery mildew (Phyllactinia kakicola);
Fig disease:
   rust (Phakopsora nishidana);
Diseases of gourd family:
   anthracnose (Colletotrichum lagenarium), powdery mildew (Sphaerotheca fuliginea), gummy stem blight (Didymella bryoniae), Corynespora leaf spot (Corynespora cassiicola), fusarium wilt (Fusarium oxysporum), downy mildew (Pseudoperonospora cubensis), phytophthora rot (Phytophthora capsici), damping-off (Pythium sp.), and Black root rot (Phomopsis sp.);
Tomato diseases:
   early blight (Alternaria solani), leaf mold (Cladosporium fulvum), Cercospora leaf mold (Pseudocercospora fuligena), late blight (Phytophthora infestans), and powdery mildew (Leveillula taurica);
Eggplant diseases:
   brown spot (Phomopsis vexans), powdery mildew (Erysiphe cichoracearum), black blight (Corynespora melongenae), leaf mold (Mycovellosiella nattrassii), and brown leaf spot (Thanatephorus cucumeris);
Cruciferous vegetables diseases:
   alternaria leaf spot (Alternaria japonica), white spot (Cercosporella brassicae), clubroot (Plasmodiophora brassicae), downy mildew (Peronospora parasitica), white rust (Albugo candida), and Alternaria sooty spot (Alternaria brassicicola);
Welsh onion diseases:
   rust (Puccinia allii), black spot (Alternaria porri), white rot (Sclerotium cepivorum), leaf blight (Botrytis squamosa), leaf spot (Stemphylium vesicarium and Stemphylium botryosum), southern blight (Sclerotium rolfsii), leaf blight (Botrytis squamosa);
Soybean diseases:
   purple stain (Cercospora kikuchii), sphaceloma scab (Elsinoe glycines), pod and stem blight (Diaporthe phaseolorum var. sojae), rust (Phakopsora pachyrhizi), target spot (Corynespora cassiicola), anthracnose (Colletotrichum glycines and Colletotrichum truncatum), Rhizoctonia rot (Rhizoctonia solani), septoria brown spot (Septoria glycines), Cercospora leaf spot (Cercospora sojina), stem rot (Sclerotinia sclerotiorum), powdery mildew (Microsphaera diffusa), phytophthora stem and root rot (Phytophthora sojae), downy mildew (Peronospora manshurica), sudden death syndrome (Fusarium virguliforme), red crown rot (Calonectria ilicicola), and Diaporthe/Phomopsis complex (Diaporthe longicolla, Diaporthe phaseolorum, Phomopsis longicolla), and pod anomalies caused by various causative bacteria;
Kidney bean diseases:
   stem rot (Sclerotinia sclerotiorum), rust (Uromyces appendiculatus), angular leaf spot (Phaeoisariopsis griseola), anthracnose (Colletotrichum lindemuthianum), and Fusarium root-rot (Fusarium solani);
Peanut diseases:
   leaf spot (Cercospora personata), brown leaf spot (Cercospora arachidicola), southern blight (Sclerotium rolfsii), and Cylindrocladium black rot (Calonectria ilicicola) ;
Garden pea diseases:
   powdery mildew (Erysiphe pisi) and root rot (Fusarium solani);
Potato diseases:
   early blight (Alternaria solani), late blight (Phytophthora infestans), Pink rot (Phytophthora erythroseptica), powdery scab (Spongospora subterranea f. sp. subterranea), verticillium wilt (Verticillium albo-atrum, Verticillium dahliae, and Verticillium nigrescens), dry rot (Fusarium solani), and potato wart (Synchytrium endobioticum);
Strawberry diseases:
   powdery mildew (Sphaerotheca humuli) and crown rot (Glomerella cingulata and Colletotrichum acutatum);
Tea diseases:
   net blister blight (Exobasidium reticulatum), white scab (Elsinoe leucospila), gray blight (Pestalotiopsis sp.), and anthracnose (Colletotrichum theae-sinensis);
Tobacco diseases:
   brown spot (Alternaria longipes), anthracnose (Colletotrichum tabacum), blue mold (Peronospora tabacina), and black shank (Phytophthora nicotianae);
Sugar beet diseases:
   cercospora leaf spot (Cercospora beticola), leaf blight (Thanatephorus cucumeris), root rot (Thanatephorus cucumeris), aphanomyces root rot (Aphanomyces cochlioides), and rust (Uromyces betae);
Rose diseases:
   black spot (Diplocarpon rosae) and powdery mildew (Sphaerotheca pannosa);
Chrysanthemum diseases:
   leaf blight (Septoria chrysanthemi-indici) and white rust (Puccinia horiana);
Onion diseases:
   botrytis leaf blight (Botrytis cinerea, Botrytis byssoidea, and Botrytis squamosa), gray-mold neck rot (Botrytis allii), and small sclerotial neck rot (Botrytis squamosa);
Various crops diseases:
   Botrytis rot (Botrytis cinerea), sclerotinia rot (Sclerotinia sclerotiorum), white rot (Sclerotium cepivorum), southern blight (Sclerotium rolfsii), and seedling blight (Pythium aphanidermatum, Pythium irregulare, and Pythium ultimum);
Japanese radish disease:
   alternaria leaf spot (Alternaria brassicicola);
Sweet potato diseases:
   stem rot (Fusarium oxysporum f. sp. batatas), foot rot (Diaporthe destruens), and black rot (Ceratocystis fimbriata);
Turfgrass diseases:
   dollar spot (Sclerotinia homoeocarpa), brown patch and large patch (Rhizoctonia solani), pythium blight (Pythium aphanidermatum), and anthracnose (Colletotrichum caudatum);
Banana disease:
   Sigatoka disease (Mycosphaerella fijiensis and Mycosphaerella musicola);
Lentils disease:
   ascochyta blight (Ascochyta lentis);
Chickpea disease:
   ascochyta blight (Ascochyta rabiei);
Green pepper diseases:
   anthracnose (Colletotrichum scovillei), powdery mildew (Leveillula taurica), and southern blight (Sclerotium rolfsii);
Mango disease:
   anthracnose (Colletotrichum acutatum);
Cacao diseases:
   Witches' broom (Moniliophthora perniciosa) and Frosty pod rot (Moniliophthora roreri);
Pecan disease:
   scab (Venturia effusa);
Lychee diseases:
   downy mildew (Peronophythora litchii) and anthracnose (Colletotrichum siemense);
Fruit trees diseases:
   white root rot (Rosellinia necatrix) and violet root rot (Helicobasidium mompa);
Postharvest disease of fruits (for example, apple and pear) :
   Mucor rot diseases (Mucor piriformis);
Seed diseases or diseases in the early stages of the growth of various plants caused by Aspergillus spp., Penicillium spp., Fusarium spp., Gibberella spp., Tricoderma spp., Thielaviopsis spp., Rhizopus spp., Mucor spp., Corticium spp., Phoma spp., Rhizoctonia spp. or Diplodia spp., or the like;
Viral diseases:
   Lettuce big-vein disease transmitted by Olpidium brassicae, and viral diseases of several crops transmitted by Polymyxa spp. (e.g., Polymyxa betae and Polymyxa graminis);
Diseases caused by bacteria:
   bacterial seedling blight of rice (Burkholderia plantarii), bacterial palea browning of rice (Pantoea ananatis), Bacterial leaf blight of rice (Xanthomonas oryzae pv. oryzae.), bacterial spot of cucumber (Pseudomonas syringae pv. lachrymans), bacterial wilt of eggplant (Ralstonia solanacearum), canker of citrus (Xanthomonas citri), bacterial soft rot of Chinese cabbage (Erwinia carotovora), scab of potato (Streptomyces scabiei), black leg of potato (Pectobacterium carotovorum and Dickeya sp.), bacterial leaf spot of peach (Pseudomona syringae, Erwinia nigrifluens, and Xanthomonas campestris), bacterial canker of Japanese apricot (Prunus mume) (Pseudomonas syringae pv. morsprunorum and Erwinia sp.), Goss's wilt of corn (Clavibacter michiganensis), Pierce's disease of grapes, olive, peach, and the like (Xylella fastidiosa), and crown gall of Rosaceae plants such as apple, peach, and cherries (Agrobacterium tumefaciens);
and the others.

A rust fungus of soybean having an amino acid substitution of F129L in the mitochondrial cytochrome b protein means a rust fungus of soybean (scientific name: Phakopsora pachyrhizi) which has a mutation in the mitochondrial cytochrome b gene encoding the mitochondrial cytochrome protein, and as a result of said mutation, has an amino acid substitution of F129L, thereby shows resistance to QoI fungicides.

The method for controlling a plant disease of the present invention is carried out by applying an effective amount of the Present compound, the Compound of the present invention, or the Composition A to a plant or soil for cultivating a plant. Examples of the treatment method include foliage treatment, soil treatment, and seed treatment.

The Present compound, the Compound of the present invention, or the Composition A is usually used by mixing it with inert carrier(s) such as solid carrier(s) and liquid carrier(s), surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, or the like. In addition to these formulations, the Present compound, the Compound of the present invention, or the Composition A may be used by formulating it into a dosage form described in Manual on development and use of FAO and WHO Specifications for pesticides, FAO Plant Production and Protection Papers- 271-276, prepared by the FAO/WHO Joint Meeting on Pesticide Specifications, 2016, ISSN:0259-2517.

These formulations usually comprise 0.0001% to 99% by weight ratio of the Present compound, the Compound of the present invention, or the Composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

Also, adjuvant(s) may be used as ingredient(s) for enhancing or assisting the efficacy of the Present compound, the Compound of the present invention, or the Composition A. Specific examples thereof include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol A8 (registered trademark), NP-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANOLE (registered trademark).

In the present invention, the plants include whole plants and specific parts of plants. Examples of the specific parts of plants include foliages, flowers, ears, fruits, tree stems, branches, tree crowns, seeds, vegetative reproductive organs, and nursery plants.

A vegetative reproductive organ means a part of plant such as root, stem, and leaf which has a growth capability even when said part is separated from the plant body and placed into soil. Examples of the vegetative reproductive organ include tuberous root, creeping root, bulb, corm or solid bulb, tuber, rhizome, stolon, rhizophore, cane cuttings, propagule, and vine cutting. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous root are also collectively referred to as "bulb". For example, cultivation of potato starts with planting a tuber into soil, and the tuber to be used is generally referred to as "seed potato".

Nursery plants include seedlings and saplings.

Examples of a method for controlling plant diseases by applying an effective amount of the Present compound, the Compound of the present invention, or the Composition A to soil include a method of applying an effective amount of the Present compound, the Compound of the present invention, or the Composition A to soil before planting plants or after planting plants. Specifically, examples of the application method include planting hole treatment (for example, spraying into planting holes and soil mixing after planting hole treatment), plant foot treatment (for example, plant foot spraying, soil mixing after plant foot treatment, irrigation at plant foot, and plant foot treatment at a later seeding raising stage), planting furrow treatment (for example, planting furrow spraying and soil mixing after planting furrow treatment), planting row treatment (for example, planting row spraying, soil mixing after planting row treatment, and planting row spraying at a growing stage), planting row treatment at the time of sowing (for example, planting row spraying at the time of sowing and soil mixing after planting row treatment at the time of sowing), broadcast treatment (for example, overall soil surface spraying and soil mixing after broadcast treatment), side-article treatment, treatment of water surface (for example, application to water surface and application to water surface after flooding), other soil spraying treatment (for example, spraying of a granular formulation on leaves at a growing stage, spraying under a canopy or around a tree stem, spraying on the soil surface, mixing with surface soil, spraying into seed holes, spraying on the ground surfaces of furrows, and spraying between plants), other irrigation treatment (for example, soil irrigation, irrigation at a seedling raising stage, chemical solution injection treatment, irrigation of a plant part just above the ground, chemical solution drip irrigation, and chemigation), seedling raising box treatment (for example, spraying into a seedling raising box, irrigation of a seedling raising box, and flooding into a seedling raising box with chemical solution), seedling raising tray treatment (for example, spraying on a seedling raising tray, irrigation of a seedling raising tray, and flooding into a seedling raising tray with chemical solution), seedbed treatment (for example, spraying on a seedbed, irrigation of a seedbed, spraying on a lowland rice nursery, and immersion of seedlings), seedbed soil incorporation treatment (for example, mixing with seedbed soil, mixing with seedbed soil before sowing, spraying at sowing before covering with soil, spraying at sowing after covering with soil, and mixing with covering with soil), and other treatment (for example, mixing with culture soil, plowing under, mixing with surface soil, mixing with soil at the place where raindrops fall from a canopy, treatment at a planting position, spraying of a granule formulation on flower clusters, and mixing with a paste fertilizer).

Examples of the application to seeds (or seed treatments) include an application of the Present compound, the Compound of the present invention, or the Composition A to seeds or vegetative reproductive organs, and specific examples thereof include spraying treatment in which a suspension of the Present compound, the Compound of the present invention, or the Composition A is sprayed onto seed surface or the vegetative reproductive organ surface in the form of mist; smearing treatment in which the Present compound, the Compound of the present invention, or the Composition A is coated on a surface of seeds or the vegetative reproductive organs; a soaking treatment in which the seeds or vegetative reproductive organs are soaked into the solution of the Present compound, the Compound of the present invention, or the Composition A for a certain time; and a method for coating the seeds or the vegetative reproductive organs with a carrier containing the Present compound, the Compound of the present invention, or the Composition A (for example, film coating treatment and pellet coating treatment). Examples of the above-described vegetative reproductive organ include particularly seed potato.

As used herein, seeds or vegetative reproductive organs holding the Present compound, the Compound of the present invention, or the Composition A mean seeds or vegetative reproductive organs in the state where the Present compound, the Compound of the present invention, or the Composition A is adhered to a surface of the seeds or the vegetative reproductive organs. The above-described seeds or vegetative reproductive organs holding the Present compound, the Compound of the present invention, or the Composition A may be adhered by any other materials that are different from the Present compound, the Compound of the present invention, or the Composition A before or after being adhered by the Present compound, the Compound of the present invention, or the Composition A to the seeds or vegetative reproductive organs.

Also, when the Composition A is adhered in a form of layer(s) to a surface of seeds or vegetative reproductive organs, the layer(s) is/are composed of one layer or multiple layers. Also, when multiple layers are formed, each of the layer may be composed of a layer comprising one or more active ingredients, or a combination of a layer comprising one or more active ingredients and a layer not comprising an active ingredient.

Seeds or vegetative reproductive organs holding the Present compound, the Compound of the present invention, or the Composition A can be obtained, for example, by applying the formulations comprising the Present compound, the Compound of the present invention, or the Composition A by the above-described application method to seeds or vegetative reproductive organs.

The amount of the Present compound, the Compound of the present invention, or the Composition A to be applied varies depending on the climate condition, the dosage form, the application period, the application method, the application site, diseases to be controlled, crops to be protected, and the like, and is usually within the range of 1 g to 500 g per 1000 m² of area for cultivating plants in case of foliage treatment or soil treatment. In case of seed treatment, the amount of the Present compound, the Compound of the present invention, or the Composition A is usually within the range of 0.001 g to 100 g per 1 Kg of seed. When the Present compound, the Compound of the present invention, or the Composition A is formulated into an emulsifiable concentrate, a wettable powder, a flowable, or the like, such formulation is usually applied after diluting it with water in such a way that a concentration of the active ingredient is within the range of 0.01 ppm to 10000 ppm, and then sparging it. In the case of being formulated into a dust formulation, a granule, or the like, such formulation is usually used as itself without diluting it.

The Present compound, the Compound of the present invention, or the Composition A may be used as an agent for controlling plant diseases in croplands such as fields, paddy fields, grasses, and orchards. Examples of the plants include the followings.
corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

### EXAMPLES

Hereinafter, the present invention is illustrated more in detail by Preparation Examples, Reference Preparation Examples, Formulation Examples, and Test Examples, but the present invention is not limited to these Examples only.

In the present description, Me represents a methyl group, Et represents an ethyl group, Pr represents a propyl group, i-Pr represents an isopropyl group, c-Pr represents a cyclopropyl group, Bu represents a butyl group, Ph represents a phenyl group, 2-Thie represents a 2-thienyl group, 3-Thie represents a 3-thienyl group, 2-Fura represents a 2-furanyl group, 3-Fura represents a 3-furanyl group, 2-Py represents a 2-pyridyl group, 3-Py represents a 3-pyridyl group, 4-Py represents a 4-pyridyl group, 2-Pyrazin represents a pyrazin-2-yl group, 2-Pyrimid represents a pyrimidin-2-yl group, 4-Pyrimid represents a pyrimidin-4-yl group, c-Pen represents a cyclopentyl group, and 2-Me-3-Tet-fura represents a 2-methyl-tetrahydrofuran-3-yl group. When these groups have substituent(s), the substituent(s) is/are indicated before the symbols of these groups with the substitution position(s). For example, 4-OMe-Ph represents a 4-methoxyphenyl group, and 3,5-Me₂-Ph represents a 3,5-dimethylphenyl group.

When a physical property of a compound is measured by liquid chromatography / mass spectrometry (hereinafter referred to as "LC/MS"), the measured molecular ion value [M+H]⁺ or [M-H]⁻, and retention time (hereinafter referred to as "RT") are described. The conditions of liquid chromatography (hereinafter referred to as "LC") and mass spectrometry (hereinafter referred to as "MS") are as follows.
[LC conditions]
Column: L-column2 ODS, inner diameter: 4.6 mm, length: 30 mm, particle size: 3 µm (Chemicals Evaluation and Research Institute, Japan)
UV measurement wavelength: 254 nm
Mobile phase: Solution A: 0.1% formic acid in water, Solution B: 0.1% formic acid in acetonitrile
Flow rate: 2.0 mL/min
Pump: LC-20AD (manufactured by Shimadzu Corporation) two machines (high pressure gradient)
Gradient conditions: sending a solution with the concentration gradient described in Table LC1.

**Table LC1**

| Time (min) | Solution A (%) | Solution B (%) |
|---|---|---|
| 0.01 | 90 | 10 |
| 2.00 | 0 | 100 |
| 4.00 | 0 | 100 |
| 4.01 | 90 | 10 |

### [MS conditions]

Detector: LCMS-2020 (manufactured by Shimadzu Corporation) Ionization Method: DUIS (ESI, APCI)

First, Preparation Examples of the Present compounds (including the Compounds of the present invention) are shown below.

### Preparation Example 1

A mixture of the Intermediate 1 (0.40 g), phenylboronic acid (0.25 g), {1,1'-bis(diphenylphosphino)ferrocene}dichloropalladium(II) (0.10 g), tripotassium phosphate (0.85 g), DME (4 mL), and water (0.02 mL) was stirred under reflux for 5 hours. The resulting mixture was allowed to cool to room temperature, then water was added thereto, and the resulting mixture was subjected to extraction with ethyl acetate. The resulting organic layer was concentrated under reduced pressure, and the resulting residue was subjected to silica gel column chromatography (hexane : ethyl acetate = 1 : 1) to give the Present compound 1 represented by the following formula (0.30 g). 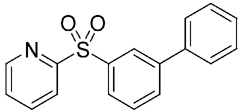 Present compound 1: ¹H-NMR (CDCl₃) δ: 8.70 (1H, dq), 8.28 (1H, t), 8.24 (1H, dt), 8.05 (1H, dq), 7.94 (1H, td), 7.83 (1H, dq), 7.63-7.59 (3H, m), 7.49-7.45 (3H, m), 7.42-7.38 (1H, m).

### Preparation Example 1-1

The compounds prepared according to the Preparation Example 1 and physical properties thereof are shown below.

A compound represented by formula (IA-1) 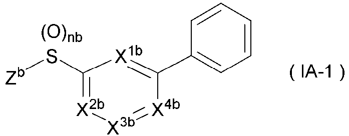 (hereinafter referred to as "Compound (IA-1)") wherein the combination of Z^{b}, X^{1b}, X^{2b}, X^{3b}, X^{4b}, and nb represents any one combination indicated in Table A-1.

**Table A-1**

| Present compound | Z^{b} | X^{1b} | X^{2b} | X^{3b} | X^{4b} | nb |
|---|---|---|---|---|---|---|
| 2 | 3-Py | CH | CH | CH | CH | 2 |
| 3 | 4-Py | CH | CH | CH | CH | 2 |
| 4 | 2-Pyrazin | CH | CH | CH | CH | 2 |
| 5 | Et | CH | CH | N | CH | 2 |
| 6 | 2-Py | CH | CH | N | CH | 0 |
| 7 | 2-Pyrazin | CH | CH | N | CH | 0 |
| 32 | Ph | CH | CH | N | CH | 2 |

Present compound 2: ¹H-NMR (CDCl₃) δ: 9.18 (1H, dd), 8.80 (1H, dd), 8.25 (1H, ddd), 8.18 (1H, t), 7.96-7.93 (1H, m), 7.84-7.81 (1H, m), 7.64-7.56 (3H, m), 7.51-7.40 (4H, m). Present compound 3: LC/MS [M+H]+ = 296, RT = 1.84 min Present compound 4: ¹H-NMR (CDCl₃) δ: 9.42 (1H, d), 8.78 (1H, d), 8.66 (1H, dd), 8.29 (1H, t), 8.06 (1H, dt), 7.88 (1H, dt), 7.67-7.59 (3H, m), 7.50-7.40 (3H, m).

Present compound 5: LC/MS: 248 [M+H]⁺, RT = 1.60 min. Present compound 6: ¹H-NMR (CDCl₃) δ: 8.85 (1H, d), 8.73 (1H, d), 8.46-8.42 (1H, m), 8.10 (1H, t), 7.63-7.58 (2H, m), 7.56-7.46 (3H, m), 7.45-7.40 (1H, m), 7.10-7.04 (2H, m). Present compound 7: ¹H-NMR (CDCl₃) δ: 8.89 (1H, d), 8.75 (1H, d), 8.43 (1H, d), 8.36-8.35 (1H, m), 8.32 (1H, d), 8.12 (1H, t), 7.62-7.59 (2H, m), 7.52-7.48 (2H, m), 7.46-7.42 (1H, m).

Present compound 32: ¹H-NMR (CDCl₃) δ: 9.09 (1H, d), 9.00 (1H, d), 8.38 (1H, t), 8.05-7.97 (2H, m), 7.68-7.44 (8H, m).

### Preparation Example 2

To a mixture of the Present compound 6 (0.4 g) and chloroform (5 mL) was added mCPBA (purity: 75%, wetted with 25% water) (0.56 g) at 0°C, and the resulting mixture was stirred at room temperature for 3 hours. To the resulting mixture were added a saturated aqueous solution of sodium thiosulfate (5 mL) and a saturated aqueous solution of sodium hydrogen carbonate (5 mL), and the resulting mixture was stirred for 1 hour and then subjected to extraction with chloroform. The resulting organic layer was washed with saturated brine, and concentrated under reduced pressure. The resulting residue was subjected to silica gel column chromatography (hexane : ethyl acetate = 1 : 1) to give the Present compound 8 represented by the following formula (0.11 g) and the Present compound 9 (0.19 g). 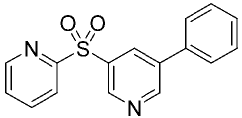 Present compound 8: ¹H-NMR (CDCl₃) δ: 9.20 (1H, d), 9.05 (1H, d), 8.70 (1H, d), 8.56 (1H, t), 8.26 (1H, d), 7.98 (1H, td), 7.63-7.61 (2H, m), 7.54-7.45 (4H, m). 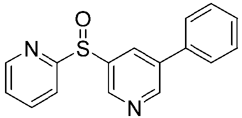 Present compound 9: ¹H-NMR (CDCl₃) δ: 8.99 (1H, d), 8.90 (1H, d), 8.61-8.59 (1H, m), 8.32-8.31 (1H, m), 8.08 (1H, d), 7.92 (1H, td), 7.60-7.58 (2H, m), 7.51-7.41 (3H, m), 7.37-7.34 (1H, m).

### Preparation Example 2-1

The compounds prepared according to the Preparation Example 2 and physical properties thereof are shown below.

The Compound (IA-1) wherein the combination of Z^{b}, X^{1b}, X^{2b}, X^{3b}, X^{4b}, and nb represents any one combination indicated in Table A-2.

**Table A-2**

| Present compound | Z^{b} | X^{1b} | X^{2b} | X^{3b} | X^{4b} | nb |
|---|---|---|---|---|---|---|
| 10 | 2-Pyrazin | CH | CH | N | CH | 2 |
| 11 | 2-Pyrazin | CH | CH | N | CH | 1 |
| 12 | Pr | CH | CH | N | CH | 2 |
| 13 | 2-Thie | CH | CH | N | CH | 2 |
| 14 | 2-Thie | CH | CH | N | CH | 1 |
| 15 | Bu | CH | CH | N | CH | 2 |
| 16 | c-Pen | CH | CH | N | CH | 2 |
| 20 | Pr | N | CH | N | CH | 2 |
| 21 | 2-Thie | N | CH | N | CH | 2 |
| 24 | 2-Me-3-Tet-fura | CH | CH | N | CH | 1 |

Present compound 10: ¹H-NMR (CDCl₃) δ: 9.45 (1H, d), 9.22 (1H, d), 9.09 (1H, d), 8.83 (1H, d), 8.67 (1H, t), 8.54 (1H, t), 7.66-7.60 (2H, m), 7.56-7.45 (3H, m).

Present compound 11: ¹H-NMR (CDCl₃) δ:9.29 (1H, s), 9.00-8.92 (2H, m), 8.69 (1H, d), 8.57 (1H, t), 8.33 (1H, t), 7.64-7.59 (2H, m), 7.53-7.43 (3H, m).

Present compound 12: ¹H-NMR (CDCl₃) δ: 9.10 (1H, d), 9.08 (1H, d), 8.36 (1H, t), 7.65-7.62 (2H, m), 7.56-7.47 (3H, m), 3.18-3.14 (2H, m), 1.87-1.78 (2H, m), 1.05 (3H, t).

Present compound 13: ¹H-NMR (CDCl₃) δ: 9.14 (1H, d), 9.01 (1H, d), 8.41 (1H, t), 7.79 (1H, dd), 7.72 (1H, dd), 7.62-7.59 (2H, m), 7.55-7.46 (3H, m), 7.14 (1H, dd).

Present compound 14: ¹H-NMR (CDCl₃) δ: 8.95 (1H, d), 8.72 (1H, d), 8.31 (1H, t), 7.68-7.67 (2H, m), 7.65-7.62 (2H, m), 7.54-7.44 (3H, m), 7.13 (1H, dd).

Present compound 15: ¹H-NMR (CDCl₃) δ: 9.10 (1H, d), 9.07 (1H, d), 8.36 (1H, t), 7.65-7.62 (2H, m), 7.56-7.46 (3H, m), 3.20-3.16 (2H, m), 1.80-1.73 (2H, m), 1.49-1.40 (2H, m), 0.92 (3H, t).

Present compound 16: ¹H-NMR (CDCl₃) δ: 9.08 (1H, d), 9.07 (1H, d), 8.36 (1H, t), 7.64-7.61 (2H, m), 7.56-7.46 (3H, m), 3.60-3.52 (1H, m), 2.17-2.08 (2H, m), 1.99-1.90 (2H, m), 1.87-1.78 (2H, m), 1.70-1.60 (2H, m).

Present compound 20: ¹H-NMR (CDCl₃) δ: 9.28 (1H, s), 9.20 (1H, s), 8.10-8.08 (2H, m), 7.58-7.56 (3H, m), 3.47-3.43 (2H, m), 1.93-1.83 (2H, m), 1.08 (3H, t).

Present compound 21: ¹H-NMR (CDCl₃) δ: 9.26 (1H, s), 9.20 (1H, s), 8.06-8.04 (2H, m), 7.92 (1H, dd), 7.80 (1H, dd), 7.55-7.51 (3H, m), 7.18 (1H, dd).

Present compound 24: LC/MS: 288 [M+H]⁺, RT = 1.37 min.

### Preparation Example 2-2

The compounds prepared according to the Preparation Example 2 and physical properties thereof are shown below. 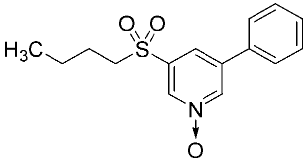 Present compound 17: ¹H-NMR (CDCl₃) δ: 8.62-8.59 (2H, m), 7.86 (1H, t), 7.60-7.52 (5H, m), 3.20-3.16 (2H, m), 1.81-1.73 (2H, m), 1.51-1.41 (2H, m), 0.94 (3H, t). 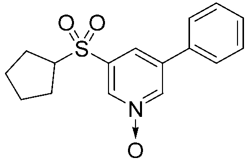 Present compound 18: ¹H-NMR (CDCl₃) δ: 8.61 (1H, t), 8.58 (1H, t), 7.86 (1H, t), 7.59-7.52 (5H, m), 3.60-3.51 (1H, m), 2.14-2.07 (2H, m), 2.02-1.94 (2H, m), 1.87-1.83 (2H, m), 1.73-1.65 (2H, m). 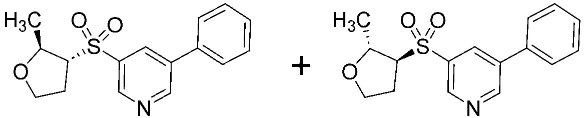 Present compound 22: ¹H-NMR (CDCl₃) δ: 9.12 (1H, d), 9.08 (1H, d), 8.36 (1H, t), 7.65-7.62 (2H, m), 7.57-7.48 (3H, m), 4.45-4.38 (1H, m), 4.00-3.95 (1H, m), 3.83-3.77 (1H, m), 3.38-3.33 (1H, m), 2.45-2.38 (1H, m), 2.28-2.17 (1H, m), 1.32 (3H, d). 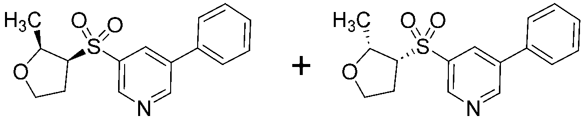 Present compound 23: ¹H-NMR (CDCl₃) δ: 9.10 (1H, d), 9.08 (1H, d), 8.36 (1H, t), 7.64-7.62 (2H, m), 7.57-7.49 (3H, m), 4.40-4.33 (1H, m), 4.15-4.10 (1H, m), 3.80-3.66 (2H, m), 2.51-2.42 (1H, m), 2.16-2.08 (1H, m), 1.66 (3H, d).

The Present compound 22 and the Present compound 23 are racemic compounds.

### Preparation Example 3

A mixture of 3-fluoro-5-phenylpyridine (1.00 g), 2-methyltetrahydrofuran-3-thiol (0.79 mL), cesium carbonate (0.82 g), and DMF (10 mL) was stirred at room temperature for 7 hours. To the resulting mixture was added water, and the resulting mixture was subjected to extraction with a mixed solution of ethyl acetate and hexane. The resulting organic layer was washed with saturated brine, and concentrated under reduced pressure. The resulting residue was subjected to silica gel column chromatography (hexane : ethyl acetate = 1 : 1) to give the Present compound 25 represented by the following formula (0.32 g) and the Present compound 26 (0.91 g). 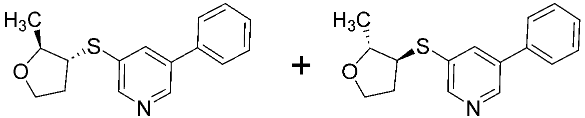 Present compound 25: ¹H-NMR (CDCl₃) δ: 8.71 (1H, d), 8.61 (1H, d), 7.91 (1H, t), 7.59-7.56 (2H, m), 7.52-7.48 (2H, m), 7.46-7.41 (1H, m), 4.01-3.96 (1H, m), 3.90-3.80 (2H, m), 3.32-3.26 (1H, m), 2.50-2.41 (1H, m), 2.03-1.95 (1H, m), 1.30 (3H, d). 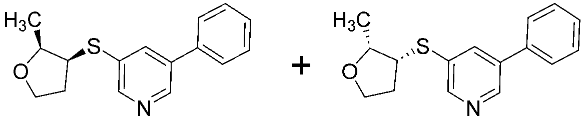 Present compound 26: ¹H-NMR (CDCl₃) δ: 8.68 (1H, d), 8.58 (1H, d), 7.88 (1H, t), 7.59-7.56 (2H, m), 7.52-7.47 (2H, m), 7.45-7.41 (1H, m), 4.29-4.22 (1H, m), 4.10-4.04 (1H, m), 3.90-3.85 (1H, m), 3.84-3.78 (1H, m), 2.48-2.39 (1H, m), 2.11-2.03 (1H, m), 1.35 (3H, d).

The Present compound 25 and the Present compound 26 are racemic compounds.

### Preparation Example 3-1

The compounds prepared according to the Preparation Example 3 and physical properties thereof are shown below.

The Compound (IA-1) wherein the combination of Z^{b}, X^{1b}, X^{2 b}, X^{3 b}, X^{4b}, and nb represents any one combination indicated in Table A-3.

**Table A-3**

| Present compound | Z^{b} | X^{1 b} | X^{2 b} | X^{3 b} | X^{4 b} | nb |
|---|---|---|---|---|---|---|
| 27 | Pr | CH | CH | N | CH | 0 |
| 28 | Bu | CH | CH | N | CH | 0 |
| 29 | c-Pen | CH | CH | N | CH | 0 |
| 30 | Pr | N | CH | N | CH | 0 |
| 31 | 2-Thie | N | CH | N | CH | 0 |

Present compound 27: ¹H-NMR (CDCl₃) δ: 8.63 (1H, d), 8.53 (1H, d), 7.81 (1H, t), 7.59-7.56 (2H, m), 7.51-7.47 (2H, m), 7.44-7.40 (1H, m), 2.97 (2H, t), 1.76-1.67 (2H, m), 1.05 (3H, t).

Present compound 28: ¹H-NMR (CDCl₃) δ: 8.63 (1H, d), 8.53 (1H, d), 7.81 (1H, t), 7.58-7.55 (2H, m), 7.50-7.46 (2H, m), 7.44-7.39 (1H, m), 2.99 (2H, t), 1.71-1.63 (2H, m), 1.52-1.43 (2H, m), 0.94 (3H, t).

Present compound 29: ¹H-NMR (CDCl₃) δ: 8.64 (1H, d), 8.56 (1H, d), 7.85 (1H, t), 7.58-7.56 (2H, m), 7.50-7.46 (2H, m), 7.41 (1H, tt), 3.69-3.62 (1H, m), 2.13-2.04 (2H, m), 1.86-1.75 (2H, m), 1.70-1.61 (4H, m).

Present compound 30: ¹H-NMR (CDCl₃) δ: 8.65 (1H, s), 8.36 (1H, s), 8.04-8.01 (2H, m), 7.53-7.44 (3H, m), 3.26 (2H, t), 1.86-1.77 (2H, m), 1.09 (3H, t).

Present compound 31: ¹H-NMR (CDCl₃) δ: 8.72 (1H, s), 8.11 (1H, s), 7.98-7.96 (2H, m), 7.63 (1H, dd), 7.50-7.45 (3H, m), 7.42 (1H, dd), 7.18 (1H, dd).

### Reference Preparation Example 1

To a mixture of the Intermediate 7 (3.50 g) and chloroform (35 mL) was added mCPBA (purity: 70%, wetted with 30% water) (7.13 g) at 0°C, and the resulting mixture was stirred at room temperature for 1 hour. To the resulting mixture were added a saturated aqueous solution of sodium thiosulfate (10 mL) and a saturated aqueous solution of sodium hydrogen carbonate (10 mL), and the resulting mixture was stirred for 1 hour and then subjected to extraction with chloroform. The resulting organic layer was washed with saturated brine, dried over sodium sulfate, and concentrated under reduced pressure to give the Intermediate 1 represented by the following formula (3.94 g). 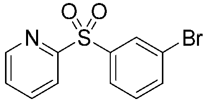 Intermediate 1: ¹H-NMR (CDCl₃) δ: 8.70 (1H, dq), 8.22-8.20 (2H, m), 8.02 (1H, dq), 7.96 (1H, td), 7.74 (1H, dq), 7.50 (1H, ddd), 7.42 (1H, t).

### Reference Preparation Example 1-1

The compounds prepared according to the Reference Preparation Example 1 and physical properties thereof are shown below.

A compound represented by formula (IB-1) 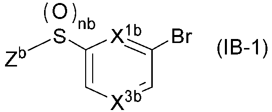 (hereinafter referred to as "Compound (IB-1)") wherein the combination of Z^{b}, X^{1b}, X^{3b}, and nb represents any one combination indicated in Table A-6.

**Table A-6**

| Intermediate | Z^{b} | X^{1b} | X^{3b} | nb |
|---|---|---|---|---|
| 2 | 3-Py | CH | CH | 2 |
| 3 | 4-Py | CH | CH | 2 |
| 4 | 2-Pyrimid | CH | CH | 2 |
| 5 | 4-Pyrimid | CH | CH | 2 |
| 6 | 2-Pyrazin | CH | CH | 2 |

Intermediate 2: ¹H-NMR (CDCl₃) δ: 9.15 (1H, dd), 8.83 (1H, dd), 8.22 (1H, ddd), 8.11 (1H, t), 7.91 (1H, dq), 7.74 (1H, dq), 7.49 (1H, ddd), 7.43 (1H, t).

Intermediate 3: ¹H-NMR (CDCl₃) δ: 8.87 (2H, dd), 8.10 (1H, t), 7.90 (1H, dq), 7.78-7.75 (3H, m), 7.44 (1H, t). Intermediate 4: ¹H-NMR (CDCl₃) δ: 8.93 (2H, d), 8.28 (1H, t), 8.07 (1H, dq), 7.80 (1H, dq), 7.52 (1H, t), 7.46 (1H, t).

Intermediate 5: ¹H-NMR (CDCl₃) δ: 9.33 (1H, d), 9.10 (1H, d), 8.21 (1H, t), 8.13 (1H, dd), 8.03 (1H, dq), 7.82 (1H, dq), 7.47 (1H, t).

Intermediate 6: ¹H-NMR (CDCl₃) δ: 9.40 (1H, d), 8.81 (1H, d), 8.67 (1H, dd), 8.22 (1H, t), 8.03 (1H, dq), 7.79 (1H, dq), 7.46 (1H, t).

### Reference Preparation Example 2

A mixture of 2-iodopyridine (5.69 g), 3-bromothiophenol (5.00 g), copper(I) oxide (0.11 g), cesium carbonate (10.77 g), and DMSO (25 mL) was stirred at 110°C for 3 hours. The resulting mixture was allowed to cool to room temperature, then water was added thereto, and the resulting mixture was subjected to extraction with ethyl acetate. The resulting organic layer was washed with saturated brine, dried over sodium sulfate, and concentrated under reduced pressure to give the Intermediate 7 represented by the following formula (7.46 g).

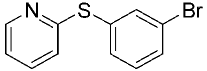

Intermediate 7: ¹H-NMR (CDCl₃) δ: 8.45 (1H, dq), 7.73 (1H, t), 7.54-7.49 (3H, m), 7.29 (1H, t), 7.07-7.03 (1H, m), 7.00 (1H, dt).

### Reference Preparation Example 2-1

The compounds prepared according to the Reference Preparation Example 2 and physical properties thereof are shown below.

The Compound (IB-1) wherein the combination of Z^{b}, X^{1 b}, X^{3b}, and nb represents any one combination indicated in Table A-7.

**Table A-7**

| Intermediate | Z^{b} | X^{1b} | X^{3b} | nb |
|---|---|---|---|---|
| 8 | 3-Py | CH | CH | 0 |
| 9 | 4-Py | CH | CH | 0 |

Intermediate 8: ¹H-NMR (CDCl₃) δ: 8.61 (1H, s), 8.54 (1H, d), 7.67 (1H, ddd), 7.45 (1H, t), 7.40 (1H, dq), 7.29-7.23 (2H, m), 7.18 (1H, t).

Intermediate 9: ¹H-NMR (CDCl₃) δ: 8.39 (2H, dd), 7.70 (1H, t), 7.59 (1H, dq), 7.48 (1H, dq), 7.33 (1H, t), 6.98 (2H, dd).

### Reference Preparation Example 3

To a mixture of 3-bromothiophenol (3.00 g), sodium hydride (in oil, 60%) (0.76 g), and DMF (30 mL) was added 2-chloropyrimidine (1.82 g), and the resulting mixture was stirred at room temperature for 2 hours. To the resulting mixture was added water, and the resulting mixture was subjected to extraction with ethyl acetate. The resulting organic layer was dried over sodium sulfate, and concentrated under reduced pressure. The resulting residue was subjected to silica gel column chromatography (hexane : ethyl acetate = 7 : 3) to give the Intermediate 10 represented by the following formula (3.00 g).

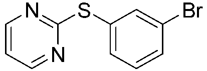

Intermediate 10: ¹H-NMR (CDCl₃) δ: 8.51 (2H, d), 7.80 (1H, t), 7.58-7.55 (2H, m), 7.31 (1H, t), 7.00 (1H, t).

### Reference Preparation Example 3-1

The compounds prepared according to the Reference Preparation Example 3 and physical properties thereof are shown below.

The Compound (IB-1) wherein the combination of Z^{b}, X^{1b}, X^{3b}, and nb represents any one combination indicated in Table A-8.

**Table A-8**

| Intermediate | Z^{b} | X^{1b} | X^{3b} | nb |
|---|---|---|---|---|
| 11 | 4-Pyrimid | CH | CH | 0 |
| 12 | 2-Pyrazin | CH | CH | 0 |

Intermediate 11: ¹H-NMR (CDCl₃) δ: 8.92 (1H, d), 8.39 (1H, d), 7.78 (1H, t), 7.64 (1H, dq), 7.55 (1H, dq), 7.37 (1H, t), 6.86 (1H, dd).

Intermediate 12: ¹H-NMR (CDCl₃) δ: 8.37 (1H, dd), 8.30 (2H, dd), 7.76 (1H, t), 7.59-7.52 (2H, m), 7.32 (1H, t).

### Reference Preparation Example 4

A mixture of 3-fluoro-5-bromopyridine (1.0 g), 2-mercaptopyridine (0.66 g), cesium carbonate (1.6 g), and DMF (7 mL) was stirred at 70°C for 4 hours, and then stirred at 100°C for 30 minutes. The resulting mixture was allowed to cool to room temperature, then water was added thereto, and the resulting mixture was subjected to extraction with ethyl acetate. The resulting organic layer was dried over sodium sulfate, and concentrated under reduced pressure. The resulting residue was subjected to silica gel column chromatography (hexane : ethyl acetate = 7 : 3) to give the Intermediate 13 represented by the following formula (0.70 g). 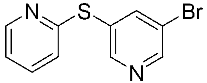 Intermediate 13: LC/MS: 267 [M+H]⁺, RT = 1.69 min.

### Reference Preparation Example 4-1

The compound prepared according to the Reference Preparation Example 4 and a physical property thereof are shown below.

The Compound (IB-1) wherein the combination of Z^{b}, X^{1b}, X^{3b}, and nb represents the combination indicated in Table A-9.

**Table A-9**

| Intermediate | Z^{b} | X^{1b} | X^{3b} | nb |
|---|---|---|---|---|
| 14 | 2-Pyrazin | CH | N | 0 |

Intermediate 14: LC/MS: 268 [M+H]⁺, RT = 1.53 min.

Examples of the Present compounds and the Compounds of the present invention prepared according to the above Production methods and Preparation Examples are shown below.

A compound represented by formula (A-1) 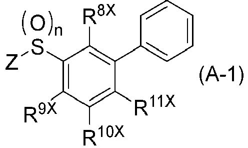 (hereinafter referred to as "Compound (A-1)") wherein n represents 0, and the combination of Z, R^{8X}, R^{9X}, R^{10X}, and R^{11x} represents any one combination indicated in the Combination A (hereinafter referred to as "Compound group SX1").

Combination A consists of Substituent Numbers A1 to A527. Substituent Numbers A1 to A527 indicate the combinations of Z, R^{8X}, R^{9X}, R^{10X}, and R^{11X} in the Compound (A-1), and are hereinafter referred to as [Substituent Number; Z,R^{8X},R^{9X},R^{10X},R^{11X}].

For example, Substituent Number A5 indicates a combination wherein Z represents an ethyl group, R^{8X}, R^{9X}, and R^{10X} each represent a hydrogen atom, and R^{11x} represents a fluorine atom.

Combination A: [A1;Et,H,H,H,H], [A2;Et,F,H,H,H], [A3;Et,H,F,H,H], [A4;Et,H,H,F,H], [A5;Et,H,H,H,F], [A6;Et,Cl,H,H,H], [A7;Et,H,Cl,H,H], [A8;Et,H,H,Cl,H], [A9;Et,H,H,H,Cl], [A10;Et,Me,H,H,H], [A11;Et,H,Me,H,H], [A12;Et,H,H,Me,H], [A13;Et,H,H,H,Me], [A14;Et,F,F,H,H], [A15;Et,F,H,F,H], [A16;Et,F,H,H,F], [A17;Et,H,F,F,H], [A18;Et,H,F,H,F], [A19;Et,H,H,F,F], [A20;Et,Cl,Cl,H,H], [A21;Et,Cl,H,Cl,H], [A22;Et,Cl,H,H,Cl], [A23;Et,H,Cl,Cl,H], [A24;Et,H,Cl,H,Cl], [A25;Et,H,H,Cl,Cl], [A26;Et,Me,Me,H,H], [A27;Et,Me,H,Me,H], [A28;Et,Me,H,H,Me], [A29;Et,H,Me,Me,H], [A30;Et,H,Me,H,Me], [A31;Et,H,H,Me,Me], [A32;Pr,H,H,H,H], [A33;Pr,F,H,H,H], [A34;Pr,H,F,H,H], [A35;Pr,H,H,F,H], [A36;Pr,H,H,H,F], [A37;Pr,Cl,H,H,H], [A38;Pr,H,Cl,H,H], [A39;Pr,H,H,Cl,H], [A40;Pr,H,H,H,Cl], [A41;Pr,Me,H,H,H], [A42;Pr,H,Me,H,H], [A43;Pr,H,H,Me,H], [A44;Pr,H,H,H,Me], [A45;Pr,F,F,H,H], [A46;Pr,F,H,F,H], [A47;Pr,F,H,H,F], [A48;Pr,H,F,F,H], [A49;Pr,H,F,H,F], [A50;Pr,H,H,F,F], [A51;Pr,Cl,Cl,H,H], [A52;Pr,Cl,H,Cl,H], [A53;Pr,Cl,H,H,Cl], [A54;Pr,H,Cl,Cl,H], [A55;Pr,H,Cl,H,Cl], [A56;Pr,H,H,Cl,Cl], [A57;Pr,Me,Me,H,H], [A58;Pr,Me,H,Me,H], [A59;Pr,Me,H,H,Me], [A60;Pr,H,Me,Me,H], [A61;Pr,H,Me,H,Me], [A62;Pr,H,H,Me,Me], [A63;i-Pr,H,H,H,H], [A64;i-Pr,F,H,H,H], [A65;i-Pr,H,F,H,H], [A66;i-Pr,H,H,F,H], [A67;i-Pr,H,H,H,F], [A68;i-Pr,Cl,H,H,H], [A69;i-Pr,H,Cl,H,H], [A70;i-Pr,H,H,Cl,H], [A71;i-Pr,H,H,H,Cl], [A72;i-Pr,Me,H,H,H], [A73;i-Pr,H,Me,H,H], [A74;i-Pr,H,H,Me,H], [A75;i-Pr,H,H,H,Me], [A76;i-Pr,F,F,H,H], [A77;i-Pr,F,H,F,H], [A78;i-Pr,F,H,H,F], [A79;i-Pr,H,F,F,H], [A80;i-Pr,H,F,H,F], [A81;i-Pr,H,H,F,F], [A82;i-Pr,Cl,Cl,H,H], [A83;i-Pr,Cl,H,Cl,H], [A84;i-Pr,Cl,H,H,Cl], [A85;i-Pr,H,Cl,Cl,H], [A86;i-Pr,H,Cl,H,Cl], [A87;i-Pr,H,H,Cl,Cl], [A88;i-Pr,Me,Me,H,H], [A89;i-Pr,Me,H,Me,H], [A90;i-Pr,Me,H,H,Me], [A91;i-Pr,H,Me,Me,H], [A92;i-Pr,H,Me,H,Me], [A93;i-Pr,H,H,Me,Me], [A94;CH=CHCH₃,H,H,H,H], [A95;CH=CHCH₃,F,H,H,H], [A96;CH=CHCH₃,H,F,H,H], [A97;CH=CHCH₃,H,H,F,H], [A98;CH=CHCH₃,H,H,H,F], [A99;CH=CHCH₃,Cl,H,H,H], [A100;CH=CHCH₃,H,Cl,H,H], [A101;CH=CHCH₃,H,H,Cl,H], [A102;CH=CHCH₃,H,H,H,Cl], [A103;CH=CHCH₃,Me,H,H,H], [A104;CH=CHCH₃,H,Me,H,H], [A105;CH=CHCH₃,H,H,Me,H], [A106;CH=CHCH₃,H,H,H,Me], [A107;CH=CHCH₃,F,F,H,H], [A108;CH=CHCH₃,F,H,F,H], [A109;CH=CHCH₃,F,H,H,F], [A110;CH=CHCH₃,H,F,F,H], [A111;CH=CHCH₃,H,F,H,F], [A112;CH=CHCH₃,H,H,F,F], [A113;CH=CHCH₃,Cl,Cl,H,H], [A114;CH=CHCH₃,Cl,H,Cl,H], [A115;CH=CHCH₃,Cl,H,H,Cl], [A116;CH=CHCH₃,H,Cl,Cl,H], [A117;CH=CHCH₃,H,Cl,H,Cl], [A118;CH=CHCH₃,H,H,Cl,Cl], [A119;CH=CHCH₃,Me,Me,H,H], [A120;CH=CHCH₃,Me,H,Me,H], [A121;CH=CHCH₃,Me,H,H,Me], [A122;CH=CHCH₃,H,Me,Me,H], [A123;CH=CHCH₃,H,Me,H,Me], [A124;CH=CHCH₃,H,H,Me,Me], [A125;CH₂CH=CH₂,H,H,H,H], [A126;CH₂CH=CH₂,F,H,H,H], [A127;CH₂CH=CH₂,H,F,H,H], [A128;CH₂CH=CH₂,H,H,F,H], [A129;CH₂CH=CH₂,H,H,H,F], [A130;CH₂CH=CH₂,Cl,H,H,H], [A131;CH₂CH=CH₂,H,Cl,H,H], [A132;CH₂CH=CH₂,H,H,Cl,H], [A133;CH₂CH=CH₂,H,H,H,Cl], [A134;CH₂CH=CH₂,Me,H,H,H], [A135;CH₂CH=CH₂,H,Me,H,H], [A136;CH₂CH=CH₂,H,H,Me,H], [A137;CH₂CH=CH₂,H,H,H,Me], [A138;CH₂CH=CH₂,F,F,H,H], [A139;CH₂CH=CH₂,F,H,F,H], [A140;CH₂CH=CH₂,F,H,H,F], [A141;CH₂CH=CH₂,H,F,F,H], [A142;CH₂CH=CH₂,H,F,H,F], [A143;CH₂CH=CH₂,H,H,F,F], [A144;CH₂CH=CH₂,Cl,Cl,H,H], [A145;CH₂CH=CH₂,Cl,H,Cl,H], [A146;CH₂CH=CH₂,Cl,H,H,Cl], [A147;CH₂CH=CH₂,H,Cl,Cl,H], [A148;CH₂CH=CH₂,H,Cl,H,Cl], [A149;CH₂CH=CH₂,H,H,Cl,Cl], [A150;CH₂CH=CH₂,Me,Me,H,H], [A151;CH₂CH=CH₂,Me,H,Me,H], [A152;CH₂CH=CH₂,Me,H,H,Me], [A153;CH₂CH=CH₂,H,Me,Me,H], [A154;CH₂CH=CH₂,H,Me,H,Me], [A155;CH₂CH=CH₂,H,H,Me,Me], [A156;Bu,H,H,H,H], [A157;Bu,F,H,H,H], [A158;Bu,H,F,H,H], [A159;Bu,H,H,F,H], [A160;Bu,H,H,H,F], [A161;Bu,Cl,H,H,H], [A162;Bu,H,Cl,H,H], [A163;Bu,H,H,Cl,H], [A164;Bu,H,H,H,Cl], [A165;Bu,Me,H,H,H], [A166;Bu,H,Me,H,H], [A167;Bu,H,H,Me,H], [A168;Bu,H,H,H,Me], [A169;Bu,F,F,H,H], [A170;Bu,F,H,F,H], [A171;Bu,F,H,H,F], [A172;Bu,H,F,F,H], [A173;Bu,H,F,H,F], [A174;Bu,H,H,F,F], [A175;Bu,Cl,Cl,H,H], [A176;Bu,Cl,H,Cl,H], [A177;Bu,Cl,H,H,Cl], [A178;Bu,H,Cl,Cl,H], [A179;Bu,H,Cl,H,Cl], [A180;Bu,H,H,Cl,Cl], [A181;Bu,Me,Me,H,H], [A182;Bu,Me,H,Me,H], [A183;Bu,Me,H,H,Me], [A184;Bu,H,Me,Me,H], [A185;Bu,H,Me,H,Me], [A186;Bu,H,H,Me,Me], [A187;c-Pr,H,H,H,H], [A188;c-Pr,F,H,H,H], [A189;c-Pr,H,F,H,H], [A190;c-Pr,H,H,F,H], [A191;c-Pr,H,H,H,F], [A192;c-Pr,Cl,H,H,H], [A193;c-Pr,H,Cl,H,H], [A194;c-Pr,H,H,Cl,H], [A195;c-Pr,H,H,H,Cl], [A196;c-Pr,Me,H,H,H], [A197;c-Pr,H,Me,H,H], [A198;c-Pr,H,H,Me,H], [A199;c-Pr,H,H,H,Me], [A200;c-Pr,F,F,H,H], [A201;c-Pr,F,H,F,H], [A202;c-Pr,F,H,H,F], [A203;c-Pr,H,F,F,H], [A204;c-Pr,H,F,H,F], [A205;c-Pr,H,H,F,F], [A206;c-Pr,Cl,Cl,H,H], [A207;c-Pr,Cl,H,Cl,H], [A208;c-Pr,Cl,H,H,Cl], [A209;c-Pr,H,Cl,Cl,H], [A210;c-Pr,H,Cl,H,Cl], [A211;c-Pr,H,H,Cl,Cl], [A212;c-Pr,Me,Me,H,H], [A213;c-Pr,Me,H,Me,H], [A214;c-Pr,Me,H,H,Me], [A215;c-Pr,H,Me,Me,H], [A216;c-Pr,H,Me,H,Me], [A217;c-Pr,H,H,Me,Me], [A218;2-Thie,H,H,H,H], [A219;2-Thie,F,H,H,H], [A220;2-Thie,H,F,H,H], [A221;2-Thie,H,H,F,H], [A222;2-Thie,H,H,H,F], [A223;2-Thie,Cl,H,H,H], [A224;2-Thie,H,Cl,H,H], [A225;2-Thie,H,H,Cl,H], [A226;2-Thie,H,H,H,Cl], [A227;2-Thie,Me,H,H,H], [A228;2-Thie,H,Me,H,H], [A229;2-Thie,H,H,Me,H], [A230;2-Thie,H,H,H,Me], [A231;2-Thie,F,F,H,H], [A232;2-Thie,F,H,F,H], [A233;2-Thie,F,H,H,F], [A234;2-Thie,H,F,F,H], [A235;2-Thie,H,F,H,F], [A236;2-Thie,H,H,F,F], [A237;2-Thie,Cl,Cl,H,H], [A238;2-Thie,Cl,H,Cl,H], [A239;2-Thie,Cl,H,H,Cl], [A240;2-Thie,H,Cl,Cl,H], [A241;2-Thie,H,Cl,H,Cl], [A242;2-Thie,H,H,Cl,Cl], [A243;2-Thie,Me,Me,H,H], [A244;2-Thie,Me,H,Me,H], [A245;2-Thie,Me,H,H,Me], [A246;2-Thie,H,Me,Me,H], [A247;2-Thie,H,Me,H,Me], [A248;2-Thie,H,H,Me,Me], [A249;3-Thie,H,H,H,H], [A250;3-Thie,F,H,H,H], [A251;3-Thie,H,F,H,H], [A252;3-Thie,H,H,F,H], [A253;3-Thie,H,H,H,F], [A254;3-Thie,Cl,H,H,H], [A255;3-Thie,H,Cl,H,H], [A256;3-Thie,H,H,Cl,H], [A257;3-Thie,H,H,H,Cl], [A258;3-Thie,Me,H,H,H], [A259;3-Thie,H,Me,H,H], [A260;3-Thie,H,H,Me,H], [A261;3-Thie,H,H,H,Me], [A262;3-Thie,F,F,H,H], [A263;3-Thie,F,H,F,H], [A264;3-Thie,F,H,H,F], [A265;3-Thie,H,F,F,H], [A266;3-Thie,H,F,H,F], [A267;3-Thie,H,H,F,F], [A268;3-Thie,Cl,Cl,H,H], [A269;3-Thie,Cl,H,Cl,H], [A270;3-Thie,Cl,H,H,Cl], [A271;3-Thie,H,Cl,Cl,H], [A272;3-Thie,H,Cl,H,Cl], [A273;3-Thie,H,H,Cl,Cl], [A274;3-Thie,Me,Me,H,H], [A275;3-Thie,Me,H,Me,H], [A276;3-Thie,Me,H,H,Me], [A277;3-Thie,H,Me,Me,H], [A278;3-Thie,H,Me,H,Me], [A279;3-Thie,H,H,Me,Me], [A280;2-Fura,H,H,H,H], [A281;2-Fura,F,H,H,H], [A282;2-Fura,H,F,H,H], [A283;2-Fura,H,H,F,H], [A284;2-Fura,H,H,H,F], [A285;2-Fura,Cl,H,H,H], [A286;2-Fura,H,Cl,H,H], [A287;2-Fura,H,H,Cl,H], [A288;2-Fura,H,H,H,Cl], [A289;2-Fura,Me,H,H,H], [A290;2-Fura,H,Me,H,H], [A291;2-Fura,H,H,Me,H], [A292;2-Fura,H,H,H,Me], [A293;2-Fura,F,F,H,H], [A294;2-Fura,F,H,F,H], [A295;2-Fura,F,H,H,F], [A296;2-Fura,H,F,F,H], [A297;2-Fura,H,F,H,F], [A298;2-Fura,H,H,F,F], [A299;2-Fura,Cl,Cl,H,H], [A300;2-Fura,Cl,H,Cl,H], [A301;2-Fura,Cl,H,H,Cl], [A302;2-Fura,H,Cl,Cl,H], [A303;2-Fura,H,Cl,H,Cl], [A304;2-Fura,H,H,Cl,Cl], [A305;2-Fura,Me,Me,H,H], [A306;2-Fura,Me,H,Me,H], [A307;2-Fura,Me,H,H,Me], [A308;2-Fura,H,Me,Me,H], [A309;2-Fura,H,Me,H,Me], [A310;2-Fura,H,H,Me,Me], [A311;3-Fura,H,H,H,H], [A312;3-Fura,F,H,H,H], [A313;3-Fura,H,F,H,H], [A314;3-Fura,H,H,F,H], [A315;3-Fura,H,H,H,F], [A316;3-Fura,Cl,H,H,H], [A317;3-Fura,H,Cl,H,H], [A318;3-Fura,H,H,Cl,H], [A319;3-Fura,H,H,H,Cl], [A320;3-Fura,Me,H,H,H], [A321;3-Fura,H,Me,H,H], [A322;3-Fura,H,H,Me,H], [A323;3-Fura,H,H,H,Me], [A324;3-Fura,F,F,H,H], [A325;3-Fura,F,H,F,H], [A326;3-Fura,F,H,H,F], [A327;3-Fura,H,F,F,H], [A328;3-Fura,H,F,H,F], [A329;3-Fura,H,H,F,F], [A330;3-Fura,Cl,Cl,H,H], [A331;3-Fura,Cl,H,Cl,H], [A332;3-Fura,Cl,H,H,Cl], [A333;3-Fura,H,Cl,Cl,H], [A334;3-Fura,H,Cl,H,Cl], [A335;3-Fura,H,H,Cl,Cl], [A336;3-Fura,Me,Me,H,H], [A337;3-Fura,Me,H,Me,H], [A338;3-Fura,Me,H,H,Me], [A339;3-Fura,H,Me,Me,H], [A340;3-Fura,H,Me,H,Me], [A341;3-Fura,H,H,Me,Me], [A342;2-Py,H,H,H,H], [A343;2-Py,F,H,H,H], [A344;2-Py,H,F,H,H], [A345;2-Py,H,H,F,H], [A346;2-Py,H,H,H,F], [A347;2-Py,Cl,H,H,H], [A348;2-Py,H,Cl,H,H], [A349;2-Py,H,H,Cl,H], [A350;2-Py,H,H,H,Cl], [A351;2-Py,Me,H,H,H], [A352;2-Py,H,Me,H,H], [A353;2-Py,H,H,Me,H], [A354;2-Py,H,H,H,Me], [A355;2-Py,F,F,H,H], [A356;2-Py,F,H,F,H], [A357;2-Py,F,H,H,F], [A358;2-Py,H,F,F,H], [A359;2-Py,H,F,H,F], [A360;2-Py,H,H,F,F], [A361;2-Py,Cl,Cl,H,H], [A362;2-Py,Cl,H,Cl,H], [A363;2-Py,Cl,H,H,Cl], [A364;2-Py,H,Cl,Cl,H], [A365;2-Py,H,Cl,H,Cl], [A366;2-Py,H,H,Cl,Cl], [A367;2-Py,Me,Me,H,H], [A368;2-Py,Me,H,Me,H], [A369;2-Py,Me,H,H,Me], [A370;2-Py,H,Me,Me,H], [A371;2-Py,H,Me,H,Me], [A372;2-Py,H,H,Me,Me], [A373;3-Py,H,H,H,H], [A374;3-Py,F,H,H,H], [A375;3-Py,H,F,H,H], [A376;3-Py,H,H,F,H], [A377;3-Py,H,H,H,F], [A378;3-Py,Cl,H,H,H], [A379;3-Py,H,Cl,H,H], [A380;3-Py,H,H,Cl,H], [A381;3-Py,H,H,H,Cl], [A382;3-Py,Me,H,H,H], [A383;3-Py,H,Me,H,H], [A384;3-Py,H,H,Me,H], [A385;3-Py,H,H,H,Me], [A386;3-Py,F,F,H,H], [A387;3-Py,F,H,F,H], [A388;3-Py,F,H,H,F], [A389;3-Py,H,F,F,H], [A390;3-Py,H,F,H,F], [A391;3-Py,H,H,F,F], [A392;3-Py,Cl,Cl,H,H], [A393;3-Py,Cl,H,Cl,H], [A394;3-Py,Cl,H,H,Cl], [A395;3-Py,H,Cl,Cl,H], [A396;3-Py,H,Cl,H,Cl], [A397;3-Py,H,H,Cl,Cl], [A398;3-Py,Me,Me,H,H], [A399;3-Py,Me,H,Me,H], [A400;3-Py,Me,H,H,Me], [A401;3-Py,H,Me,Me,H], [A402;3-Py,H,Me,H,Me], [A403;3-Py,H,H,Me,Me], [A404;4-Py,H,H,H,H], [A405;4-Py,F,H,H,H], [A406;4-Py,H,F,H,H], [A407;4-Py,H,H,F,H], [A408;4-Py,H,H,H,F], [A409;4-Py,Cl,H,H,H], [A410;4-Py,H,Cl,H,H], [A411;4-Py,H,H,Cl,H], [A412;4-Py,H,H,H,Cl], [A413;4-Py,Me,H,H,H], [A414;4-Py,H,Me,H,H], [A415;4-Py,H,H,Me,H], [A416;4-Py,H,H,H,Me], [A417;4-Py,F,F,H,H], [A418;4-Py,F,H,F,H], [A419;4-Py,F,H,H,F], [A420;4-Py,H,F,F,H], [A421;4-Py,H,F,H,F], [A422;4-Py,H,H,F,F], [A423;4-Py,Cl,Cl,H,H], [A424;4-Py,Cl,H,Cl,H], [A425;4-Py,Cl,H,H,Cl], [A426;4-Py,H,Cl,Cl,H], [A427;4-Py,H,Cl,H,Cl], [A428;4-Py,H,H,Cl,Cl], [A429;4-Py,Me,Me,H,H], [A430;4-Py,Me,H,Me,H], [A431;4-Py,Me,H,H,Me], [A432;4-Py,H,Me,Me,H], [A433;4-Py,H,Me,H,Me], [A434;4-Py,H,H,Me,Me], [A435;2-Pyrimid,H,H,H,H], [A436;2-Pyrimid,F,H,H,H], [A437;2-Pyrimid,H,F,H,H], [A438;2-Pyrimid,H,H,F,H], [A439;2-Pyrimid,H,H,H,F], [A440;2-Pyrimid,Cl,H,H,H], [A441;2-Pyrimid,H,Cl,H,H], [A442;2-Pyrimid,H,H,Cl,H], [A443;2-Pyrimid,H,H,H,Cl], [A444;2-Pyrimid,Me,H,H,H], [A445;2-Pyrimid,H,Me,H,H], [A446;2-Pyrimid,H,H,Me,H], [A447;2-Pyrimid,H,H,H,Me], [A448;2-Pyrimid,F,F,H,H], [A449;2-Pyrimid,F,H,F,H], [A450;2-Pyrimid,F,H,H,F], [A451;2-Pyrimid,H,F,F,H], [A452;2-Pyrimid,H,F,H,F], [A453;2-Pyrimid,H,H,F,F], [A454;2-Pyrimid,Cl,Cl,H,H], [A455;2-Pyrimid,Cl,H,Cl,H], [A456;2-Pyrimid,Cl,H,H,Cl], [A457;2-Pyrimid,H,Cl,Cl,H], [A458;2-Pyrimid,H,Cl,H,Cl], [A459;2-Pyrimid,H,H,Cl,Cl], [A460;2-Pyrimid,Me,Me,H,H], [A461;2-Pyrimid,Me,H,Me,H], [A462;2-Pyrimid,Me,H,H,Me], [A463;2-Pyrimid,H,Me,Me,H], [A464;2-Pyrimid,H,Me,H,Me], [A465;2-Pyrimid,H,H,Me,Me], [A466;4-Pyrimid,H,H,H,H], [A467;4-Pyrimid,F,H,H,H], [A468;4-Pyrimid,H,F,H,H], [A469;4-Pyrimid,H,H,F,H], [A470;4-Pyrimid,H,H,H,F], [A471;4-Pyrimid,Cl,H,H,H], [A472;4-Pyrimid,H,Cl,H,H], [A473;4-Pyrimid,H,H,Cl,H], [A474;4-Pyrimid,H,H,H,Cl], [A475;4-Pyrimid,Me,H,H,H], [A476;4-Pyrimid,H,Me,H,H], [A477;4-Pyrimid,H,H,Me,H], [A478;4-Pyrimid,H,H,H,Me], [A479;4-Pyrimid,F,F,H,H], [A480;4-Pyrimid,F,H,F,H], [A481;4-Pyrimid,F,H,H,F], [A482;4-Pyrimid,H,F,F,H], [A483;4-Pyrimid,H,F,H,F], [A484;4-Pyrimid,H,H,F,F], [A485;4-Pyrimid,Cl,Cl,H,H], [A486;4-Pyrimid,Cl,H,Cl,H], [A487;4-Pyrimid,Cl,H,H,Cl], [A488;4-Pyrimid,H,Cl,Cl,H], [A489;4-Pyrimid,H,Cl,H,Cl], [A490;4-Pyrimid,H,H,Cl,Cl], [A491;4-Pyrimid,Me,Me,H,H], [A492;4-Pyrimid,Me,H,Me,H], [A493;4-Pyrimid,Me,H,H,Me], [A494;4-Pyrimid,H,Me,Me,H], [A495;4-Pyrimid,H,Me,H,Me], [A496;4-Pyrimid,H,H,Me,Me], [A497;2-Pyrazin,H,H,H,H], [A498;2-Pyrazin,F,H,H,H], [A499;2-Pyrazin,H,F,H,H], [A500;2-Pyrazin,H,H,F,H], [A501;2-Pyrazin,H,H,H,F], [A502;2-Pyrazin,Cl,H,H,H], [A503;2-Pyrazin,H,Cl,H,H], [A504;2-Pyrazin,H,H,Cl,H], [A505;2-Pyrazin,H,H,H,Cl], [A506;2-Pyrazin,Me,H,H,H], [A507;2-Pyrazin,H,Me,H,H], [A508;2-Pyrazin,H,H,Me,H], [A509;2-Pyrazin,H,H,H,Me], [A510;2-Pyrazin,F,F,H,H], [A511;2-Pyrazin,F,H,F,H], [A512;2-Pyrazin,F,H,H,F], [A513;2-Pyrazin,H,F,F,H], [A514;2-Pyrazin,H,F,H,F], [A515;2-Pyrazin,H,H,F,F], [A516;2-Pyrazin,Cl,Cl,H,H], [A517;2-Pyrazin,Cl,H,Cl,H], [A518;2-Pyrazin,Cl,H,H,Cl], [A519;2-Pyrazin,H,Cl,Cl,H], [A520;2-Pyrazin,H,Cl,H,Cl], [A521;2-Pyrazin,H,H,Cl,Cl], [A522;2-Pyrazin,Me,Me,H,H], [A523;2-Pyrazin,Me,H,Me,H], [A524;2-Pyrazin,Me,H,H,Me], [A525;2-Pyrazin,H,Me,Me,H], [A526;2-Pyrazin,H,Me,H,Me], [A527;2-Pyrazin,H,H,Me,Me]

The Compound (A-1) wherein n represents 1, and the combination of Z, R^{8X}, R^{9X}, R^{10X}, and R^{11X} represents any one combination indicated in the Combination A (hereinafter referred to as "Compound group SX2").

The Compound (A-1) wherein n represents 2, and the combination of Z, R^{8X}, R^{9X}, R^{10X}, and R^{11X} represents any one combination indicated in the Combination A (hereinafter referred to as "Compound group SX3").

A compound represented by formula (A-2) 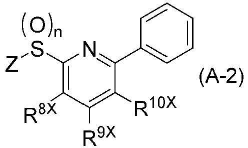 (hereinafter referred to as "Compound (A-2)") wherein n represents 0, and the combination of Z, R^{8X}, R^{9X}, and R^{10X} represents any one combination indicated in the Combination B (hereinafter referred to as "Compound group SX4").

Combination B consists of Substituent Numbers B1 to B323. Substituent Numbers B1 to B323 indicate the combinations of Z, R^{8X}, R^{9X}, and R^{10X} in the Compound (A-2), a compound represented by formula (A-3), a compound represented by formula (A-4), and a compound represented by formula (A-5), and are hereinafter referred to as [Substituent Number; Z,R^{8X},R^{9X},R^{10X}].

For example, Substituent Number B5 indicates a combination wherein Z represents an ethyl group, R^{8X} represents a chlorine atom, and R^{9X} and R^{10X} each represent a hydrogen atom.

Combination B: [B1;Et,H,H,H], [B2;Et,F,H,H], [B3;Et,H,F,H], [B4;Et,H,H,F], [B5;Et,Cl,H,H], [B6;Et,H,Cl,H], [B7;Et,H,H,Cl], [B8;Et,Me,H,H], [B9;Et,H,Me,H], [B10;Et,H,H,Me], [B11;Et,F,F,H], [B12;Et,F,H,F], [B13;Et,H,F,F], [B14;Et,Cl,Cl,H], [B15;Et,Cl,H,Cl], [B16;Et,H,Cl,Cl], [B17;Et,Me,Me,H], [B18;Et,Me,H,Me], [B19;Et,H,Me,Me], [B20;Pr,H,H,H], [B21;Pr,F,H,H], [B22;Pr,H,F,H], [B23;Pr,H,H,F], [B24;Pr,Cl,H,H], [B25;Pr,H,Cl,H], [B26;Pr,H,H,Cl], [B27;Pr,Me,H,H], [B28;Pr,H,Me,H], [B29;Pr,H,H,Me], [B30;Pr,F,F,H], [B31;Pr,F,H,F], [B32;Pr,H,F,F], [B33;Pr,Cl,Cl,H], [B34;Pr,Cl,H,Cl], [B35;Pr,H,Cl,Cl], [B36;Pr,Me,Me,H], [B37;Pr,Me,H,Me], [B38;Pr,H,Me,Me], [B39;i-Pr,H,H,H], [B40;i-Pr,F,H,H], [B41;i-Pr,H,F,H], [B42;i-Pr,H,H,F], [B43;i-Pr,Cl,H,H], [B44;i-Pr,H,Cl,H], [B45;i-Pr,H,H,Cl], [B46;i-Pr,Me,H,H], [B47;i-Pr,H,Me,H], [B48;i-Pr,H,H,Me], [B49;i-Pr,F,F,H], [B50;i-Pr,F,H,F], [B51;i-Pr,H,F,F], [B52;i-Pr,Cl,Cl,H], [B53;i-Pr,Cl,H,Cl], [B54;i-Pr,H,Cl,Cl], [B55;i-Pr,Me,Me,H], [B56;i-Pr,Me,H,Me], [B57;i-Pr,H,Me,Me], [B58;CH=CHCH₃,H,H,H], [B59;CH=CHCH₃,F,H,H], [B60;CH=CHCH₃,H,F,H], [B61;CH=CHCH₃,H,H,F], [B62;CH=CHCH₃,Cl,H,H], [B63;CH=CHCH₃,H,Cl,H], [B64;CH=CHCH₃,H,H,Cl], [B65;CH=CHCH₃,Me,H,H], [B66;CH=CHCH₃,H,Me,H], [B67;CH=CHCH₃,H,H,Me], [B68;CH=CHCH₃,F,F,H], [B69;CH=CHCH₃,F,H,F], [B70;CH=CHCH₃,H,F,F], [B71;CH=CHCH₃,Cl,Cl,H], [B72;CH=CHCH₃,Cl,H,Cl], [B73;CH=CHCH₃,H,Cl,Cl], [B74;CH=CHCH₃,Me,Me,H], [B75;CH=CHCH₃,Me,H,Me], [B76;CH=CHCH₃,H,Me,Me], [B77;CH₂CH=CH₂,H,H,H], [B78;CH₂CH=CH₂,F,H,H], [B79;CH₂CH=CH₂,H,F,H], [B80;CH₂CH=CH₂,H,H,F], [B81;CH₂CH=CH₂,Cl,H,H], [B82;CH₂CH=CH₂,H,Cl,H], [B83;CH₂CH=CH₂,H,H,Cl], [B84;CH₂CH=CH₂,Me,H,H], [B85;CH₂CH=CH₂,H,Me,H], [B86;CH₂CH=CH₂,H,H,Me], [B87;CH₂CH=CH₂,F,F,H], [B88;CH₂CH=CH₂,F,H,F], [B89;CH₂CH=CH₂,H,F,F], [B90;CH₂CH=CH₂,Cl,Cl,H], [B91;CH₂CH=CH₂,Cl,H,Cl], [B92;CH₂CH=CH₂,H,Cl,Cl], [B93;CH₂CH=CH₂,Me,Me,H], [B94;CH₂CH=CH₂,Me,H,Me], [B95;CH₂CH=CH₂,H,Me,Me], [B96;Bu,H,H,H], [B97;Bu,F,H,H], [B98;Bu,H,F,H], [B99;Bu,H,H,F], [B100;Bu,Cl,H,H], [B101;Bu,H,Cl,H], [B102;Bu,H,H,Cl], [B103;Bu,Me,H,H], [B104;Bu,H,Me,H], [B105;Bu,H,H,Me], [B106;Bu,F,F,H], [B107;Bu,F,H,F], [B108;Bu,H,F,F], [B109;Bu,Cl,Cl,H], [B110;Bu,Cl,H,Cl], [B111;Bu,H,Cl,Cl], [B112;Bu,Me,Me,H], [B113;Bu,Me,H,Me], [B114;Bu,H,Me,Me], [B115;c-Pr,H,H,H], [B116;c-Pr,F,H,H], [B117;c-Pr,H,F,H], [B118;c-Pr,H,H,F], [B119;c-Pr,Cl,H,H], [B120;c-Pr,H,Cl,H], [B121;c-Pr,H,H,Cl], [B122;c-Pr,Me,H,H], [B123;c-Pr,H,Me,H], [B124;c-Pr,H,H,Me], [B125;c-Pr,F,F,H], [B126;c-Pr,F,H,F], [B127;c-Pr,H,F,F], [B128;c-Pr,Cl,Cl,H], [B129;c-Pr,Cl,H,Cl], [B130;c-Pr,H,Cl,Cl], [B131;c-Pr,Me,Me,H], [B132;c-Pr,Me,H,Me], [B133;c-Pr,H,Me,Me], [B134;2-Thie,H,H,H], [B135;2-Thie,F,H,H], [B136;2-Thie,H,F,H], [B137;2-Thie,H,H,F], [B138;2-Thie,Cl,H,H], [B139;2-Thie,H,Cl,H], [B140;2-Thie,H,H,Cl], [B141;2-Thie,Me,H,H], [B142;2-Thie,H,Me,H], [B143;2-Thie,H,H,Me], [B144;2-Thie,F,F,H], [B145;2-Thie,F,H,F], [B146;2-Thie,H,F,F], [B147;2-Thie,Cl,Cl,H], [B148;2-Thie,Cl,H,Cl], [B149;2-Thie,H,Cl,Cl], [B150;2-Thie,Me,Me,H], [B151;2-Thie,Me,H,Me], [B152;2-Thie,H,Me,Me], [B153;3-Thie,H,H,H], [B154;3-Thie,F,H,H], [B155;3-Thie,H,F,H], [B156;3-Thie,H,H,F], [B157;3-Thie,Cl,H,H], [B158;3-Thie,H,Cl,H], [B159;3-Thie,H,H,Cl], [B160;3-Thie,Me,H,H], [B161;3-Thie,H,Me,H], [B162;3-Thie,H,H,Me], [B163;3-Thie,F,F,H], [B164;3-Thie,F,H,F], [B165;3-Thie,H,F,F], [B166;3-Thie,Cl,Cl,H], [B167;3-Thie,Cl,H,Cl], [B168;3-Thie,H,Cl,Cl], [B169;3-Thie,Me,Me,H], [B170;3-Thie,Me,H,Me], [B171;3-Thie,H,Me,Me], [B172;2-Fura,H,H,H], [B173;2-Fura,F,H,H], [B174;2-Fura,H,F,H], [B175;2-Fura,H,H,F], [B176;2-Fura,Cl,H,H], [B177;2-Fura,H,Cl,H], [B178;2-Fura,H,H,Cl], [B179;2-Fura,Me,H,H], [B180;2-Fura,H,Me,H], [B181;2-Fura,H,H,Me], [B182;2-Fura,F,F,H], [B183;2-Fura,F,H,F], [B184;2-Fura,H,F,F], [B185;2-Fura,Cl,Cl,H], [B186;2-Fura,Cl,H,Cl], [B187;2-Fura,H,Cl,Cl], [B188;2-Fura,Me,Me,H], [B189;2-Fura,Me,H,Me], [B190;2-Fura,H,Me,Me], [B191;3-Fura,H,H,H], [B192;3-Fura,F,H,H], [B193;3-Fura,H,F,H], [B194;3-Fura,H,H,F], [B195;3-Fura,Cl,H,H], [B196;3-Fura,H,Cl,H], [B197;3-Fura,H,H,Cl], [B198;3-Fura,Me,H,H], [B199;3-Fura,H,Me,H], [B200;3-Fura,H,H,Me], [B201;3-Fura,F,F,H], [B202;3-Fura,F,H,F], [B203;3-Fura,H,F,F], [B204;3-Fura,Cl,Cl,H], [B205;3-Fura,Cl,H,Cl], [B206;3-Fura,H,Cl,Cl], [B207;3-Fura,Me,Me,H], [B208;3-Fura,Me,H,Me], [B209;3-Fura,H,Me,Me], [B210;2-Py,H,H,H], [B211;2-Py,F,H,H], [B212;2-Py,H,F,H], [B213;2-Py,H,H,F], [B214;2-Py,Cl,H,H], [B215;2-Py,H,Cl,H], [B216;2-Py,H,H,Cl], [B217;2-Py,Me,H,H], [B218;2-Py,H,Me,H], [B219;2-Py,H,H,Me], [B220;2-Py,F,F,H], [B221;2-Py,F,H,F], [B222;2-Py,H,F,F], [B223;2-Py,Cl,Cl,H], [B224;2-Py,Cl,H,Cl], [B225;2-Py,H,Cl,Cl], [B226;2-Py,Me,Me,H], [B227;2-Py,Me,H,Me], [B228;2-Py,H,Me,Me], [B229;3-Py,H,H,H], [B230;3-Py,F,H,H], [B231;3-Py,H,F,H], [B232;3-Py,H,H,F], [B233;3-Py,Cl,H,H], [B234;3-Py,H,Cl,H], [B235;3-Py,H,H,Cl], [B236;3-Py,Me,H,H], [B237;3-Py,H,Me,H], [B238;3-Py,H,H,Me], [B239;3-Py,F,F,H], [B240;3-Py,F,H,F], [B241;3-Py,H,F,F], [B242;3-Py,Cl,Cl,H], [B243;3-Py,Cl,H,Cl], [B244;3-Py,H,Cl,Cl], [B245;3-Py,Me,Me,H], [B246;3-Py,Me,H,Me], [B247;3-Py,H,Me,Me], [B248;4-Py,H,H,H], [B249;4-Py,F,H,H], [B250;4-Py,H,F,H], [B251;4-Py,H,H,F], [B252;4-Py,Cl,H,H], [B253;4-Py,H,Cl,H], [B254;4-Py,H,H,Cl], [B255;4-Py,Me,H,H], [B256;4-Py,H,Me,H], [B257;4-Py,H,H,Me], [B258;4-Py,F,F,H], [B259;4-Py,F,H,F], [B260;4-Py,H,F,F], [B261;4-Py,Cl,Cl,H], [B262;4-Py,Cl,H,Cl], [B263;4-Py,H,Cl,Cl], [B264;4-Py,Me,Me,H], [B265;4-Py,Me,H,Me], [B266;4-Py,H,Me,Me], [B267;2-Pyrimid,H,H,H], [B268;2-Pyrimid,F,H,H], [B269;2-Pyrimid,H,F,H], [B270;2-Pyrimid,H,H,F], [B271;2-Pyrimid,Cl,H,H], [B272;2-Pyrimid,H,Cl,H], [B273;2-Pyrimid,H,H,Cl], [B274;2-Pyrimid,Me,H,H], [B275;2-Pyrimid,H,Me,H], [B276;2-Pyrimid,H,H,Me], [B277;2-Pyrimid,F,F,H], [B278;2-Pyrimid,F,H,F], [B279;2-Pyrimid,H,F,F], [B280;2-Pyrimid,Cl,Cl,H], [B281;2-Pyrimid,Cl,H,Cl], [B282;2-Pyrimid,H,Cl,Cl], [B283;2-Pyrimid,Me,Me,H], [B284;2-Pyrimid,Me,H,Me], [B285;2-Pyrimid,H,Me,Me], [B286;4-Pyrimid,H,H,H], [B287;4-Pyrimid,F,H,H], [B288;4-Pyrimid,H,F,H], [B289;4-Pyrimid,H,H,F], [B290;4-Pyrimid,Cl,H,H], [B291;4-Pyrimid,H,Cl,H], [B292;4-Pyrimid,H,H,Cl], [B293;4-Pyrimid,Me,H,H], [B294;4-Pyrimid,H,Me,H], [B295;4-Pyrimid,H,H,Me], [B296;4-Pyrimid,F,F,H], [B297;4-Pyrimid,F,H,F], [B298;4-Pyrimid,H,F,F], [B299;4-Pyrimid,Cl,Cl,H], [B300;4-Pyrimid,Cl,H,Cl], [B301;4-Pyrimid,H,Cl,Cl], [B302;4-Pyrimid,Me,Me,H], [B303;4-Pyrimid,Me,H,Me], [B304;4-Pyrimid,H,Me,Me], [B305;2-Pyrazin,H,H,H], [B306;2-Pyrazin,F,H,H], [B307;2-Pyrazin,H,F,H], [B308;2-Pyrazin,H,H,F], [B309;2-Pyrazin,Cl,H,H], [B310;2-Pyrazin,H,Cl,H], [B311;2-Pyrazin,H,H,Cl], [B312;2-Pyrazin,Me,H,H], [B313;2-Pyrazin,H,Me,H], [B314;2-Pyrazin,H,H,Me], [B315;2-Pyrazin,F,F,H], [B316;2-Pyrazin,F,H,F], [B317;2-Pyrazin,H,F,F], [B318;2-Pyrazin,Cl,Cl,H], [B319;2-Pyrazin,Cl,H,Cl], [B320;2-Pyrazin,H,Cl,Cl], [B321;2-Pyrazin,Me,Me,H], [B322;2-Pyrazin,Me,H,Me], [B323;2-Pyrazin,H,Me,Me]

The Compound (A-2) wherein n represents 1, and the combination of Z, R^{8X}, R^{9X}, and R^{10X} represents any one combination indicated in the Combination B (hereinafter referred to as "Compound group SX5").

The Compound (A-2) wherein n represents 2, and the combination of Z, R^{8X}, R^{9X}, and R^{10X} represents any one combination indicated in the Combination B (hereinafter referred to as "Compound group SX6").

A compound represented by formula (A-3) 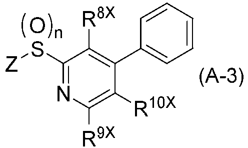 (hereinafter referred to as "Compound (A-3)") wherein n represents 0, and the combination of Z, R^{8X}, R^{9X}, and R^{10X} represents any one combination indicated in the Combination B (hereinafter referred to as "Compound group SX7").

The Compound (A-3) wherein n represents 1, and the combination of Z, R^{8X}, R^{9X}, and R^{10X} represents any one combination indicated in the Combination B (hereinafter referred to as "Compound group SX8").

The Compound (A-3) wherein n represents 2, and the combination of Z, R^{8X}, R^{9X}, and R^{10X} represents any one combination indicated in the Combination B (hereinafter referred to as "Compound group SX9").

A compound represented by formula (A-4) 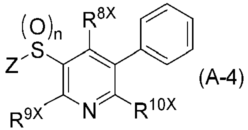 (hereinafter referred to as "Compound (A-4)") wherein n represents 0, and the combination of Z, R^{8X}, R^{9X}, and R^{10X} represents any one combination indicated in the Combination B (hereinafter referred to as "Compound group SX10").

The Compound (A-4) wherein n represents 1, and the combination of Z, R^{8X}, R^{9X}, and R^{10X} represents any one combination indicated in the Combination B (hereinafter referred to as "Compound group SX11").

The Compound (A-4) wherein n represents 2, and the combination of Z, R^{8X}, R^{9X}, and R^{10X} represents any one combination indicated in the Combination B (hereinafter referred to as "Compound group SX12").

The Compound (A-4) wherein n represents 0, and the combination of Z, R^{8X}, R^{9X}, and R^{10X} represents any one combination indicated in the Combination D (hereinafter referred to as "Compound group SX13").

Combination D consists of Substituent Numbers D1 to D532. Substituent Numbers D1 to D532 indicate the combinations of Z, R^{8X}, R^{9X}, and R^{10X} in the Compound (A-4), and are hereinafter referred to as [Substituent Number; Z,R^{8X},R^{9X},R^{10X}].

For example, Substituent Number D5 indicates a combination wherein Z represents a phenyl group, R^{8X} represents a chlorine atom, and R^{9X} and R^{10X} each represent a hydrogen atom.

Combination D: [D1;Ph,H,H,H], [D2;Ph,F,H,H], [D3;Ph,H,F,H], [D4;Ph,H,H,F], [D5;Ph,Cl,H,H], [D6;Ph,H,Cl,H], [D7;Ph,H,H,Cl], [D8;Ph,Me,H,H], [D9;Ph,H,Me,H], [D10;Ph,H,H,Me], [D11;Ph,F,F,H], [D12;Ph,F,H,F], [D13;Ph,H,F,F], [D14;Ph,Cl,Cl,H], [D15;Ph,Cl,H,Cl], [D16;Ph,H,Cl,Cl], [D17;Ph,Me,Me,H], [D18;Ph,Me,H,Me], [D19;Ph,H,Me,Me], [D20;2-F-Ph,H,H,H], [D21;2-F-Ph,F,H,H], [D22;2-F-Ph,H,F,H], [D23;2-F-Ph,H,H,F], [D24;2-F-Ph,Cl,H,H], [D25;2-F-Ph,H,Cl,H], [D26;2-F-Ph,H,H,Cl], [D27;2-F-Ph,Me,H,H], [D28;2-F-Ph,H,Me,H], [D29;2-F-Ph,H,H,Me], [D30;2-F-Ph,F,F,H], [D31;2-F-Ph,F,H,F], [D32;2-F-Ph,H,F,F], [D33;2-F-Ph,Cl,Cl,H], [D34;2-F-Ph,Cl,H,Cl], [D35;2-F-Ph,H,Cl,Cl], [D36;2-F-Ph,Me,Me,H], [D37;2-F-Ph,Me,H,Me], [D38;2-F-Ph,H,Me,Me], [D39;3-F-Ph,H,H,H], [D40;3-F-Ph,F,H,H], [D41;3-F-Ph,H,F,H], [D42;3-F-Ph,H,H,F], [D43;3-F-Ph,Cl,H,H], [D44;3-F-Ph,H,Cl,H], [D45;3-F-Ph,H,H,Cl], [D46;3-F-Ph,Me,H,H], [D47;3-F-Ph,H,Me,H], [D48;3-F-Ph,H,H,Me], [D49;3-F-Ph,F,F,H], [D50;3-F-Ph,F,H,F], [D51;3-F-Ph,H,F,F], [D52;3-F-Ph,Cl,Cl,H], [D53;3-F-Ph,Cl,H,Cl], [D54;3-F-Ph,H,Cl,Cl], [D55;3-F-Ph,Me,Me,H], [D56;3-F-Ph,Me,H,Me], [D57;3-F-Ph,H,Me,Me], [D58;4-F-Ph,H,H,H], [D59;4-F-Ph,F,H,H], [D60;4-F-Ph,H,F,H], [D61;4-F-Ph,H,H,F], [D62;4-F-Ph,Cl,H,H], [D63;4-F-Ph,H,Cl,H], [D64;4-F-Ph,H,H,Cl], [D65;4-F-Ph,Me,H,H], [D66;4-F-Ph,H,Me,H], [D67;4-F-Ph,H,H,Me], [D68;4-F-Ph,F,F,H], [D69;4-F-Ph,F,H,F], [D70;4-F-Ph,H,F,F], [D71;4-F-Ph,Cl,Cl,H], [D72;4-F-Ph,Cl,H,Cl], [D73;4-F-Ph,H,Cl,Cl], [D74;4-F-Ph,Me,Me,H], [D75;4-F-Ph,Me,H,Me], [D76;4-F-Ph,H,Me,Me], [D77;2-Cl-Ph,H,H,H], [D78;2-Cl-Ph,F,H,H], [D79;2-Cl-Ph,H,F,H], [D80;2-Cl-Ph,H,H,F], [D81;2-Cl-Ph,Cl,H,H], [D82;2-Cl-Ph,H,Cl,H], [D83;2-Cl-Ph,H,H,Cl], [D84;2-Cl-Ph,Me,H,H], [D85;2-Cl-Ph,H,Me,H], [D86;2-Cl-Ph,H,H,Me], [D87;2-Cl-Ph,F,F,H], [D88;2-Cl-Ph,F,H,F], [D89;2-Cl-Ph,H,F,F], [D90;2-Cl-Ph,Cl,Cl,H], [D91;2-Cl-Ph,Cl,H,Cl], [D92;2-Cl-Ph,H,Cl,Cl], [D93;2-Cl-Ph,Me,Me,H], [D94;2-Cl-Ph,Me,H,Me], [D95;2-Cl-Ph,H,Me,Me], [D96;3-Cl-Ph,H,H,H], [D97;3-Cl-Ph,F,H,H], [D98;3-Cl-Ph,H,F,H], [D99;3-Cl-Ph,H,H,F], [D100;3-Cl-Ph,Cl,H,H], [D101;3-Cl-Ph,H,Cl,H], [D102;3-Cl-Ph,H,H,Cl], [D103;3-Cl-Ph,Me,H,H], [D104;3-Cl-Ph,H,Me,H], [D105;3-Cl-Ph,H,H,Me], [D106;3-Cl-Ph,F,F,H], [D107;3-Cl-Ph,F,H,F], [D108;3-Cl-Ph,H,F,F], [D109;3-Cl-Ph,Cl,Cl,H], [D110;3-Cl-Ph,Cl,H,Cl], [D111;3-Cl-Ph,H,Cl,Cl], [D112;3-Cl-Ph,Me,Me,H], [D113;3-Cl-Ph,Me,H,Me], [D114;3-Cl-Ph,H,Me,Me], [D115;4-Cl-Ph,H,H,H], [D116;4-Cl-Ph,F,H,H], [D117;4-Cl-Ph,H,F,H], [D118;4-Cl-Ph,H,H,F], [D119;4-Cl-Ph,Cl,H,H], [D120;4-Cl-Ph,H,Cl,H], [D121;4-Cl-Ph,H,H,Cl], [D122;4-Cl-Ph,Me,H,H], [D123;4-Cl-Ph,H,Me,H], [D124;4-Cl-Ph,H,H,Me], [D125;4-Cl-Ph,F,F,H], [D126;4-Cl-Ph,F,H,F], [D127;4-Cl-Ph,H,F,F], [D128;4-Cl-Ph,Cl,Cl,H], [D129;4-Cl-Ph,Cl,H,Cl], [D130;4-Cl-Ph,H,Cl,Cl], [D131;4-Cl-Ph,Me,Me,H], [D132;4-Cl-Ph,Me,H,Me], [D133;4-Cl-Ph,H,Me,Me], [D134;2-Me-Ph,H,H,H], [D135;2-Me-Ph,F,H,H], [D136;2-Me-Ph,H,F,H], [D137;2-Me-Ph,H,H,F], [D138;2-Me-Ph,Cl,H,H], [D139;2-Me-Ph,H,Cl,H], [D140;2-Me-Ph,H,H,Cl], [D141;2-Me-Ph,Me,H,H], [D142;2-Me-Ph,H,Me,H], [D143;2-Me-Ph,H,H,Me], [D144;2-Me-Ph,F,F,H], [D145;2-Me-Ph,F,H,F], [D146;2-Me-Ph,H,F,F], [D147;2-Me-Ph,Cl,Cl,H], [D148;2-Me-Ph,Cl,H,Cl], [D149;2-Me-Ph,H,Cl,Cl], [D150;2-Me-Ph,Me,Me,H], [D151;2-Me-Ph,Me,H,Me], [D152;2-Me-Ph,H,Me,Me], [D153;3-Me-Ph,H,H,H], [D154;3-Me-Ph,F,H,H], [D155;3-Me-Ph,H,F,H], [D156;3-Me-Ph,H,H,F], [D157;3-Me-Ph,Cl,H,H], [D158;3-Me-Ph,H,Cl,H], [D159;3-Me-Ph,H,H,Cl], [D160;3-Me-Ph,Me,H,H], [D161;3-Me-Ph,H,Me,H], [D162;3-Me-Ph,H,H,Me], [D163;3-Me-Ph,F,F,H], [D164;3-Me-Ph,F,H,F], [D165;3-Me-Ph,H,F,F], [D166;3-Me-Ph,Cl,Cl,H], [D167;3-Me-Ph,Cl,H,Cl], [D168;3-Me-Ph,H,Cl,Cl], [D169;3-Me-Ph,Me,Me,H], [D170;3-Me-Ph,Me,H,Me], [D171;4-Me-Ph,H,Me,Me], [D172;4-Me-Ph,H,H,H], [D173;4-Me-Ph,F,H,H], [D174;4-Me-Ph,H,F,H], [D175;4-Me-Ph,H,H,F], [D176;4-Me-Ph,Cl,H,H], [D177;4-Me-Ph,H,Cl,H], [D178;4-Me-Ph,H,H,Cl], [D179;4-Me-Ph,Me,H,H], [D180;4-Me-Ph,H,Me,H], [D181;4-Me-Ph,H,H,Me], [D182;4-Me-Ph,F,F,H], [D183;4-Me-Ph,F,H,F], [D184;4-Me-Ph,H,F,F], [D185;4-Me-Ph,Cl,Cl,H], [D186;4-Me-Ph,Cl,H,Cl], [D187;4-Me-Ph,H,Cl,Cl], [D188;4-Me-Ph,Me,Me,H], [D189;4-Me-Ph,Me,H,Me], [D190;4-Me-Ph,H,Me,Me], [D191;2-OMe-Ph,H,H,H], [D192;2-OMe-Ph,F,H,H], [D193;2-OMe-Ph,H,F,H], [D194;2-OMe-Ph,H,H,F], [D195;2-OMe-Ph,Cl,H,H], [D196;2-OMe-Ph,H,Cl,H], [D197;2-OMe-Ph,H,H,Cl], [D198;2-OMe-Ph,Me,H,H], [D199;2-OMe-Ph,H,Me,H], [D200;2-OMe-Ph,H,H,Me], [D201;2-OMe-Ph,F,F,H], [D202;2-OMe-Ph,F,H,F], [D203;2-OMe-Ph,H,F,F], [D204;2-OMe-Ph,Cl,Cl,H], [D205;2-OMe-Ph,Cl,H,Cl], [D206;2-OMe-Ph,H,Cl,Cl], [D207;2-OMe-Ph,Me,Me,H], [D208;2-OMe-Ph,Me,H,Me], [D209;3-OMe-Ph,H,Me,Me], [D210;3-OMe-Ph,H,H,H], [D211;3-OMe-Ph,F,H,H], [D212;3-OMe-Ph,H,F,H], [D213;3-OMe-Ph,H,H,F], [D214;3-OMe-Ph,Cl,H,H], [D215;3-OMe-Ph,H,Cl,H], [D216;3-OMe-Ph,H,H,Cl], [D217;3-OMe-Ph,Me,H,H], [D218;3-OMe-Ph,H,Me,H], [D219;3-OMe-Ph,H,H,Me], [D220;3-OMe-Ph,F,F,H], [D221;3-OMe-Ph,F,H,F], [D222;3-OMe-Ph,H,F,F], [D223;3-OMe-Ph,Cl,Cl,H], [D224;3-OMe-Ph,Cl,H,Cl], [D225;3-OMe-Ph,H,Cl,Cl], [D226;3-OMe-Ph,Me,Me,H], [D227;3-OMe-Ph,Me,H,Me], [D228;3-OMe-Ph,H,Me,Me], [D229;4-OMe-Ph,H,H,H], [D230;4-OMe-Ph,F,H,H], [D231;4-OMe-Ph,H,F,H], [D232;4-OMe-Ph,H,H,F], [D233;4-OMe-Ph,Cl,H,H], [D234;4-OMe-Ph,H,Cl,H], [D235;4-OMe-Ph,H,H,Cl], [D236;4-OMe-Ph,Me,H,H], [D237;4-OMe-Ph,H,Me,H], [D238;4-OMe-Ph,H,H,Me], [D239;4-OMe-Ph,F,F,H], [D240;4-OMe-Ph,F,H,F], [D241;4-OMe-Ph,H,F,F], [D242;4-OMe-Ph,Cl,Cl,H], [D243;4-OMe-Ph,Cl,H,Cl], [D244;4-OMe-Ph,H,Cl,Cl], [D245;4-OMe-Ph,Me,Me,H], [D246;4-OMe-Ph,Me,H,Me], [D247;4-OMe-Ph,H,Me,Me], [D248;2-CN-Ph,H,H,H], [D249;2-CN-Ph,F,H,H], [D250;2-CN-Ph,H,F,H], [D251;2-CN-Ph,H,H,F], [D252;2-CN-Ph,Cl,H,H], [D253;2-CN-Ph,H,Cl,H], [D254;2-CN-Ph,H,H,Cl], [D255;2-CN-Ph,Me,H,H], [D256;2-CN-Ph,H,Me,H], [D257;2-CN-Ph,H,H,Me], [D258;2-CN-Ph,F,F,H], [D259;2-CN-Ph,F,H,F], [D260;2-CN-Ph,H,F,F], [D261;2-CN-Ph,Cl,Cl,H], [D262;2-CN-Ph,Cl,H,Cl], [D263;2-CN-Ph,H,Cl,Cl], [D264;2-CN-Ph,Me,Me,H], [D265;2-CN-Ph,Me,H,Me], [D266;2-CN-Ph,H,Me,Me], [D267;3-CN-Ph,H,H,H], [D268;3-CN-Ph,F,H,H], [D269;3-CN-Ph,H,F,H], [D270;3-CN-Ph,H,H,F], [D271;3-CN-Ph,Cl,H,H], [D272;3-CN-Ph,H,Cl,H], [D273;3-CN-Ph,H,H,Cl], [D274;3-CN-Ph,Me,H,H], [D275;3-CN-Ph,H,Me,H], [D276;3-CN-Ph,H,H,Me], [D277;3-CN-Ph,F,F,H], [D278;3-CN-Ph,F,H,F], [D279;3-CN-Ph,H,F,F], [D280;3-CN-Ph,Cl,Cl,H], [D281;3-CN-Ph,Cl,H,Cl], [D282;3-CN-Ph,H,Cl,Cl], [D283;3-CN-Ph,Me,Me,H], [D284;3-CN-Ph,Me,H,Me], [D285;3-CN-Ph,H,Me,Me], [D286;4-CN-Ph,H,H,H], [D287;4-CN-Ph,F,H,H], [D288;4-CN-Ph,H,F,H], [D289;4-CN-Ph,H,H,F], [D290;4-CN-Ph,Cl,H,H], [D291;4-CN-Ph,H,Cl,H], [D292;4-CN-Ph,H,H,Cl], [D293;4-CN-Ph,Me,H,H], [D294;4-CN-Ph,H,Me,H], [D295;4-CN-Ph,H,H,Me], [D296;4-CN-Ph,F,F,H], [D297;4-CN-Ph,F,H,F], [D298;4-CN-Ph,H,F,F], [D299;4-CN-Ph,Cl,Cl,H], [D300;4-CN-Ph,Cl,H,Cl], [D301;4-CN-Ph,H,Cl,Cl], [D302;4-CN-Ph,Me,Me,H], [D303;4-CN-Ph,Me,H,Me], [D304;4-CN-Ph,H,Me,Me], [D305;2,3-F₂-Ph,H,H,H], [D306;2,3-F₂-Ph,F,H,H], [D307;2,3-F₂-Ph,H,F,H], [D308;2,3-F₂-Ph,H,H,F], [D309;2,3-F₂-Ph,Cl,H,H], [D310;2,3-F₂-Ph,H,Cl,H], [D311;2,3-F₂-Ph,H,H,Cl], [D312;2,3-F₂-Ph,Me,H,H], [D313;2,3-F₂-Ph,H,Me,H], [D314;2,3-F₂-Ph,H,H,Me], [D315;2,3-F₂-Ph,F,F,H], [D316;2,3-F₂-Ph,F,H,F], [D317;2,3-F₂-Ph,H,F,F], [D318;2,3-F₂-Ph,Cl,Cl,H], [D319;2,3-F₂-Ph,Cl,H,Cl], [D320;2,3-F₂-Ph,H,Cl,Cl], [D321;2,3-F₂-Ph,Me,Me,H], [D322;2,3-F₂-Ph,Me,H,Me], [D323;2,3-F₂-Ph,H,Me,Me], [D324;2,4-F₂-Ph,H,H,H], [D325;2,4-F₂-Ph,F,H,H], [D326;2,4-F₂-Ph,H,F,H], [D327;2,4-F₂-Ph,H,H,F], [D328;2,4-F₂-Ph,Cl,H,H], [D329;2,4-F₂-Ph,H,Cl,H], [D330;2,4-F₂-Ph,H,H,Cl], [D331;2,4-F₂-Ph,Me,H,H], [D332;2,4-F₂-Ph,H,Me,H], [D333;2,4-F₂-Ph,H,H,Me], [D334;2,4-F₂-Ph,F,F,H], [D335;2,4-F₂-Ph,F,H,F], [D336;2,4-F₂-Ph,H,F,F], [D337;2,4-F₂-Ph,Cl,Cl,H], [D338;2,4-F₂-Ph,Cl,H,Cl], [D339;2,4-F₂-Ph,H,Cl,Cl], [D340;2,4-F₂-Ph,Me,Me,H], [D341;2,4-F₂-Ph,Me,H,Me], [D342;2,4-F₂-Ph,H,Me,Me], [D343;2,5-F₂-Ph,H,H,H], [D344;2,5-F₂-Ph,F,H,H], [D345;2,5-F₂-Ph,H,F,H], [D346;2,5-F₂-Ph,H,H,F], [D347;2,5-F₂-Ph,Cl,H,H], [D348;2,5-F₂-Ph,H,Cl,H], [D349;2,5-F₂-Ph,H,H,Cl], [D350;2,5-F₂-Ph,Me,H,H], [D351;2,5-F₂-Ph,H,Me,H], [D352;2,5-F₂-Ph,H,H,Me], [D353;2,5-F₂-Ph,F,F,H], [D354;2,5-F₂-Ph,F,H,F], [D355;2,5-F₂-Ph,H,F,F], [D356;2,5-F₂-Ph,Cl,Cl,H], [D357;2,5-F₂-Ph,Cl,H,Cl], [D358;2,5-F₂-Ph,H,Cl,Cl], [D359;2,5-F₂-Ph,Me,Me,H], [D360;2,5-F₂-Ph,Me,H,Me], [D361;2,5-F₂-Ph,H,Me,Me], [D362;2,6-F₂-Ph,H,H,H], [D363;2,6-F₂-Ph,F,H,H], [D364;2,6-F₂-Ph,H,F,H], [D365;2,6-F₂-Ph,H,H,F], [D366;2,6-F₂-Ph,Cl,H,H], [D367;2,6-F₂-Ph,H,Cl,H], [D368;2,6-F₂-Ph,H,H,Cl], [D369;2,6-F₂-Ph,Me,H,H], [D370;2,6-F₂-Ph,H,Me,H], [D371;2,6-F₂-Ph,H,H,Me], [D372;2,6-F₂-Ph,F,F,H], [D373;2,6-F₂-Ph,F,H,F], [D374;2,6-F₂-Ph,H,F,F], [D375;2,6-F₂-Ph,Cl,Cl,H], [D376;2,6-F₂-Ph,Cl,H,Cl], [D377;2,6-F₂-Ph,H,Cl,Cl], [D378;2,6-F₂-Ph,Me,Me,H], [D379;2,6-F₂-Ph,Me,H,Me], [D380;2,6-F₂-Ph,H,Me,Me], [D381;3,4-F₂-Ph,H,H,H], [D382;3,4-F₂-Ph,F,H,H], [D383;3,4-F₂-Ph,H,F,H], [D384;3,4-F₂-Ph,H,H,F], [D385;3,4-F₂-Ph,Cl,H,H], [D386;3,4-F₂-Ph,H,Cl,H], [D387;3,4-F₂-Ph,H,H,Cl], [D388;3,4-F₂-Ph,Me,H,H], [D389;3,4-F₂-Ph,H,Me,H], [D390;3,4-F₂-Ph,H,H,Me], [D391;3,4-F₂-Ph,F,F,H], [D392;3,4-F₂-Ph,F,H,F], [D393;3,4-F₂-Ph,H,F,F], [D394;3,4-F₂-Ph,Cl,Cl,H], [D395;3,4-F₂-Ph,Cl,H,Cl], [D396;3,4-F₂-Ph,H,Cl,Cl], [D397;3,4-F₂-Ph,Me,Me,H], [D398;3,4-F₂-Ph,Me,H,Me], [D399;3,4-F₂-Ph,H,Me,Me], [D400;3,5-F₂-Ph,H,H,H], [D401;3,5-F₂-Ph,F,H,H], [D402;3,5-F₂-Ph,H,F,H], [D403;3,5-F₂-Ph,H,H,F], [D404;3,5-F₂-Ph,Cl,H,H], [D405;3,5-F₂-Ph,H,Cl,H], [D406;3,5-F₂-Ph,H,H,Cl], [D407;3,5-F₂-Ph,Me,H,H], [D408;3,5-F₂-Ph,H,Me,H], [D409;3,5-F₂-Ph,H,H,Me], [D410;3,5-F₂-Ph,F,F,H], [D411;3,5-F₂-Ph,F,H,F], [D412;3,5-F₂-Ph,H,F,F], [D413;3,5-F₂-Ph,Cl,Cl,H], [D414;3,5-F₂-Ph,Cl,H,Cl], [D415;3,5-F₂-Ph,H,Cl,Cl], [D416;3,5-F₂-Ph,Me,Me,H], [D417;3,5-F₂-Ph,Me,H,Me], [D418;3,5-F₂-Ph,H,Me,Me], [D419;2,3-Me₂-Ph,H,H,H], [D420;2,3-Me₂-Ph,F,H,H], [D421;2,3-Me₂-Ph,H,F,H], [D422;2,3-Me₂-Ph,H,H,F], [D423;2,3-Me₂-Ph,Cl,H,H], [D424;2,3-Me₂-Ph,H,Cl,H], [D425;2,3-Me₂-Ph,H,H,Cl], [D426;2,3-Me₂-Ph,Me,H,H], [D427;2,3-Me₂-Ph,H,Me,H], [D428;2,3-Me₂-Ph,H,H,Me], [D429;2,3-Me₂-Ph,F,F,H], [D430;2,3-Me₂-Ph,F,H,F], [D431;2,3-Me₂-Ph,H,F,F], [D432;2,3-Me₂-Ph,Cl,Cl,H], [D433;2,3-Me₂-Ph,Cl,H,Cl], [D434;2,3-Me₂-Ph,H,Cl,Cl], [D435;2,3-Me₂-Ph,Me,Me,H], [D436;2,3-Me₂-Ph,Me,H,Me], [D437;2,3-Me₂-Ph,H,Me,Me], [D438;2,4-Me₂-Ph,H,H,H], [D439;2,4-Me₂-Ph,F,H,H], [D440;2,4-Me₂-Ph,H,F,H], [D441;2,4-Me₂-Ph,H,H,F], [D442;2,4-Me₂-Ph,Cl,H,H], [D443;2,4-Me₂-Ph,H,Cl,H], [D444;2,4-Me₂-Ph,H,H,Cl], [D445;2,4-Me₂-Ph,Me,H,H], [D446;2,4-Me₂-Ph,H,Me,H], [D447;2,4-Me₂-Ph,H,H,Me], [D448;2,4-Me₂-Ph,F,F,H], [D449;2,4-Me₂-Ph,F,H,F], [D450;2,4-Me₂-Ph,H,F,F], [D451;2,4-Me₂-Ph,Cl,Cl,H], [D452;2,4-Me₂-Ph,Cl,H,Cl], [D453;2,4-Me₂-Ph,H,Cl,Cl], [D454;2,4-Me₂-Ph,Me,Me,H], [D455;2,4-Me₂-Ph,Me,H,Me], [D456;2,4-Me₂-Ph,H,Me,Me], [D457;2,5-Me₂-Ph,H,H,H], [D458;2,5-Me₂-Ph,F,H,H], [D459;2,5-Me₂-Ph,H,F,H], [D460;2,5-Me₂-Ph,H,H,F], [D461;2,5-Me₂-Ph,Cl,H,H], [D462;2,5-Me₂-Ph,H,Cl,H], [D463;2,5-Me₂-Ph,H,H,Cl], [D464;2,5-Me₂-Ph,Me,H,H], [D465;2,5-Me₂-Ph,H,Me,H], [D466;2,5-Me₂-Ph,H,H,Me], [D467;2,5-Me₂-Ph,F,F,H], [D468;2,5-Me₂-Ph,F,H,F], [D469;2,5-Me₂-Ph,H,F,F], [D470;2,5-Me₂-Ph,Cl,Cl,H], [D47l;2,5-Me₂-Ph,Cl,H,Cl], [D472;2,5-Me₂-Ph,H,Cl,Cl], [D473;2,5-Me₂-Ph,Me,Me,H], [D474;2,5-Me₂-Ph,Me,H,Me], [D475;2,5-Me₂-Ph,H,Me,Me], [D476;2,6-Me₂-Ph,H,H,H], [D477;2,6-Me₂-Ph,F,H,H], [D478;2,6-Me₂-Ph,H,F,H], [D479;2,6-Me₂-Ph,H,H,F], [D480;2,6-Me₂-Ph,Cl,H,H], [D481;2,6-Me₂-Ph,H,Cl,H], [D482;2,6-Me₂-Ph,H,H,Cl], [D483;2,6-Me₂-Ph,Me,H,H], [D484;2,6-Me₂-Ph,H,Me,H], [D485;2,6-Me₂-Ph,H,H,Me], [D486;2,6-Me₂-Ph,F,F,H], [D487;2,6-Me₂-Ph,F,H,F], [D488;2,6-Me₂-Ph,H,F,F], [D489;2,6-Me₂-Ph,Cl,Cl,H], [D490;2,6-Me₂-Ph,Cl,H,Cl], [D491;2,6-Me₂-Ph,H,Cl,Cl], [D492;2,6-Me₂-Ph,Me,Me,H], [D493;2,6-Me₂-Ph,Me,H,Me], [D494;2,6-Me₂-Ph,H,Me,Me], [D495;3,4-Me₂-Ph,H,H,H], [D496;3,4-Me₂-Ph,F,H,H], [D497;3,4-Me₂-Ph,H,F,H], [D498;3,4-Me₂-Ph,H,H,F], [D499;3,4-Me₂-Ph,Cl,H,H], [D500;3,4-Me₂-Ph,H,Cl,H], [D501;3,4-Me₂-Ph,H,H,Cl], [D502;3,4-Me₂-Ph,Me,H,H], [D503;3,4-Me₂-Ph,H,Me,H], [D504;3,4-Me₂-Ph,H,H,Me], [D505;3,4-Me₂-Ph,F,F,H], [D506;3,4-Me₂-Ph,F,H,F], [D507;3,4-Me₂-Ph,H,F,F], [D508;3,4-Me₂-Ph,Cl,Cl,H], [D509;3,4-Me₂-Ph,Cl,H,Cl], [D510;3,4-Me₂-Ph,H,Cl,Cl], [D511;3,4-Me₂-Ph,Me,Me,H], [D512;3,4-Me₂-Ph,Me,H,Me], [D513;3,4-Me₂-Ph,H,Me,Me], [D514;3,5-Me₂-Ph,H,H,H], [D515;3,5-Me₂-Ph,F,H,H], [D516;3,5-Me₂-Ph,H,F,H], [D517;3,5-Me₂-Ph,H,H,F], [D518;3,5-Me₂-Ph,Cl,H,H], [D519;3,3-Me₂-Ph,H,Cl,H], [D520;3,5-Me₂-Ph,H,H,Cl], [D521;3,5-Me₂-Ph,Me,H,H], [D522;3,5-Me₂-Ph,H,Me,H], [D523;3,5-Me₂-Ph,H,H,Me], [D524;3,5-Me₂-Ph,F,F,H], [D525;3,5-Me₂-Ph,F,H,F], [D526;3,5-Me₂-Ph,H,F,F], [D527;3,5-Me₂-Ph,Cl,Cl,H], [D528;3,5-Me₂-Ph,Cl,H,Cl], [D529;3,5-Me₂-Ph,H,Cl,Cl], [D530;3,5-Me₂-Ph,Me,Me,H], [D531;3,5-Me₂-Ph,Me,H,Me], [D532;3,5-Me₂-Ph,H,Me,Me]

The Compound (A-4) wherein n represents 1, and the combination of Z, R^{8X}, R^{9X}, and R^{10X} represents any one combination indicated in the Combination D (hereinafter referred to as "Compound group SX14").

The Compound (A-4) wherein n represents 2, and the combination of Z, R^{8X}, R^{9X}, and R^{10X} represents any one combination indicated in the Combination D (hereinafter referred to as "Compound group SX15").

A compound represented by formula (A-5) 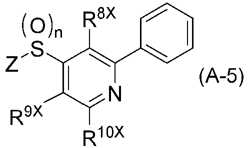 (hereinafter referred to as "Compound (A-5)") wherein n represents 0, and the combination of Z, R^{8X}, R^{9X}, and R^{10X} represents any one combination indicated in the Combination B (hereinafter referred to as "Compound group SX16").

The Compound (A-5) wherein n represents 1, and the combination of Z, R^{8X}, R^{9X}, and R^{10X} represents any one combination indicated in the Combination B (hereinafter referred to as "Compound group SX17").

The Compound (A-5) wherein n represents 2, and the combination of Z, R^{8X}, R^{9X}, and R^{10X} represents any one combination indicated in the Combination B (hereinafter referred to as "Compound group SX18").

A compound represented by formula (A-6) 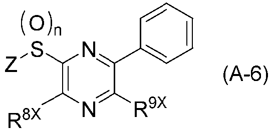 (hereinafter referred to as "Compound (A-6)") wherein n represents 0, and the combination of Z, R^{8X}, and R^{9X} represents any one combination indicated in the Combination C (hereinafter referred to as "Compound group SX19").

Combination C consists of Substituent Numbers C1 to C450. Substituent Numbers C1 to C450 indicate the combinations of Z, R^{8X}, and R^{9X} in the Compound (A-6), and are hereinafter referred to as [Substituent Number; 2,R^{8X},R^{9X}].

For example, Substituent Number C5 indicates a combination wherein Z represents an ethyl group, R^{8X} represents a hydrogen atom, and R^{9X} represents a chlorine atom.

Combination C: [C1;Et,H,H], [C2;Et,F,H], [C3;Et,H,F], [C4;Et,Cl,H], [C5;Et,H,Cl], [C6;Et,Me,H], [C7;Et,H,Me], [C8;Et,F,F], [C9;Et,Cl,Cl], [C10;Et,Me,Me], [C11;Pr,H,H], [C12;Pr,F,H], [C13;Pr,H,F], [C14;Pr,Cl,H], [C15;Pr,H,Cl], [C16;Pr,Me,H], [C17;Pr,H,Me], [C18;Pr,F,F], [C19;Pr,Cl,Cl], [C20;Pr,Me,Me], [C21;i-Pr,H,H], [C22;i-Pr,F,H], [C23;i-Pr,H,F], [C24;i-Pr,Cl,H], [C25;i-Pr,H,Cl], [C26;i-Pr,Me,H], [C27;i-Pr,H,Me], [C28;i-Pr,F,F], [C29;i-Pr,Cl,Cl], [C30;i-Pr,Me,Me], [C31;CH=CHCH₃,H,H][C32; CH=CHCH₃,F,H], [C33; CH=CHCH₃,H,F], [C34; CH=CHCH₃,Cl,H], [C35; CH=CHCH₃,H,Cl], [C36; CH=CHCH₃,Me,H], [C37; CH=CHCH₃,H,Me], [C38; CH=CHCH₃,F,F], [C39; CH=CHCH₃,Cl,Cl], [C40; CH=CHCH₃,Me,Me], [C41;CH₂CH=CH₂,H,H], [C42; CH₂CH=CH₂,F,H], [C43; CH₂CH=CH₂,H,F], [C44; CH₂CH=CH₂,Cl,H], [C45; CH₂CH=CH₂,H,Cl], [C46; CH₂CH=CH₂,Me,H], [C47; CH₂CH=CH₂,H,Me], [C48; CH₂CH=CH₂,F,F], [C49; CH₂CH=CH₂,Cl,Cl], [C50; CH₂CH=CH₂,Me,Me], [C51;Bu,H,H], [C52;Bu,F,H], [C53;Bu,H,F], [C54;Bu,C1,H], [C55;Bu,H,Cl], [C56;Bu,Me,H], [C57;Bu,H,Me], [C58;Bu,F,F], [C59;Bu,Cl,Cl], [C60;Bu,Me,Me], [C61;c-Pr,H,H], [C62;c-Pr,F,H], [C63;c-Pr,H,F], [C64;c-Pr,Cl,H], [C65;c-Pr,H,Cl], [C66;c-Pr,Me,H], [C67;c-Pr,H,Me], [C68;c-Pr,F,F], [C69;c-Pr,Cl,Cl], [C70;c-Pr,Me,Me], [C71;2-Thie,H,H], [C72;2-Thie,F,H], [C73;2-Thie,H,F], [C74;2-Thie,Cl,H], [C75;2-Thie,H,Cl], [C76;2-Thie,Me,H], [C77;2-Thie,H,Me], [C78;2-Thie,F,F], [C79;2-Thie,Cl,Cl], [C80;2-Thie,Me,Me], [C81;3-Thie,H,H], [C82;3-Thie,F,H], [C83;3-Thie,H,F], [C84;3-Thie,Cl,H], [C85;3-Thie,H,Cl], [C86;3-Thie,Me,H], [C87;3-Thie,H,Me], [C88;3-Thie,F,F], [C89;3-Thie,Cl,Cl], [C90;3-Thie,Me,Me], [C91;2-Fura,H,H], [C92;2-Fura,F,H], [C93;2-Fura,H,F], [C94;2-Fura,Cl,H], [C95;2-Fura,H,Cl], [C96;2-Fura,Me,H], [C97;2-Fura,H,Me], [C98;2-Fura,F,F], [C99;2-Fura,Cl,Cl], [C100;2-Fura,Me,Me], [C101;3-Fura,H,HJ, [C102;3-Fura,F,H], [C103;3-Fura,H,F], [C104;3-Fura,Cl,H], [C105;3-Fura,H,Cl], [C106;3-Fura,Me,H], [C107;3-Fura,H,Me], [C108;3-Fura,F,F], [C109;3-Fura,Cl,Cl], [C110;3-Fura,Me,Me], [C111;2-Py,H,H], [C112;2-Py,F,H], [C113;2-Py,H,F], [C114;2-Py,Cl,H], [C115;2-Py,H,Cl], [C116;2-Py,Me,H], [C117;2-Py,H,Me], [C118;2-Py,F,F], [C119;2-Py,Cl,Cl], [C120;2-Py,Me,Me], [C121;3-Py,H,H], [C122;3-Py,F,H], [C123;3-Py,H,F], [C124;3-Py,Cl,H], [C125;3-Py,H,Cl], [C126;3-Py,Me,H], [C127;3-Py,H,Me], [C128;3-Py,F,F], [C129;3-Py,Cl,Cl], [C130;3-Py,Me,Me], [C131;4-Py,H,H], [C132;4-Py,F,H], [C133;4-Py,H,F], [C134;4-Py,Cl,H], [C135;4-Py,H,Cl], [C136;4-Py,Me,H], [C137;4-Py,H,Me], [C138;4-Py,F,F], [C139;4-Py,Cl,Cl], [C140;4-Py,Me,Me], [C141;2-Pyrimid,H,H], [C142;2-Pyrimid,F,H], [C143;2-Pyrimid,H,F], [C144;2-Pyrimid,Cl,H], [C145;2-Pyrimid,H,Cl], [C146;2-Pyrimid,Me,H], [C147;2-Pyrimid,H,Me], [C148;2-Pyrimid,F,F], [C149;2-Pyrimid,Cl,Cl], [C150;2-Pyrimid,Me,Me], [C151;4-Pyrimid,H,H], [C152;4-Pyrimid,F,H], [C153;4-Pyrimid,H,F], [C154;4-Pyrimid,Cl,H], [C155;4-Pyrimid,H,Cl], [C156;4-Pyrimid,Me,H], [C157;4-Pyrimid,H,Me], [C158;4-Pyrimid,F,F], [C159;4-Pyrimid,Cl,Cl], [C160;4-Pyrimid,Me,Me], [C161;2-Pyrazin,H,H], [C162;2-Pyrazin,F,H], [C163;2-Pyrazin,H,F], [C164;2-Pyrazin,Cl,H], [C165;2-Pyrazin,H,Cl], [C166;2-Pyrazin,Me,H], [C167;2-Pyrazin,H,Me], [C168;2-Pyrazin,F,F], [C169;2-Pyrazin,Cl,Cl], [C170;2-Pyrazin,Me,Me] , [C171;Ph,H,H], [C172;Ph,F,H], [C173;Ph,H,F], [C174;Ph,Cl,H], [C175;Ph,H,Cl], [C176;Ph,Me,H], [C177;Ph,H,Me], [C178;Ph,F,F], [C179;Ph,Cl,Cl], [C180;Ph,Me,Me], [C181;2-F-Ph,H,H], [C182;2-F-Ph,F,H], [C183;2-F-Ph,H,F], [C184;2-F-Ph,Cl,H], [C185;2-F-Ph,H,Cl], [C186;2-F-Ph,Me,H], [C187;2-F-Ph,H,Me], [C188;2-F-Ph,F,F], [C189;2-F-Ph,Cl,Cl], [C190;2-F-Ph,Me,Me], [C191;3-F-Ph,H,H], [C192;3-F-Ph,F,H], [C193;3-F-Ph,H,F], [C194;3-F-Ph,Cl,H], [C195;3-F-Ph,H,Cl], [C196;3-F-Ph,Me,H], [C197;3-F-Ph,H,Me], [C198;3-F-Ph,F,F], [C199;3-F-Ph,Cl,Cl], [C200;3-F-Ph,Me,Me], [C201;4-F-Ph,H,H], [C202;4-F-Ph,F,H], [C203;4-F-Ph,H,F], [C204;4-F-Ph,Cl,H], [C205;4-F-Ph,H,Cl], [C206;4-F-Ph,Me,H], [C207;4-F-Ph,H,Me], [C208;4-F-Ph,F,F], [C209;4-F-Ph,Cl,Cl], [C210;4-F-Ph,Me,Me], [C211;2-Cl-Ph,H,H], [C212;2-Cl-Ph,F,H], [C213;2-Cl-Ph,H,F], [C214;2-Cl-Ph,Cl,H], [C215;2-Cl-Ph,H,Cl], [C216;2-Cl-Ph,Me,H], [C217;2-Cl-Ph,H,Me], [C218;2-Cl-Ph,F,F], [C219;2-Cl-Ph,Cl,Cl], [C220;2-Cl-Ph,Me,Me], [C221;2-F-Ph,H,H], [C222;3-Cl-Ph,F,H], [C223;3-Cl-Ph,H,F], [C224;3-Cl-Ph,Cl,H], [C225;3-Cl-Ph,H,Cl], [C226;3-Cl-Ph,Me,H], [C227;3-Cl-Ph,H,Me], [C228;3-Cl-Ph,F,F], [C229;3-Cl-Ph,Cl,Cl], [C230;3-Cl-Ph,Me,Me], [C231;4-Cl-Ph,H,H], [C232;4-Cl-Ph,F,H], [C233;4-Cl-Ph,H,F], [C234;4-Cl-Ph,Cl,H], [C235;4-Cl-Ph,H,Cl], [C236;4-Cl-Ph,Me,H], [C237;4-Cl-Ph,H,Me], [C238;4-Cl-Ph,F,F], [C239;4-Cl-Ph,Cl,Cl], [C240;4-Cl-Ph,Me,Me], [C241;2-Me-Ph,H,H], [C242;2-Me-Ph,F,H], [C243;2-Me-Ph,H,F], [C244;2-Me-Ph,Cl,H], [C245;2-Me-Ph,H,Cl], [C246;2-Me-Ph,Me,H], [C247;2-Me-Ph,H,Me], [C248;2-Me-Ph,F,F], [C249;2-Me-Ph,Cl,Cl], [C250;2-Me-Ph,Me,Me], [C251;3-Me-Ph,H,H], [C252;3-Me-Ph,F,H], [C253;3-Me-Ph,H,F], [C254;3-Me-Ph,Cl,H], [C255;3-Me-Ph,H,Cl], [C256;3-Me-Ph,Me,H], [C257;3-Me-Ph,H,Me], [C258;3-Me-Ph,F,F], [C259;3-Me-Ph,Cl,Cl], [C260;3-Me-Ph,Me,Me], [C261;4-Me-Ph,H,H], [C262;4-Me-Ph,F,H], [C263;4-Me-Ph,H,F], [C264;4-Me-Ph,Cl,H], [C265;4-Me-Ph,H,Cl], [C266;4-Me-Ph,Me,H], [C267;4-Me-Ph,H,Me], [C268;4-Me-Ph,F,F], [C269;4-Me-Ph,Cl,Cl], [C270;4-Me-Ph,Me,Me], [C271;2-OMe-Ph,H,H], [C272;2-OMe-Ph,F,H], [C273;2-OMe-Ph,H,F], [C274;2-OMe-Ph,Cl,H], [C275;2-OMe-Ph,H,Cl], [C276;2-OMe-Ph,Me,H], [C277;2-OMe-Ph,H,Me], [C278;2-OMe-Ph,F,F], [C279;2-OMe-Ph,Cl,Cl], [C280;2-OMe-Ph,Me,Me], [C281;3-OMe-Ph,H,H], [C282;3-OMe-Ph,F,H], [C283;3-OMe-Ph,H,F], [C284;3-OMe-Ph,Cl,H], [C285;3-OMe-Ph,H,Cl], [C286;3-OMe-Ph,Me,H], [C287;3-OMe-Ph,H,Me], [C288;3-OMe-Ph,F,F], [C289;3-OMe-Ph,Cl,Cl], [C290;3-OMe-Ph,Me,Me], [C291;4-OMe-Ph,H,H], [C292;4-OMe-Ph,F,H], [C293;4-OMe-Ph,H,F], [C294;4-OMe-Ph,Cl,H], [C295;4-OMe-Ph,H,Cl], [C296;4-OMe-Ph,Me,H], [C297;4-OMe-Ph,H,Me], [C298;4-OMe-Ph,F,F], [C299;4-OMe-Ph,Cl,Cl], [C300;4-OMe-Ph,Me,Me], [C301;2-CN-Ph,H,H], [C302;2-CN-Ph,F,H], [C303;2-CN-Ph,H,F], [C304;2-CN-Ph,Cl,H], [C305;2-CN-Ph,H,Cl], [C306;2-CN-Ph,Me,H], [C307;2-CN-Ph,H,Me], [C308;2-CN-Ph,F,F], [C309;2-CN-Ph,Cl,Cl], [C310;2-CN-Ph,Me,Me], [C311;3-CN-Ph,H,H], [C312;3-CN-Ph,F,H], [C313;3-CN-Ph,H,F], [C314;3-CN-Ph,Cl,H], [C315;3-CN-Ph,H,Cl], [C316;3-CN-Ph,Me,H], [C317;3-CN-Ph,H,Me], [C318;3-CN-Ph,F,F], [C319;3-CN-Ph,Cl,Cl], [C320;3-CN-Ph,Me,Me], [C321;4-CN-Ph,H,H], [C322;4-CN-Ph,F,H], [C323;4-CN-Ph,H,F], [C324;4-CN-Ph,Cl,H], [C325;4-CN-Ph,H,Cl], [C326;4-CN-Ph,Me,H], [C327;4-CN-Ph,H,Me], [C328;4-CN-Ph,F,F], [C329;4-CN-Ph,Cl,Cl], [C330;4-CN-Ph,Me,Me], [C331;2,3-F₂-Ph,H,H], [C332;2,3-F₂-Ph,F,H], [C333;2,3-F₂-Ph,H,F], [C334;2,3-F₂-Ph,Cl,H], [C335;2,3-F₂-Ph,H,Cl], [C336;2,3-F₂-Ph,Me,H], [C337;2,3-F₂-Ph,H,Me], [C338;2,3-F₂-Ph,F,F], [C339;2,3-F₂-Ph,Cl,Cl], [C340;2,3-F₂-Ph,Me,Me], [C341;2,4-F₂-Ph,H,H], [C342;2,4-F₂-Ph,F,H], [C343;2,4-F₂-Ph,H,F], [C344;2,4-F₂-Ph,Cl,H], [C345;2,4-F₂-Ph,H,Cl], [C346;2,4-F₂-Ph,Me,H], [C347;2,4-F₂-Ph,H,Me], [C348;2,4-F₂-Ph,F,F], [C349;2,4-F₂-Ph,Cl,Cl], [C350;2,4-F₂-Ph,Me,Me], [C351;2,5-F₂-Ph,H,H], [C352;2,5-F₂-Ph,F,H], [C353;2,5-F₂-Ph,H,F], [C354;2,5-F₂-Ph,Cl,H], [C355;2,5-F₂-Ph,H,Cl], [C356;2,5-F₂-Ph,Me,H], [C357;2,5-F₂-Ph,H,Me], [C358;2,5-F₂-Ph,F,F], [C359;2,5-F₂-Ph,Cl,Cl], [C360;2,5-F₂-Ph,Me,Me], [C361;2,6-F₂-Ph,H,H], [C362;2,6-F₂-Ph,F,H], [C363;2,6-F₂-Ph,H,F], [C364;2,6-F2-Ph,Cl,H], [C365;2,6-F₂-Ph,H,Cl], [C366;2,6-F₂-Ph,Me,H], [C367;2,6-F₂-Ph,H,Me], [C368;2,6-F₂-Ph,F,F], [C369;2,6-F₂-Ph,Cl,Cl], [C370;2,6-F₂-Ph,Me,Me], [C371;3,4-F₂-Ph,H,H], [C372;3,4-F₂-Ph,F,H], [C373;3,4-F₂-Ph,H,F], [C374;3,4-F₂-Ph,Cl,H], [C375;3,4-F₂-Ph,H,Cl], [C376;3,4-F₂-Ph,Me,H], [C377;3,4-F₂-Ph,H,Me], [C378;3,4-F₂-Ph,F,F], [C379;3,4-F₂-Ph,Cl,Cl], [C380;3,4-F₂-Ph,Me,Me], [C381;3,5-F₂-Ph,H,H], [C382;3,5-F₂-Ph,F,H], [C383;3,5-F₂-Ph,H,F], [C384;3,5-F₂-Ph,Cl,H], [C385;3,5-F₂-Ph,H,Cl], [C386;3,5-F₂-Ph,Me,H], [C387;3,5-F₂-Ph,H,Me], [C388;3,5-F₂-Ph,F,F], [C389;3,5-F₂-Ph,Cl,Cl], [C390;3,5-F₂-Ph,Me,Me], [C391;2,3-Me₂-Ph,H,H], [C392;2,3-Me₂-Ph,F,H], [C393;2,3-Me₂-Ph,H,F], [C394;2,3-Me₂-Ph,Cl,H], [C395;2,3-Me₂-Ph,H,Cl], [C396;2,3-Me₂-Ph, Me, H], [C397;2,3-Me₂-Ph,H,Me], [C398;2,3-Me₂-Ph,F,F], [C399;2,3-Me₂-Ph,Cl,Cl], [C400;2,3-Me₂-Ph,Me,Me], [C401;2,4-Me₂-Ph,H,H], [C402;2,4-Me₂-Ph,F,H], [C403;2,4-Me₂-Ph,H,F], [C404;2,4-Me₂-Ph,Cl,HJ, [C405; 2, 4-Mez -Ph, H, Cl] , [C406;2,4-Me₂-Ph,Me,H], [C407;2,4-Me₂-Ph,H,Me], [C408;2,4-Me₂-Ph,F,F], [C409;2,4-Me₂-Ph,C1,Cl], [C410;2,4-Me₂-Ph,Me,Me], [C411;2,5-Me₂-Ph,H,H], [C412;2,5-Me₂-Ph,F,H], [C413;2,5-Me₂-Ph,H,F], [C414;2,5-Me₂-Ph,C1,H], [C415;2,5-Me₂-Ph,H,Cl], [C416;2,5-Me₂-Ph,Me,H], [C417;2,5-Me₂-Ph,H,Me], [C418;2,5-Me₂-Ph,F,F], [C419;2,5-Me₂-Ph,C1,Cl], [C420;2,5-Me₂-Ph,Me,Me], [C421;2,6-Me₂-Ph,H,H], [C422;2,6-Me₂-Ph,F,H], [C423;2,6-Me₂-Ph,H,F], [C424;2,6-Me₂-Ph,C1,H], [C425;2,6-Me₂-Ph,H,Cl], [C426;2,6-Me₂-Ph,Me,H], [C427;2,6-Me₂-Ph,H,Me], [C428;2,6-Me₂-Ph,F,F], [C429;2,6-Me₂-Ph, Cl, Cl J, [C430;2,6-Me₂-Ph,Me,Me], [C431;3,4-Me₂-Ph,H,H], [C432;3,4-Me₂-Ph,F,H], [C433;3,4-Me₂-Ph,H,F], [C434;3,4-Me₂ -Ph, Cl, H], [C43S; 3,4-Me₂ -Ph, H, ClJ, [C436;3,4-Me₂-Ph,Me,H], [C437;3,4-Me₂-Ph,H,Me], [C438;3,4-Me₂-Ph,F,F], [C439;3,4-Me₂-Ph,Cl,Cl], [C440;3,4-Me₂-Ph,Me,Me], [C441;3,5-Me₂-Ph,H,H], [C442;3,5-Me₂-Ph,F,H], [C443;3,5-Me₂-Ph,H,F], [C444;3,5-Me₂-Ph,Cl,H], [C445;3,5-Me₂-Ph,H,Cl], [C446;3,5-Me₂ -Ph,Me,H], [C447;3,5-Me₂-Ph,H,Me], [C448;3,5-Me₂-Ph,F,F], [C449;3,5-Me₂-Ph,C1,Cl], [C450;3,5-Me₂-Ph,Me,Me]

The Compound (A-6) wherein n represents 1, and the combination of Z, R^{8X}, and R^{9X} represents any one combination indicated in the Combination C (hereinafter referred to as "Compound group SX20").

The Compound (A-6) wherein n represents 2, and the combination of Z, R^{8X}, and R^{9X} represents any one combination indicated in the Combination C (hereinafter referred to as "Compound group SX21").

Next, Formulation Examples are shown below. The "part(s)" represents "part(s) by weight". Also, the expression of "Present compound S" represents the compounds described in the Compound groups SX1 to SX21.

### Formulation Example 1

A mixture of polyoxyethylene alkyl ether sulfate ammonium salt and silica (weight ratio of 1 : 1) (35 parts), any one of the Present compound S (10 parts), and water (55 parts) are mixed, and the resulting mixture is subjected to fine grinding according to a wet grinding method to obtain each formulation.

### Formulation Example 2

Any one of the Present compound S (50 parts), calcium lignin sulfonate (3 parts), sodium lauryl sulfate (2 parts), and silica (45 parts) are ground and mixed to obtain each formulation.

### Formulation Example 3

Any one of the Present compound S (5 parts), polyoxyethylene styryl phenyl ether (9 parts), polyoxyethylene decyl ether (number of added ethyleneoxide: 5) (5 parts), calcium dodecylbenzene sulfonate (6 parts), and xylene (75 parts) are mixed to obtain each formulation.

### Formulation Example 4

Any one of the Present compound S (2 parts), silica (1 part), calcium lignin sulfonate (2 parts), bentonite (30 parts), and kaolin clay (65 parts) are ground and mixed, an appropriate amount of water is added thereto, the resulting mixture is kneaded, subjected to granulation with a granulator, and then dried to obtain each formulation.

### Formulation Example 5

Any one of the Present compound S (10 parts), and a mixture of benzyl alcohol (18 parts) and DMSO (9 parts) are mixed, GERONOL (registered trademark) TE250 (6.3 parts), Ethylan (registered trademark) NS-500LQ (2.7 parts), and solvent naphtha (54 parts) are added thereto, and the resulting mixture is mixed to obtain each formulation.

Next, Test Examples are shown below.

The term of "non-treated well" in Test Example 15 represents a well wherein a test was carried out under the same conditions as those described in the Test Example except for dispensing DMSO instead of a diluted solution with DMSO comprising the Present compound. Also, the term of "non-treated leaf disk" in Test Example 7 represents a leaf disk wherein a test was carried out under the same conditions as those described in the Test Example except for dispensing DMSO instead of a diluted solution with DMSO comprising the Present compound. Also, the term of "non-treated" in Test Example 11 means that a diluted solution with water of a formulation comprising the Present compound was not sprayed.

### Test Example 1 Control test against wheat leaf blotch fungus (Septoria tritici)

Each Present compound is diluted with DMSO to obtain a diluted solution containing 150 ppm of the compound, 1 pL of the diluted solution is dispensed into a titer plate (96 well), and thereto is then dispensed 150 pL of a YBG medium to which spores of Septoria tritici are inoculated in advance. This plate is cultured at 18°C for 3 days, thereby allowing Septoria tritici to undergo proliferation, and the absorbance at 550 nm of each well of the titer plate is then measured to determine a degree of growth of Septoria tritici. As a result, each Present compound shows a control effect against the plant disease.

### Test Example 2 Control test against corn smut fungus (Ustilago maydis)

Each Present compound is diluted with DMSO to obtain a diluted solution containing 150 ppm of the compound, 1 pL of the diluted solution is dispensed into a titer plate (96 well), and thereto is then dispensed 150 µL of a potato dextrose broth medium (PDB medium) to which spores of Ustilago maydis are inoculated in advance. This plate is cultured at 18°C for 4 days, thereby allowing Ustilago maydis to undergo proliferation, and the absorbance at 550 nm of each well of the titer plate is then measured to determine a degree of growth of Ustilago maydis. As a result, each Present compound shows a control effect against the plant disease.

### Test Example 3 Control test against barley scald fungus (Rhynchosporium secalis)

Each Present compound is diluted with DMSO to obtain a diluted solution containing 150 ppm of the compound, 1 µL of the diluted solution is dispensed into a titer plate (96 well), and thereto is then dispensed 150 µL of a potato dextrose broth medium (PDB medium) to which spores of Rhynchosporium secalis are inoculated in advance. This plate is cultured at 18°C for 7 days, thereby allowing Rhynchosporium secalis to undergo proliferation, and the absorbance at 550 nm of each well of the titer plate is then measured to determine a degree of growth of Rhynchosporium secalis. As a result, each Present compound shows a control effect against the plant disease.

### Test Example 4 Control test against cucumber Botrytis rot fungus (Botrytis cinerea)

Each Present compound is diluted with DMSO to obtain a diluted solution containing 150 ppm of the compound, 1 µL of the diluted solution is dispensed into a titer plate (96 well), and thereto is then dispensed 150 µL of a complete medium to which spores of Botrytis cinerea are inoculated in advance. This plate is cultured at 18°C for 4 days, thereby allowing Botrytis cinerea to undergo proliferation, and the absorbance at 550 nm of each well of the titer plate is then measured to determine a degree of growth of Botrytis cinerea. As a result, each Present compound shows a control effect against the plant disease.

### Test Example 5 Control test against peach scab fungus (Cladosporium carpophilum)

Each Present compound is diluted with DMSO to obtain a diluted solution containing 150 ppm of the compound, 1 µL of the diluted solution is dispensed into a titer plate (96 well), and thereto is then dispensed 150 µL of a potato dextrose broth medium (PDB medium) to which spores of Cladosporium carpophilum are inoculated in advance. This plate is cultured at 18°C for 5 days, thereby allowing Cladosporium carpophilum to undergo proliferation, and the absorbance at 550 nm of each well of the titer plate is then measured to determine a degree of growth of Cladosporium carpophilum. As a result, each Present compound shows a control effect against the plant disease.

### Test Example 6 Control test against rice brown spot fungus (Cochliobolus miyabeanus)

Each Present compound is diluted with DMSO to obtain a diluted solution containing 150 ppm of the compound, 1 µL of the diluted solution is dispensed into a titer plate (96 well), and thereto is then dispensed 150 pL of a YB liquid medium to which spores of Cochliobolus miyabeanus are inoculated in advance. This plate is cultured at 23°C for 3 days, thereby allowing Cochliobolus miyabeanus to undergo proliferation, and the absorbance at 550 nm of each well of the titer plate is then measured to determine a degree of growth of Cochliobolus miyabeanus. As a result, each Present compound shows a control effect against the plant disease.

### Test Example 7 Control test against soybean rust (Phakopsora pachyrhizi)

A true leaf of a soybean (cultivar: Kurosengoku) was cut into a diameter of 1 cm to prepare each leaf disk. To each well of a 24 well microplate was dispensed 1 mL of an agar medium (agar concentration: 1.2%), and then one of said leaf disk was placed on the agar medium in each well. To a mixture of Sorpol (registered trademark) 1200KX (0.5 pL), DMSO (4.5 pL), and xylene (5 µL) was added a DMSO solution (20 µL) comprising a test compound (10000 ppm), and the resulting mixture was mixed. The resulting mixture was diluted with ion exchange water to prepare a mixture comprising a prescribed concentration of the test compound. The resulting mixture was sprayed into each leaf disk at a ratio of 10 pL per leaf disk. After 1 day, an aqueous suspension of spores of soybean rust fungus (Phakopsora pachyrhizi) having an amino acid substitution of F129L in a mitochondrial cytochrome b protein (1.0 × 10⁵ / mL) was inoculated by spraying on each leaf disk. After the inoculation, the microplate was placed into an artificial climate chamber (Lighting: 18 hours, Lights-out: 6 hours, Temperature: 23°C, Humidity: 60%). After 1 day, the leaf disks were air-dried until water droplets on the surfaces of the leaf disks disappeared, and the microplate was placed into the artificial climate chamber again for 12 days. Then, lesion area of soybean rust was investigated. As a result, when the prescribed concentration was 200 ppm, each lesion area of the leaf disk treated with any one of the Present compound 1, 2, 4, 5, 6, 8, 9, 12, 13, 15, 16, 18, 20, 25, 27, 28, 29, 30, 31, or 32 as the test compound was 30% or less relative to the lesion area of the non-treated leaf disk.

### Test Example 8 Control test against barley net blotch (Pyrenophora teres)

A plastic pot is filled with soil, barley (cultivar: Nishinohoshi) is seeded thereto, and grown in a greenhouse for 7 days. The Present compound formulated according to the method described in the Formulation Example 1 is mixed with water in such a way that the concentration thereof is 200 ppm, and the resulting mixture is sprayed to foliage of the above barley so as to adequately adhere onto the surfaces of leaves of the above barley. After spraying the mixture, the barley is air-dried. After 1 day, an aqueous suspension of spores of Pyrenophora teres is inoculated by spraying to the barley. After the inoculation, the barley is placed in a greenhouse at 23°C in daytime and at 20°C in nighttime under a humid condition for 3 days, then cultured in a greenhouse for 7 days, and then a lesion area is investigated. As a result, each Present compound shows a control effect against the plant disease.

### Test Example 9 Control test against wheat leaf rust (Puccinia recondita)

A plastic pot is filled with soil, wheat (cultivar: Shirogane) is seeded thereto, and grown in a greenhouse for 9 days. The Present compound formulated according to the method described in the Formulation Example 1 is mixed with water in such a way that the concentration thereof is 200 ppm, and the resulting mixture is sprayed to foliage of the above wheat so as to adequately adhere onto the surfaces of leaves of the above wheat. After spraying the mixture, the wheat is air-dried, cultured at 20°C under lighting for 5 to 7 days, and then spores of Puccinia recondita are inoculated by dusting thereto. After the inoculation, the wheat is placed at 23°C under a dark humid condition for 1 day, then cultured at 20°C under lighting for 8 days, and a lesion area is investigated. As a result, each Present compound shows a control effect against the plant disease.

### Test Example 10 Control test against wheat leaf blotch (Septoria tritici)

A plastic pot is filled with soil, wheat (cultivar: Apogee) is seeded thereto, and cultured in a greenhouse for 10 days. The Present compound formulated according to the method described in the Formulation Example 1 is mixed with water in such a way that the concentration thereof is 200 ppm, and the resulting mixture is sprayed to foliage of the above wheat so as to adequately adhere onto the surfaces of leaves of the above wheat. After spraying the mixture, the wheat is air-dried. After 4 days, an aqueous suspension comprising spores of Septoria tritici is inoculated by spraying to the wheat. After the inoculation, the wheat is placed at 18°C under a humid condition for 3 days, then cultured under lighting for 14 to 18 days, and then a lesion area is investigated. As a result, each Present compound shows a control effect against the plant disease.

### Test Example 11 Control test against soybean rust (Phakopsora pachyrhizi)

A plastic pot was filled with soil, soybeans (cultivar: Kurosengoku) were seeded thereto, and cultured in a greenhouse for 10 to 14 days. The Present compound 1, 2, 4, 5, 6, 8, 9, 10, 11, or 32 formulated according to the method described in the Formulation Example 1 was mixed with water in such a way that the concentration thereof was 200 ppm, and the resulting mixture was sprayed to foliage of the above soybeans so as to adequately adhere onto the surfaces of leaves of the above soybeans. After spraying the mixture, the soybeans were air-dried. After 2 to 5 days, an aqueous suspension of spores of Phakopsora pachyrhizi was inoculated by spraying to the soybeans. After the inoculation, the soybeans were placed in a greenhouse at 23°C in daytime and at 20°C in nighttime under a humid condition for 1 to 2 day(s), then cultured in a greenhouse for 12 days, and then a lesion area was investigated. As a result, the lesion area in the soybeans treated with each of the above Present compound was 30% or less relative to the lesion area in the non-treated soybeans.

### Test Example 12 Control test against soybean rust (Phakopsora pachyrhizi)

A plastic pot is filled with soil, soybeans (cultivar: Kurosengoku) are seeded thereto, and cultured in a greenhouse for 10 days. An aqueous suspension comprising spores of Phakopsora pachyrhizi is inoculated by spraying to the soybeans. After the inoculation, the soybeans are placed in a greenhouse at 23°C in daytime and at 20°C in nighttime under a humid condition for 1 day, and then cultured in a greenhouse for 2 days. Then, the Present compound formulated according to the method described in the Formulation Example 1 is mixed with water in such a way that the concentration thereof is 200 ppm, and the resulting mixture is sprayed to foliage of the above soybeans so as to adequately adhere onto the surfaces of leaves of the above soybeans. After spraying the mixture, the soybeans are air-dried, then cultured in a greenhouse for 8 days, and then a lesion area is investigated. As a result, each Present compound shows a control effect against the plant disease.

### Test Example 13 Control test against soybean Cercospora leaf spot (Cercospora sojina)

A plastic pot is filled with soil, soybeans (cultivar: Tachinagaha) are seeded thereto, and cultured in a greenhouse for 13 days. The Present compound formulated according to the method described in the Formulation Example 1 is mixed with water in such a way that the concentration thereof is 200 ppm, and the resulting mixture is sprayed to foliage of the above soybeans so as to adequately adhere onto the surfaces of leaves of the above soybeans. After spraying the mixture, the soybeans are air-dried. After 1 day, an aqueous suspension of spores of Cercospora sojina is inoculated by spraying to the soybeans. After the inoculation, the soybeans are placed in a greenhouse at 23°C in daytime and at 20°C in nighttime under a humid condition for 3 days, then cultured in a greenhouse for 16 days, and then a lesion area is investigated. As a result, each Present compound shows a control effect against the plant disease.

### Test Example 14 Control test against tomato early blight (Alternaria solani)

A plastic pot is filled with soil, tomatoes (cultivar: Patio) are seeded thereto, and cultured in a greenhouse for 20 days. The Present compound formulated according to the method described in the Formulation Example 1 is mixed with water in such a way that the concentration thereof is 200 ppm, and the resulting mixture is sprayed to foliage of the above tomatoes so as to adequately adhere onto the surfaces of leaves of the above tomatoes. After spraying the mixture, the tomatoes are air-dried. After 1 day, an aqueous suspension of spores of Alternaria solani is inoculated by spraying to the tomatoes. After the inoculation, the tomatoes are placed at 18°C under a humid condition for 6 days, and then a lesion area is investigated. As a result, each Present compound shows a control effect against the plant disease.

### Test Example 15 Control test against soybean rust fungus (Phakopsora pachyrhizi)

Each Present compound was diluted with DMSO to obtain a diluted solution containing 150 ppm of the compound, 1 pL of the diluted solution was dispensed into a titer plate (96 well), and thereto was then dispensed 149 pL of an aqueous suspension of spores of Phakopsora pachyrhizi to which spores of Phakopsora pachyrhizi were suspended in advance (1.0 × 10⁴ / mL). This plate was cultured at 23°C for several hours, and then germinated spores of Phakopsora pachyrhizi were counted. As a result, when the treatment concentration was 1 ppm, the number of germinated spores in each well comprising any one of the Present compound 1, 2, 3, 4, 5, 6, 7, 8, 9, 12, 13, 14, 15, 16, 20, 21, 27, 28, 29, or 32 as the test compound was 50% or less relative to the number of germinated spores in the non-treated well.

### Test Example 16 Control test against soybean rust fungus (Phakopsora pachyrhizi)

A test is carried out by the method according to the Test Example 15 and using each Present compound as the test compound with the treatment concentration of 10 ppm. As a result, each Present compound shows a control effect against the plant disease.

### INDUSTRIAL APPLICABILITY

The Present compounds have control effects against plant diseases, and can be used in controlling plant diseases.

## Claims

1. A method for controlling a plant disease which comprises applying a compound represented by formula (I) 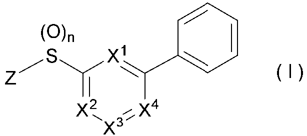
[wherein:
n represents 0, 1, or 2;
the combination of X³ and Z represents:
a combination wherein
X³ represents CR³, and
Z represents a C2-C6 chain hydrocarbon group, a methyl group substituted with one or more fluorine atom(s), a C1-C2 alkyl group {wherein said C1-C2 alkyl group is substituted with a methoxy group or an ethoxy group}, a C3-C8 alicyclic hydrocarbon group optionally substituted with one or more substituent(s) selected from Group A, a 3-8 membered nonaromatic heterocyclic group {wherein said 3-8 membered nonaromatic heterocyclic group is bound to the S(O)ₙ at a ring-constituting carbon atom, and optionally substituted with one or more substituent(s) selected from Group A}, or a 5-10 membered aromatic heterocyclic group {wherein said 5-10 membered aromatic heterocyclic group is bound to the S(O)ₙ at a ring-constituting carbon atom, and optionally substituted with one or more substituent(s) selected from Group B};
or, a combination wherein
X³ represents a nitrogen atom, and
Z represents a C2-C6 chain hydrocarbon group, a methyl group substituted with one or more fluorine atom(s), a C1-C2 alkyl group {wherein said C1-C2 alkyl group is substituted with a methoxy group or an ethoxy group}, a C3-C8 alicyclic hydrocarbon group optionally substituted with one or more substituent(s) selected from Group A, a 3-8 membered nonaromatic heterocyclic group {wherein said 3-8 membered nonaromatic heterocyclic group is bound to the S(O)ₙ at a ring-constituting carbon atom, and optionally substituted with one or more substituent(s) selected from Group A}, a phenyl group optionally substituted with one or more substituent(s) selected from Group B, or a 5-10 membered aromatic heterocyclic group {wherein said 5-10 membered aromatic heterocyclic group is bound to the S(O)ₙ at a ring-constituting carbon atom, and optionally substituted with one or more substituent(s) selected from Group B};
X¹ represents CR¹ or a nitrogen atom;
X² represents CR² or a nitrogen atom;
X⁴ represents CR⁴ or a nitrogen atom (provided that among X¹, X², X³, and X⁴, the number of nitrogen atom is 0, 1, or 2); and
R¹, R², R³, and R⁴ are identical to or different from each other, and each represent a methyl group optionally substituted with one or more fluorine atom(s), a halogen atom, or a hydrogen atom;
Group A: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group C}, an oxo group, a thioxo group, a hydroxy group, a halogen atom, and a cyano group;
Group B: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group C}, a halogen atom, and a cyano group;
Group C: a group consisting of a C1-C3 alkoxy group, a halogen atom, and a cyano group]
or an N-oxide thereof, or a salt thereof, to a plant or soil for cultivating a plant.

2. The method for controlling a plant disease according to claim 1, wherein the compound represented by formula (I) or an N-oxide thereof, or a salt thereof in claim 1 is a compound or an N-oxide thereof, or a salt thereof wherein
the combination of X³ and Z represents:
a combination wherein
X³ represents CH, and
Z represents a C2-C6 chain hydrocarbon group or a 5-10 membered aromatic heterocyclic group {wherein said 5-10 membered aromatic heterocyclic group is bound to the S(O)ₙ at a ring-constituting carbon atom, and optionally substituted with one or more substituent(s) selected from Group B};
or, a combination wherein
X³ represents a nitrogen atom, and
Z represents a C2-C6 chain hydrocarbon group, a C3-C8 alicyclic hydrocarbon group optionally substituted with one or more substituent(s) selected from Group A, a 3-8 membered nonaromatic heterocyclic group {wherein said 3-8 membered nonaromatic heterocyclic group is bound to the S(O)ₙ at a ring-constituting carbon atom, and optionally substituted with one or more substituent(s) selected from Group A}, a phenyl group optionally substituted with one or more substituent(s) selected from Group B, or a 5-10 membered aromatic heterocyclic group {wherein said 5-10 membered aromatic heterocyclic group is bound to the S(O)ₙ at a ring-constituting carbon atom, and optionally substituted with one or more substituent(s) selected from Group B};
X¹ represents CH or a nitrogen atom; and
X² and X⁴ each represent CH.

3. A compound represented by formula (II) 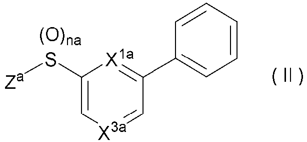 [wherein:
na represents 1 or 2;
X^{1a} represents CR^{1a} or a nitrogen atom;
the combination of X^{3a} and Z^{a} represents:
a combination wherein
X^{3a} represents CR^{3a}, and
Z² represents a C2-C6 chain hydrocarbon group, a C1-C2 alkyl group {wherein said C1-C2 alkyl group is substituted with a methoxy group or an ethoxy group}, a C3-C8 alicyclic hydrocarbon group optionally substituted with one or more substituent(s) selected from Group A^{a}, a 3-8 membered nonaromatic heterocyclic group {wherein said 3-8 membered nonaromatic heterocyclic group is bound to the S(O)ₙₐ at a ring-constituting carbon atom, and optionally substituted with one or more substituent(s) selected from Group A^{a}}, or a 5-10 membered aromatic heterocyclic group {wherein said 5-10 membered aromatic heterocyclic group is bound to the S(O)ₙₐ at a ring-constituting carbon atom, and optionally substituted with one or more substituent(s) selected from Group B^{a}};
or, a combination wherein
X^{3a} represents a nitrogen atom, and
Z² represents a C2-C6 chain hydrocarbon group, a C1-C2 alkyl group {wherein said C1-C2 alkyl group is substituted with a methoxy group or an ethoxy group}, a C3-C8 alicyclic hydrocarbon group optionally substituted with one or more substituent(s) selected from Group A^{a}, a 3-8 membered nonaromatic heterocyclic group {wherein said 3-8 membered nonaromatic heterocyclic group is bound to the S(O)ₙₐ at a ring-constituting carbon atom, and optionally substituted with one or more substituent(s) selected from Group A^{a}}, a phenyl group optionally substituted with one or more substituent(s) selected from Group B^{a}, or a 5-10 membered aromatic heterocyclic group {wherein said 5-10 membered aromatic heterocyclic group is bound to the S(O)ₙₐ at a ring-constituting carbon atom, and optionally substituted with one or more substituent(s) selected from Group B^{a}}; and
R^{1a} and R^{3a} are identical to or different from each other, and each represent a methyl group optionally substituted with one or more fluorine atom(s), a halogen atom, or a hydrogen atom;
Group A^{a}: a group consisting of a C1-C4 chain hydrocarbon group, a C1-C6 alkoxy group {wherein said C1-C4 chain hydrocarbon group and said C1-C6 alkoxy group are optionally substituted with one or more substituent(s) selected from Group C^{a}}, an oxo group, a thioxo group, a halogen atom, and a cyano group;
Group B^{a}: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group C^{a}}, a halogen atom, and a cyano group;
Group C^{a}: a group consisting of a C1-C3 alkoxy group, a halogen atom, and a cyano group]
(provided that 2-chloro-4,6-dimethyl-3-[(2-methylphenyl)sulfonyl]-5-phenylpyridine, 3-[(1-methylethyl)sulfonyl]-1,1'-biphenyl, and 4-methyl-3-[(1-methylethyl)sulfonyl]-1,1'-biphenyl are excluded) or an N-oxide thereof, or a salt thereof.

4. The compound or an N-oxide thereof, or a salt thereof according to claim 3, wherein
X^{1a} represents CH or a nitrogen atom; and
the combination of X^{3a} and Z^{a} represents:
a combination wherein
X^{3a} represents CH, and
Z^{a} represents a C2-C6 chain hydrocarbon group or a 5-10 membered aromatic heterocyclic group {wherein said 5-10 membered aromatic heterocyclic group is bound to the S(O)ₙₐ at a ring-constituting carbon atom, and optionally substituted with one or more substituent(s) selected from Group B^{a}};
or, a combination wherein
X^{3a} represents a nitrogen atom; and
Z^{a} represents a C2-C6 chain hydrocarbon group, a C3-C8 alicyclic hydrocarbon group, a 3-8 membered nonaromatic heterocyclic group {wherein said 3-8 membered nonaromatic heterocyclic group is bound to the S(O)ₙₐ at a ring-constituting carbon atom, and optionally substituted with one or more substituent(s) selected from Group A^{a}}, a phenyl group optionally substituted with one or more substituent(s) selected from Group B^{a}, or a 5-10 membered aromatic heterocyclic group {wherein said 5-10 membered aromatic heterocyclic group is bound to the S(O)ₙₐ at a ring-constituting carbon atom, and optionally substituted with one or more substituent(s) selected from Group B^{a}}.

5. A composition comprising the compound or an N-oxide thereof, or a salt thereof according to claim 3 or 4, and an inert carrier.

6. A composition comprising one or more ingredient(s) selected from the group consisting of Group (a), Group (b), Group (c), and Group (d), and the compound or an N-oxide thereof, or a salt thereof according to claim 3 or 4:
Group (a): a group consisting of insecticidal active ingredients, miticidal active ingredients, and nematicidal active ingredients;
Group (b): fungicidal active ingredients;
Group (c): plant growth regulatory ingredients;
Group (d): repellent ingredients.

7. A method for controlling a plant disease which comprises applying an effective amount of the compound or an N-oxide thereof, or a salt thereof according to claim 3 or 4 or an effective amount of the composition according to claim 6, to a plant or soil for cultivating a plant.

8. Use of the compound or an N-oxide thereof, or a salt thereof according to claim 3 or 4 or the composition according to claim 6 for controlling a plant disease.

9. A seed or a vegetative reproductive organ holding an effective amount of the compound or an N-oxide thereof, or a salt thereof according to claim 3 or 4 or an effective amount of the composition according to claim 6.
